# EUROPÄISCHE PATENTSCHRIFT

(11) **EP 3 527 157 B1**
(45) Veröffentlichungstag und Bekanntmachung des Hinweises auf die Patenterteilung: **23.09.2020**
(21) Anmeldenummer: 19164517.5
(22) Anmeldetag: 08.11.2017
(51) Int. Cl.: A61B 17/88, B01F 13/00, B01F 15/02, A61L 24/04, A61M 5/28

(54) **VERFAHREN ZUR HERSTELLUNG EINES PREPACKED-PMMA-ZEMENTKARTUSCHENSYSTEMS**
METHOD FOR PRODUCING A PREPACKED PMMA CEMENT CARTRIDGE SYSTEM
PROCÉDÉ DE FABRICATION D'UN SYSTÈME DE CARTOUCHE DE CIMENT PMMA PRÉEMBALLÉ

(30) Priorität: 11.11.2016 DE 102016121607
(43) Veröffentlichungstag der Anmeldung: 21.08.2019
(62) Teilanmeldung aus: 17200595.1
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- EP-A2- 0 796 653
- DE-A1- 4 409 610
- US-A1- 2002 049 405
- US-A1- 2016 100 875

## Beschreibung

Die Erfindung betrifft ein Verfahren zur Herstellung eines Prepacked-PMMA-Zementkartuschensystems, das zum Mischen und Applizieren eines PMMA-Knochenzementteigs geeignet ist.

Hierin wird auch beschrieben, eine Vorrichtung zum Mischen eines Polymethylmethacrylat-Knochenzements aus zwei Ausgangskomponenten und zum Lagern der Ausgangskomponenten des Knochenzements aufweisend eine rohrförmige Kartusche mit einem zylindrischen Innenraum.

Beschrieben wird außerdem auch ein Verfahren zum Mischen und Applizieren eines Knochenzements mit einer solchen Vorrichtung und ein Verfahren zur Herstellung eines Prepacked-PMMA-Zementkartuschensystems, das zum Mischen und Applizieren eines PMMA-Knochenzementteigs geeignet ist und in dem ein Pulver als eine Ausgangskomponente des Knochenzements gelagert ist.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement, der üblicherweise als Knochenzementteig bezeichnet wird. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind dann bei geeigneter Zusammensetzung lagerstabil. Erst bei Vermischung der beiden Ausgangskomponenten zu einem Knochenzementteig reagieren die zuvor getrennt als Monomerflüssigkeit und Pulver gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Aus der EP 2 730 296 A2 ist ein thixotroper Knochenzement für die Vertebroplastie bekannt, bei dem mit mehreren Additiven die thixotropen Eigenschaften erzeugt werden. Die US 3 739 947 A offenbart eine Kartuschensystem zum Mischen eines Zements, bei dem ein Flüssigkeitsbehälter zwischen zwei Kolben geöffnet wird und die Flüssigkeit in ein Zementpulver fließt. Nachteilig ist bei diesem Kartuschensystem, dass es nicht für Knochenzemente geeignet ist, da die Öffnung zum Zementpulver durch vorzeitige Reaktion des Pulvers mit der Monomerflüssigkeit verschlossen würde und da der Inhalt der Kartusche nach der Befüllung nicht sterilisierbar ist. Zudem ist bei dem Kartuschensystem immer ein Luftüberstand in dem Behälter für das Pulver vorhanden, der zu einer unerwünschten Blasenbildung im Knochenzementteig führen würde.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der DE 44 09 610 A1, der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird bei dem geschlossenen Zementiersystem PALACOS® PRO verwirklicht, das von der Firma Heraeus Medical GmbH hergestellt und vertrieben wird.

Bei der Verwendung aller bisher bekannten Full-Prepacked-Mischsystemen muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischsysteme führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Die Aufgabe der Erfindung besteht also darin, die Nachteile des Stands der Technik zu überwinden. Insbesondere besteht die Aufgabe der Erfindung in der Entwicklung eines Verfahrens zur Herstellung einer Vorrichtung, die zum Vermischen und anschließend zum Austragen und vorzugsweise auch zum Lagern von Ausgangskomponenten eines Polymethylmethacrylat-Knochenzements geeignet sind. Insbesondere soll ein Verfahren zur Herstellung eines Prepacked-PMMA-Zementkartuschensystems, das zum Mischen und Applizieren eines PMMA-Knochenzementteigs geeignet ist, bereitgestellt werden, mit dem die Probleme überwunden werden. Die mit dem Verfahren hergestellte Vorrichtung soll durch eine herkömmliche Auspresseinrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und das Öffnen von Behältern und gegebenenfalls der Kartusche, mit möglichst wenigen Arbeitsschritten und so weit als möglich automatisiert ablaufen und vorzugsweise mit einem einzigen Antrieb anzutreiben sein.

Die mit dem Verfahren hergestellte Vorrichtung soll Knochenzementpulver oder ein Pulver enthaltend ein Knochenzementpulver und mindestens eine Monomerflüssigkeit in voneinander getrennten Hohlräumen enthalten, wobei die Monomerflüssigkeit vorzugsweise in einem Behälter angeordnet sein soll, so dass die Monomerflüssigkeit auch langfristig in der Vorrichtung gelagert werden kann. Die Vorrichtung beziehungsweise ein Verschlusssystem der Vorrichtung soll einen Hohlraum, in dem das Zementpulver gelagert ist, für Pulverpartikel undurchlässig, jedoch für Gase, wie das zur Sterilisation übliche Ethylenoxid, durchlässig verschließen. Der zur Lagerung des Zementpulvers verwendete Hohlraum soll auch zur Vermischung des Pulvers mit der Monomerflüssigkeit verwendet werden. Das bedeutet, in dem Hohlraum befindet sich nach der Vermischung der Zementkomponenten der Knochenzementteig. Das Verschlusssystem soll möglichst von außen nicht zu öffnen sein. Ein Kontakt des medizinischen Anwenders mit dem Polymethylmethacrylat-Knochenzementpulver und mit der Monomerflüssigkeit soll möglichst ausgeschlossen sein.

Die Aufgabe der Erfindung besteht also in der Entwicklung eines Verfahrens zur Herstellung einer Vorrichtung zum Lagern, Vermischen und Austragen von Polymethylmethacrylat-Knochenzement. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspresseinrichtung verbinden und diese anschließend betätigen. Zusätzliche Montage- und Arbeitsschritte sollen durch den Aufbau der Vorrichtung so weit wie möglich vermieden werden. Die Vorrichtung soll eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver soll für Ethylenoxid zugänglich sein. Die Vorrichtung soll mit Hilfe einer im OP bisher üblichen manuell angetriebenen Auspresseinrichtung aktivierbar sein, so dass nach der form- oder kraftschlüssigen Verbindung der Vorrichtung mit der Auspresseinrichtung durch Betätigung der Auspresseinrichtung ein Stößel der Auspresseinrichtung auf die Vorrichtung einwirkt und die Vorrichtung aktiviert und antreibt. Die Vermischung der Monomerflüssigkeit soll ohne einen von außen manuell zu bewegenden Mischer erfolgen.

Vom medizinischen Anwender soll das Polymethylmethacrylat-Knochenzementpulver mit der Monomerflüssigkeit innerhalb der mit dem Verfahren hergestellten Vorrichtung zusammengeführt und vermischt werden können, ohne dass beide Ausgangskomponenten mit dem medizinischen Anwender in Berührung kommen. Bei der zu entwickelnden Vorrichtung handelt es sich bevorzugt um ein Full-Prepacked-Mischsystem.

Es wird ferner ein Verfahren beschrieben, das ein möglichst unkompliziertes Anwenden einer solchen mit einem erfindungsgemäßen Verfahren hergestellten Vorrichtung ermöglicht, um die Vorteile des erfindungsgemäßen Verfahrens zu verdeutlichen.

Des Weiteren soll eine mit dem erfindungsgemäßen Verfahren hergestellte und kostengünstig zu fertigende und zuverlässig funktionierende Vorrichtung zum Mischen eines medizinischen Knochenzements und zur Lagerung der Ausgangskomponenten des Knochenzements gefunden werden, bei dem eine möglichst einfache manuelle Bedienung zum Mischen der Ausgangskomponenten angewendet werden kann.

Die erste Ausgangskomponente des Polymethylmethacrylat-Knochenzements als Mischgut soll ein Pulver sein und die zweite Ausgangskomponente soll in Form der Monomerflüssigkeit vorliegen. Die beiden Ausgangskomponenten des Knochenzements sollen bevorzugt in dem Full-Prepacked-Mischsystem getrennt gelagert werden können und durch Anwendung der Vorrichtung sicher zusammengeführt werden können.

Die Aufgaben der Erfindung werden gelöst durch ein Verfahren zur Herstellung eines Prepacked-PMMA-Zementkartuschensystems, das zum Mischen und Applizieren eines PMMA-Knochenzementteigs geeignet ist, nach Anspruch 1. Besonders vorteilhafte erfindungsgemäße Varianten sind mit den Unteransprüchen 2 bis 13 beansprucht.

Mit dem erfindungsgemäßen Verfahren ist eine Vorrichtung zum Mischen eines Polymethylmethacrylat-Knochenzements aus zwei Ausgangskomponenten und zum Lagern der Ausgangskomponenten des Knochenzements als Prepacked-PMMA-Zementkartuschensystem erhältlich, die Vorrichtung aufweisend eine rohrförmige Kartusche mit einem zylindrischen Innenraum, wobei in einem vorderen Teil des Innenraums der Kartusche ein Pulver als erste Ausgangskomponente enthalten ist und in einem hinteren Teil des Innenraums der Kartusche ein Behälter enthaltend eine Monomerflüssigkeit als zweite Ausgangskomponente angeordnet ist, wobei zwischen dem Behälter und dem Pulver ein Austragskolben angeordnet ist und wobei auf der dem Austragskolben gegenüberliegenden Seite des Behälters ein Förderkolben angeordnet ist, wobei der Austragskolben und der Förderkolben in Längsrichtung beweglich im Innenraum der Kartusche angeordnet sind, wobei ein Leitungsmittel vorgesehen ist, das den vorderen Teil und den hinteren Teil des Innenraums der Kartusche miteinander für die Monomerflüssigkeit und für Gase durchlässig verbindet und das für das Pulver undurchlässig ist.

Der Innenraum der rohrförmigen Kartusche hat eine zylindrische Geometrie, beziehungsweise ist zylindrisch. Die zylindrische Form ist die einfachste, mit der sich der Innenraum der Kartusche realisieren lässt und ist für die Führung der Bewegung des Austragskolbens und des Förderkolbens besonders gut geeignet. Zudem lassen sich der vordere und der hintere Teil des Innenraums durch die beweglichen Kolben besonders einfach in jeder Position nach außen und gegeneinander abdichten, wenn der Innenraum eine zylindrische Geometrie aufweist.

Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die begrenzende Innenwand des Innenraums kann also ein Zylinder mit beliebiger Grundfläche sein und ebenso kann gegebenenfalls der Mantel der Kartusche ein Zylinder mit beliebiger Grundfläche sein, das heißt auch mit nicht kreisförmiger oder nicht runder Grundfläche. Es wird jedoch eine zylindrische Geometrie mit rotationssymmetrischer und insbesondere kreisrunder Grundfläche für den Innenraum der ersten Kartusche bevorzugt, da diese am einfachsten zu fertigen sind und der Austragskolben und der Förderkolben dann weniger leicht im Innenraum verkannten können, wenn sie axial im Innenraum bewegt werden, das heißt in Längsrichtung im Innenraum bewegt werden. Zudem werden mögliche Undichtigkeiten zwischen der Innenwand des Innenraums und dem Austragskolben sowie der Innenwand des Innenraums und dem Förderkolben während der Bewegung der Kolben unwahrscheinlicher.

Dass der Austragskolben und der Förderkolben im zylindrischen Innenraum der Kartusche axial beweglich sind bedeutet, dass sie entlang der Zylinderachse des zylindrischen Innenraums axial beweglich sind.

Die mit dem erfindungsgemäßen Verfahren hergestellte Vorrichtung ist bevorzugt auch zum Austragen des gemischten Knochenzementteigs geeignet und vorgesehen.

Die mit dem erfindungsgemäßen Verfahren hergestellte Vorrichtung zeichnet sich dadurch aus, dass in dem vorderen Teil des Innenraums keine Mischeinrichtung vorgesehen ist. Üblicherweise sind Mischeinrichtungen, wie beispielsweise von außen bedienbare Mischflügel notwendig, um das Pulver mit der Monomerflüssigkeit zu vermischen. Dies ist mit einer Vorrichtung nicht notwendig. Insbesondere wenn in dem Pulver ein hydrophiles Additiv verteilt ist, mit dem die Monomerflüssigkeit in dem Pulver zu verteilen ist, kann die Mischeinrichtung vermieden werden.

Die Vorderseite der Kartusche ist erfindungsgemäß bevorzugt mit einem Kartuschenkopf abgedeckt. Der Kartuschenkopf ist auf der dem Austragskolben gegenüberliegenden Seite des vorderen Teils des Innenraums angeordnet. In dem Kartuschenkopf befindet sich bevorzugt ein Durchgang oder eine Applikationsöffnung, durch die der Knochenzementteig appliziert werden kann.

Erfindungsgemäß enthält das Pulver ein Zementpulver, mit dem der PMMA-Knochenzement herstellbar ist. Ferner enthält das Pulver ein hydrophiles Additiv, mit dem die Monomerflüssigkeit in dem Pulver zu verteilen ist. Das Pulver kann des Weiteren noch Röntgenopaker und/oder pharmazeutisch aktive Substanzen, wie beispielsweise Antibiotika enthalten.

Das Additiv ist vorzugsweise Partikulär oder Faserförmig. Bevorzugt weist das Additiv eine chemische Substanz mit zumindest einer OH-Gruppe auf. Das Additiv hat bevorzugt ein Aufsaugvermögen von mindestens 0,6 g Methylmethacrylat pro Gramm Additiv.

Es kann erfindungsgemäß vorgesehen sein, dass das Pulver mindestens ein partikuläres Polymethylmethacrylat oder Polymethylmethacrylat-Copolymer der Siebfraktion kleiner 100 µm, einen Initiator, und mindestens ein in Methylmethacrylat unlösliches, partikuläres oder faserförmiges Additiv aufweist, wobei das Additiv ein Aufsaugvermögen größer gleich 0,6 g Methylmethacrylat pro Gramm Additiv bei Raumtemperatur besitzt.

Ein solches Pulver ist besonders gut zur Verteilung der Monomerflüssigkeit in dem Pulver geeignet, so dass ein Aufbau der Vorrichtung ermöglicht wird, mit dem ein einseitiges Einpressen der Monomerflüssigkeit auch auf einer Schmalseite des vorderen Teils des clnnenraums der Kartusche möglich ist. Dabei wurde überraschend gefunden, dass es möglich ist, durch einfaches In-Kontakt-Bringen eines solchen und insbesondere eines im folgenden definierten Pulvers mit einer Monomerflüssigkeit, insbesondere mit einer nachstehend definierten Monomerflüssigkeit, einen klebfreien, plastisch verformbaren Knochenzementteig herzustellen, der selbstständig durch radikalische Polymerisation aushärtet, ohne dass es notwendig ist, den Zementteig manuell oder mit Hilfe von technischen Hilfsmitteln zu vermischen. Es wurde beobachtet, dass durch Zusatz eines in Methylmethacrylat unlöslichen, partikulären oder faserförmigen Additivs, das ein Aufsaugvermögen von größer 0,6 g Methylmethacrylat pro Gramm Additiv bei Raumtemperatur besitzt, zu einem Zementpulver eines niedrig-viskosen Knochenzements, ein modifiziertes Pulver als Zementpulver erhalten wird, in das Monomerflüssigkeit über eine Strecke von mindestens 5 cm eingepresst werden kann. Das Additiv verbessert überraschend auch die Benetzung des Zementpulvers mit Monomerflüssigkeit. Das Additiv hat dabei einen "Docht-Effekt" und leitet schon in sehr geringen Mengen ab 0,1 Gew.-% die Monomerflüssigkeit in das Innere des Pulvers. Weiterhin verzögert das Additiv das Verkleben der Polymerpartikel im Pulver, wodurch die Ausbildung einer blockierenden Gelschicht verzögert wird und das Eindringen der Monomerflüssigkeit in das Pulver begünstigt wird. Die Monomerflüssigkeit kann dabei in das Pulver eingepresst oder auch eingesaugt werden.

Dabei kann bevorzugt vorgesehen sein, dass das Additiv kovalent gebundene Hydroxylgruppen an seiner Oberfläche besitzt. Das Additiv kann erfindungsgemäß bevorzugt ausgewählt sein aus der Gruppe, die aus mikrokristalliner Cellulose, Oxycellulose, Stärke, Titandioxid und Siliziumdioxid besteht, wobei pyrogenes Siliziumdioxid besonders bevorzugt wird. Das Additiv kann eine Partikelgröße der Siebfraktion kleiner 100 µm, bevorzugt der Siebfraktion kleiner 50 µm und ganz besonders bevorzugt von der Siebfraktion kleiner 10 µm aufweisen. Ferner kann bevorzugt vorgesehen sein, dass das Additiv im Pulver in einer Menge von 0,1 bis 2,5 Gew.-% bezogen auf das Gesamtgewicht des Pulvers enthalten ist. Des Weiteren kann vorgesehen sein, dass das Polymerpulver Dibenzoylperoxid als Initiator enthält.

Es kann vorgesehen sein, dass die Monomerflüssigkeit mindestens ein Methylmethacrylat und einen Aktivator enthält. Des Weiteren kann vorgesehen sein, dass die Monomerflüssigkeit mindestens einen Aktivator aus der Gruppe der aromatischen Amine enthält. Ferner kann vorgesehen sein, dass die Monomerflüssigkeit mindestens einen radikalischen Stabilisator aus der Gruppe der Chinone oder der sterisch gehinderten Phenole enthält.

Vorteilhaft ist es, wenn das Additiv kovalent gebundene Hydroxylgruppen an seiner Oberfläche besitzt. Besonders vorteilhaft sind dabei besonders Si-OH-Gruppen und alkoholische OH-Gruppen. Durch die oberflächlich angeordneten OH-Gruppen hat das Additiv eine hohe Oberflächenenergie, wodurch eine gute Benetzbarkeit des Additivs mit Methylmethacrylat erreicht wird. Die pyrogenen Kieselsäuren Aerosil® 380 und Aerosil® 300 sind besonders geeignet. Daneben ist es auch möglich, durch Sol-Gel-Prozesse erzeugtes Siliziumdioxid als Additiv zu verwenden.

Es ist auch möglich, je nach dem erforderlichen Volumen an Monomerflüssigkeit, zwei oder mehrere Behälter im vorderen Teil des Innenraums der Kartusche anzuordnen.

Der Austragskolben ist ein axial im Innenraum der Kartusche beweglicher Kolben, mit dem ein Knochenzementteig aus dem vorderen Teil des Innenraums aus der Kartusche austreibbar ist. Der Knochenzementteig wird durch Mischen der Monomerflüssigkeit mit dem Pulver hergestellt.

Es kann vorgesehen sein, dass in dem Austragskolben und/oder zwischen dem Austragskolben und der Innenwand des Innenraums zumindest eine Durchführung als Leitungsmittel vorgesehen ist, durch die der vordere Teil des Innenraums und der hintere Teil des Innenraums miteinander verbunden sind. Dabei kann in oder an der zumindest einen Durchführung ein für das Pulver undurchlässiger und für die Monomerflüssigkeit und Gase durchlässiger Filter angeordnet sein.

Hiermit wird erreicht, dass die Monomerflüssigkeit innerhalb des geschlossenen Innenraums der Kartusche von dem hinteren Teil des Innenraums in den vorderen Teil des Innenraums, in dem sich das Pulver befindet, transferiert werden kann, indem der Förderkolben in Richtung des Austragskolbens gedrückt wird.

Alternativ kann das Leitungsmittel auch eine Leitung oder mehrere Leitungen sein, die außen an der Kartusche oder in der Kartuschenwand angeordnet ist oder sind und die durch Durchführungen in der Kartuschenwand oder durch Öffnungen den vorderen Teil des Innenraums mit dem hinteren Teil des Innenraums der Kartusche verbindet oder verbinden. Der Austragskolben wird dabei umgangen. Die Monomerflüssigkeit kann dabei durch diese Leitungen vom hinteren in den vorderen Teil des Innenraums gedrückt werden und Gas, wie beispielsweise Ethylenoxid, kann durch die diese Leitungen vom vorderen in den hinteren Teil des Innenraums (oder umgekehrt) strömen.

Bevorzugt kann vorgesehen sein, dass die zumindest eine Durchführung mit einem für Pulver undurchlässigen aber für die Monomerflüssigkeit durchlässigen Filter abgedeckt ist. Solche Filter werden auch als Porenfilter bezeichnet. Hierdurch kann ein Vordringen des Pulvers in den hinteren Teil des Innenraums verhindert werden und so eine vorzeitige Reaktion des Pulvers mit der Monomerflüssigkeit verhindert werden. Besonders bevorzugt ist der für das Pulver undurchlässige und für die Monomerflüssigkeit durchlässige Filter zwischen dem Austragskolben und dem Pulver angeordnet, damit das Pulver nicht in die zumindest eine Durchführung eindringt und diese nicht nach einer frühzeitigen Reaktion mit der Monomerflüssigkeit verstopft werden.

Des Weiteren kann vorgesehen sein, dass durch Vortreiben des Förderkolbens in Richtung des Austragskolbens der Behälter zu öffnen ist, die Monomerflüssigkeit in das Pulver zu pressen ist und anschließend der Austragskolben mit dem Förderkolben in Richtung der Vorderseite der Kartusche zu drücken ist.

Hierdurch wird erreicht, dass schon durch eine unidirektionale Bewegung des Förderkolbens alleine alle für das Mischen und Austragen des Knochenzements notwendigen Arbeitsschritte in der Vorrichtung angetrieben werden können. Es reicht dadurch also aus, einen linearen Antrieb auf den Förderkolben wirken zu lassen, um alle Prozesse, wie das Öffnen des Behälters, das Einpressen der Monomerflüssigkeit in das Pulver und das Austragen des aus dem Pulver und der Monomerflüssigkeit gemischten Knochenzementteigs aus der Kartusche anzutreiben beziehungsweise durchzuführen. Hierdurch wird eine denkbar einfache Vorrichtung bereitgestellt, mit der ein Knochenzement hergestellt und appliziert werden kann und gleichzeitig die Ausgangskomponenten des Knochenzements, nämlich das Pulver enthaltend ein Zementpulver und die Monomerflüssigkeit, gelagert werden können. Die Vorderseite der Kartusche liegt gegenüber dem Kartuschenboden.

Dabei kann vorgesehen sein, dass durch Drücken des Förderkolbens in Richtung der Vorderseite der Kartusche der Austragskolben in Richtung des Kartuschenkopfs zu pressen ist und dadurch der im vorderen Teil des Innenraums aus dem Pulver und der Monomerflüssigkeit entstandene Knochenzementteig durch eine Öffnung auszupressen ist.

Hiermit wird auf einfach Weise erreicht, dass der Knochenzementteig mit dem gleichen Antrieb aus der Kartusche auszutreiben ist, der auch zum Öffnen des Behälters und zum Einpressen der Monomerflüssigkeit verwendet wird, nämlich mit dem unidirektional angetriebenen Förderkolben.

Gemäß einer bevorzugten Weiterentwicklung kann vorgesehen sein, dass der Behälter für die Monomerflüssigkeit eine Glasampulle oder Kunststoffampulle ist, die durch eine Bewegung des Förderkolbens aufzubrechen ist, oder der Behälter für die Monomerflüssigkeit ein Folienbeutel ist, der durch die Bewegung des Förderkolbens aufzuschlitzen, aufzustechen oder aufzureißen ist.

Der Vorteil ist hieran, dass die Monomerflüssigkeit in solchen Behältern über besonders lange Zeit in die Vorrichtung gelagert werden kann. Zum gleichen Zweck kann vorgesehen sein, dass der Folienbeutel mit einer Metallbeschichtung, insbesondere mit Aluminium, beschichtet ist. Besonders bevorzugt ist der Behälter eine Glasampulle, da die Monomerflüssigkeit besonders lange in Glasampullen gelagert werden kann.

Ferner kann vorgesehen sein, dass an dem Austragskolben ein Rastmittel angeordnet ist, so dass der Austragskolben zwischen dem vorderen und dem hinteren Teil des Innenraums mit der Kartusche rasten kann, wobei diese Rastung durch die beim Öffnen des Behälters auftretenden Kräfte und einen auf die Monomerflüssigkeit vom Förderkolben ausgeübten Druck nicht zu lösen ist, aber durch einen direkt von dem Förderkolben auf den Austragskolben wirkenden Druck lösbar ist.

Dabei wird davon ausgegangen, dass der Förderkolben von einer üblichen Auspresseinrichtung mit einem vortreibbaren Stößel angetrieben wird und keine unnormalen Druckstöße auf den Förderkolben ausgeübt werden, die sich über die Monomerflüssigkeit als Stöße auf den Austragskolben übertragen könnten. Bei einem derartigen Stoß könnte sich der Austragskolben von dem Innenraum lösen. Durch diese Maßnahme wird erreicht, dass zunächst der Behälter durch Vortreiben des Förderkolbens geöffnet werden kann und die auslaufende Monomerflüssigkeit mit dem Förderkolben anschließend in den vorderen Teil des Innenraums der Kartusche, also in das Pulver, gepresst werden kann, wobei der Austragskolben dabei relativ zur Kartusche und zum Innenraum die Position hält. Erst nachdem die Monomerflüssigkeit weitgehend in das Pulver gepresst wurde, und somit der Knochenzementteig im vorderen Teil des Innenraums der Kartusche vorliegt, kann dann anschließend der Knochenzementteig mit dem Austragskolben aus dem vorderen Teil der Kartusche gedrückt werden, indem der Förderkolben unmittelbar (das heißt, bis auf eventuell dazwischen verbleibende Festkörper, wie Behälterteile oder Füllmaterial) auf den Austragskolben drückt und diesen antreibt. Die Kraft zur Lösung der Rastung ist also größer, als die zur Öffnung und wenn nötig auch die zur Zerstörung des Behälters der Monomerflüssigkeit notwendige Kraft. Die Zerstörung des Behälters kann beispielsweise dann sinnvoll sein, wenn der Behälter eine Glasampulle ist, die weitgehend zusammengepresst und dabei zersplittert werden muss, um ihr Volumen beim Auspressen ausreichend stark zu reduzieren. Das bedeutet, dass durch die axiale Bewegung des Förderkolbens erst der komplette Behälter für die Monomerflüssigkeit zusammengeschoben wird, wobei gleichzeitig die Monomerflüssigkeit in den vorderen Teil des Innenraums der Kartusche, beziehungsweise in das Pulver gepresst wird, und erst danach das Rastelement des Austragskolbens durch den Druck des Förderkolbens auf den Austragskolben gelöst wird und der Austragskolben den gebildeten Knochenzementteig in Richtung der Vorderseite der Kartusche beziehungsweise der Austragsöffnung presst. Auf das Rastmittel kann verzichtet werden, wenn als Behälter für die Monomerflüssigkeit ein Kunststoffbeutel verwendet wird.

Des Weiteren kann vorgesehen sein, dass die Kartusche aus einem vorderen Kartuschenteil und einem hinteren Kartuschenteil zusammengesetzt ist, die fest miteinander verbunden sind, insbesondere aneinandergeschraubt sind, wobei vorzugsweise ein Kartuschenkopf an dem vorderen Kartuschenteil befestigt ist.

Dabei kann erfindungsgemäß bevorzugt vorgesehen sein, dass der vordere Teil des Innenraums der Kartusche durch den vorderen Kartuschenteil und den Kartuschenkopf oder gegebenenfalls durch das Kopfteil und den vorderen Kartuschenteil begrenzt wird und dass der hintere Teil des Innenraums, in dem der Behälter für die Monomerflüssigkeit angeordnet ist, durch den hinteren Kartuschenteil und den Kartuschenboden oder den Förderkolben begrenzt ist.

Dadurch, dass die Kartusche aus zwei oder drei Teilen zusammengesetzt ist, wird die Montage der Vorrichtung und auch die Befüllung der Kartusche mit dem Pulver und mit dem Behälter enthaltend die Monomerflüssigkeit vereinfacht. Dadurch können wiederum Herstellungskosten gespart werden.

Bevorzugt sind die beiden Kartuschenteile und gegebenenfalls das Kopfteil gegeneinander durch umlaufende Dichtungen abgedichtet, um ein Austreten des Pulvers, der aus dem geöffneten Behälter austretenden Monomerflüssigkeit und dem Knochenzementteig an den Verbindungen zu verhindern.

Gemäß einer bevorzugten Weiterbildung der vorliegenden Erfindung kann vorgesehen sein, dass in dem hinteren Teil des Innenraums neben dem Behälter zumindest ein Füllmaterial angeordnet ist, insbesondere in einem Bereich zwischen dem Behälter und dem Austragskolben zumindest ein Füllmaterial angeordnet ist, wobei vorzugsweise das Füllmaterial ein Schaumstoff ist und/oder Kunststoffkugeln sind.

Das Füllmaterial dient zur Verdrängung der aus dem Behälter ausfließenden Monomerflüssigkeit. Mit dem Füllmaterial wird somit das freie Volumen im hinteren Teil des Innenraums beziehungsweise zwischen dem Behälter und dem Austragskolben verringert. Dieses freie Volumen muss mit der Monomerflüssigkeit gefüllt werden und verbleibt nach dem Auspressen der Monomerflüssigkeit im hinteren Teil des Innenraums der Kartusche. Durch die Verringerung des freien Volumens in diesem Bereich kann also eine geringere Menge der Monomerflüssigkeit verwendet werden. Dies ist aus Kostengründen und aufgrund der chemischen Eigenschaften der Monomerflüssigkeit erstrebenswert. Die Verwendung eines Schaumstoffs ist insbesondere bei der Anwendung einer Glasampulle als Behälter für die Monomerflüssigkeit besonders bevorzugt, da die beim Öffnen der Glasampulle mit dem Förderkolben entstehenden Glassplitter in den Schaumstoff hineingedrückt werden können und dann die Bewegung des Förderkolbens nicht blockiert wird.

Erfindungsgemäß besonders bevorzugt wird ein Schaumstoffring als Füllmaterial verwendet, der um einen Kopf einer Glasampulle als Behälter für die Monomerflüssigkeit herum in dem freien Volumen zwischen der Glasampulle und dem Austragskolben angeordnet ist. Ein Schaumstoffring aber auch ein anderer Schaumstoff als Füllmaterial kann zudem auch als Transportschutz für die Glasampulle dienen. Alternativ können auch aus elastischem Kunststoff gebildete, mechanisch deformierbare Abstandhalter als Transportschutz verwendet werden.

Es kann erfindungsgemäß vorgesehen sein, dass ein Gitter, ein Sieb oder ein Splitterschutz zwischen dem Pulver und dem Behälter für die Monomerflüssigkeit angeordnet ist, vorzugsweise zwischen dem Austragskolben und dem Behälter oder in Durchführungen in oder am Austragskolben angeordnet ist.

Das Gitter, das Sieb oder der Splitterschutz können dazu zwischen dem Austragskolben und dem Behälter oder zwischen dem Austragskolben und dem Pulver oder in der zumindest einen Öffnung des Austragskolbens oder in der zumindest einen Öffnung zwischen dem Austragskolben und der Innenwand des Innenraums angeordnet sein.

Mit dem Gitter, dem Sieb oder dem Splitterschutz können Bruchstücke des Behälters oder Fetzen des Behälters zurückgehalten werden. Hierdurch wird eine Verunreinigung des Knochenzementteigs mit dem Material des Behälters für die Monomerflüssigkeit verhindert.

Des Weiteren kann vorgesehen sein, dass der Förderkolben den Innenraum der Kartusche am Kartuschenboden verschließt, insbesondere druck- und flüssigkeitsdicht verschließt.

Damit wird erreicht, dass die Monomerflüssigkeit beim Einpressen in das Pulver nicht an der Rückseite der Kartusche austreten kann. Hierzu können beispielsweise zwei umlaufende Dichtungen verwendet werden, die den Austragskolben gegen die Innenwand der Kartusche abdichten. Die Dichtungen können beispielsweise aus Gummi bestehen.

Es kann auch vorgesehen sein, dass der Austragskolben gegen die Innenwand des Innenraums der Kartusche abgedichtet ist. Auch hierfür können umlaufende Dichtungen aus Gummi verwendet werden.

Bevorzugt kann auch vorgesehen sein, dass der Austragskolben als Lochscheibe oder als Gitterscheibe ausgebildet ist und mindestens eine gasdurchlässige, aber für Pulverpartikel undurchlässige offen-poröse Kunststoffschicht die Löcher oder die Gitteröffnungen gasdurchlässig und für Pulverpartikel undurchlässig verschließt.

Damit wird erreicht, dass Partikel des Pulvers nicht in den hinteren Teil des Innenraums gelangen und dort vorzeitig mit der Monomerflüssigkeit reagieren.

Gemäß einer Weiterbildung kann auch vorgesehen sein, dass an der dem Behälter zugewandten Seite des Austragskolbens mindestens eine Öffnungshilfe zur Öffnung des Behälters für die Monomerflüssigkeit angeordnet ist, wobei vorzugsweise die mindestens eine Öffnungshilfe zumindest eine scharfkantige Schneide oder zumindest ein Stift ist.

Hiermit kann der Behälter auf einfache Weise und mit einer definierten Krafteinwirkung durch den Förderkolben geöffnet werden.

Des Weiteren kann erfindungsgemäß vorgesehen sein, dass das Volumen der Monomerflüssigkeit im Behälter mindestens so groß ist, wie das Volumen der mit Luft gefüllten Zwischenräume zwischen den Pulverpartikeln im vorderen Teil des Innenraums, vorzugsweise mindestens so groß ist oder genauso groß ist wie das Volumen der mit Luft gefüllten Zwischenräume zwischen den Pulverpartikeln im vorderen Teil des Innenraums und dem hinteren Teil des Innenraums, wenn der Förderkolben an dem Austragskolben anliegt, abzüglich des Volumens des Materials des Behälters und gegebenenfalls des Volumens von Füllmaterial in dem hinteren Teil des Innenraums.

Damit wird sichergestellt, dass die richtige Menge an Monomerflüssigkeit in der Vorrichtung bereitgehalten wird, um einen Knochenzement der gewünschten Konsistenz herzustellen. Dadurch wird gewährleistet, dass das gesamte Pulver mit dem erforderlichen Volumen an Monomerflüssigkeit benetzt wird und ein homogener Zementteig entstehen kann.

Genauso groß bedeutet in diesem Zusammenhang bevorzugt, innerhalb einer Abweichung von maximal 10%.

Ferner kann vorgesehen sein, dass das Volumen des vorderen Teils des Innenraums mindestens gleich dem Gesamtvolumen der Pulverpartikel und der aus dem hinteren Teil des Innenraums auszupressenden Monomerflüssigkeit ist.

Ferner kann vorgesehen sein, dass an dem vorderen Ende der Kartusche oder in einem Kartuschenkopf an der Vorderseite der Kartusche eine Aufnahme für überschüssige Monomerflüssigkeit vorgesehen ist, wobei das Pulver nicht in die Aufnahme eindringen kann, wobei vorzugsweise die Aufnahme ein hydrophiles schwammartiges Gebilde ist.

Damit wird erreicht, dass der Knochenzementteig die gewünschte Konsistenz erhält und nicht zu viel Monomerflüssigkeit enthält. Damit kann Monomerflüssigkeit in leichtem Überschuss verwendet werden, um Unsicherheiten bei der Menge der in das Pulver eingetragenen Monomerflüssigkeit auszugleichen.

Es kann auch vorgesehen sein, dass das Pulver in den vorderen Teil des Innenraums eingepresst ist, vorzugsweise in dem vorderen Teil des Innenraums unter Druck steht.

Es kann vorgesehen sein, dass die Zwischenräume zwischen den Zementpartikeln des Pulvers zwischen 25 Volumenprozent und 40 Volumenprozent des eingepressten Pulvers ausmachen.

Durch das Einpressen des Pulvers wird erreicht, dass sich die Pulverpartikel derart dicht beieinander befinden, dass ein in dem Pulver verteiltes hydrophiles Additiv auch in geringer Konzentration im Pulver die Monomerflüssigkeit leitet und im Pulver verteilt, so dass es ausreicht, wenn die Monomerflüssigkeit nur von einer Seite in das Pulver eingepresst wird.

Gemäß einer bevorzugten Weiterbildung der vorliegenden Erfindung kann auch vorgesehen sein, dass unmittelbar oberhalb des Förderkolbens eine Begasungsöffnung in der Wandung der Kartusche angeordnet ist, die den hinteren Teil des Innenraums mit der umgebenden Atmosphäre verbindet.

Diese Begasungsöffnung wird durch den Förderkolben verschlossen, sobald der Förderkolben ausreichend weit in Richtung des Austragskolbens bewegt wurde. Bevorzugt ist die Begasungsöffnung verschlossen, bevor der Behälter für die Monomerflüssigkeit durch die Bewegung des Förderkolbens geöffnet ist, um ein Austreten der Monomerflüssigkeit aus dem hinteren Teil des Innenraums zu verhindern. Mit Hilfe der Begasungsöffnung kann das Innere und der Inhalt der Kartusche mit Ethylenoxid sterilisiert werden. Das Ethylenoxid kann einmal über den Kartuschenkopf in die Kartusche gelangen und zum anderen durch die Begasungsöffnung.

Es kann ferner vorgesehen sein, dass an der Rückseite der Kartusche ein Verbindungselement vorgesehen ist, mit dem die Vorrichtung mit einer Auspresseinrichtung verbunden werden kann. Die Auspresseinrichtung weist einen vortriebbaren Stößel oder eine vortreibbare Stange auf, mit der der Förderkolben im Innenraum der Kartusche in Richtung der Vorderseite der Kartusche getrieben werden kann.

Des Weiteren kann vorgesehen sein, dass an der Vorderseite der Kartusche, gegebenenfalls am Kartuschenkopf ein Austragsrohr angeordnet ist, durch das der Knochenzementteig appliziert werden kann.

Es kann auch bevorzugt vorgesehen sein, dass an einem an der Vorderseite der Kartusche angeordnetem Kartuschenkopf ein mehrteiliges Verschlusssystem umfassend eine Austragsöffnung angeordnet ist, wobei zumindest zwei Teile des Verschlusssystems angetrieben durch eine Bewegung des gemischten Knochenzementteigs gegeneinander bewegbar sind und sich durch diese Bewegung der zumindest zwei Teile des Verschlusssystems gegeneinander die Austragsöffnung öffnet, und wobei die Bewegung des gemischten Knochenzementteigs durch einen Druck des Austragskolbens auf den Knochenzementteig anzutreiben ist, wobei vorzugsweise ein Teil des Verschlusssystems, insbesondere ein Stopfen, eine für Gase durchlässige aber für das Pulver und die Monomerflüssigkeit undurchlässige Verbindung zwischen dem vorderen Teil des Innenraums der Kartusche und der Umgebung der Vorrichtung umfasst.

Hierdurch kann die Kartusche beziehungsweise der Innenraum der Kartusche verschlossen sein, so dass kein Pulver nach außen dringen kann. Dadurch ist die Vorrichtung besser zum Lagern der Ausgangskomponenten geeignet. Gleichzeitig kann das Verschlusssystem durch Antrieb des Austragskolbens geöffnet werden, so dass kein zusätzlicher Antrieb zum Öffnen des Verschlusses notwendig ist. Durch die bevorzugte Weiterbildung kann sichergestellt werden, dass der Inhalt der Vorrichtung und damit der Monomerflüssigkeitsbehälter und vor allem das Pulver von außen mit Hilfe eines sterilisierenden Gases, wie Ethylenoxid sterilisierbar ist.

Dabei kann vorgesehen sein, dass mit dem gleichen Druck auf den Knochenzementteig, mit dem das Verschlusssystem zu öffnen ist, der Knochenzementteig anschließend durch die geöffnete Austragsöffnung austragbar ist.

Dadurch reicht es aus, die Vorrichtung mit nur einem Antrieb auszustatten, mit dem sowohl das Verschlusssystem geöffnet werden kann, als auch der fertige Knochenzementteig aus der Kartusche ausgetrieben werden kann sowie das Mischen der Ausgangskomponenten und das Öffnen des Behälters angetrieben werden kann.

Bei derartigen Vorrichtungen kann auch vorgesehen sein, dass das Verschlusssystem eine Wandung mit der Austragsöffnung und einen Stopfen aufweist, wobei die Austragsöffnung mit der Umgebung der Kartusche verbunden ist und der Stopfen die Austragsöffnung verschließt, wenn die Kartusche verschlossen ist, wobei entweder die Wandung mit der Austragsöffnung durch den Druck des Knochenzementteigs bewegbar ist und der Stopfen gegen die Kartusche fixiert ist oder der Stopfen durch den Druck des Knochenzementteigs bewegbar ist und die Wandung gegen die Kartusche fixiert oder fixierbar ist.

Der Stopfen und die Wandung bilden dann die zwei beziehungsweise die zumindest zwei Teile des Verschlusssystems. Hiermit wird ein leicht und kostengünstig zu realisierendes Verschlusssystem bereitgestellt, das relativ unanfällig für Fehlfunktionen ist. Zudem kann mit einem solchen Verschlusssystem die Kraft, die zum Austreiben des Knochenzementteigs genutzt wird auch gut zum Öffnen der Kartusche verwendet werden.

Des Weiteren kann vorgesehen sein, dass ein Austragsrohr mit der Austragsöffnung beweglich gegen die Kartusche gelagert ist, wobei ein Stopfen, der das Austragsrohr verschließt, fest mit der Kartusche verbunden ist und das Austragsrohr durch den Druck auf den Knochenzementteig gegen den Stopfen bewegbar ist und dadurch zu öffnen ist.

Dabei kann wiederum vorgesehen sein, dass das Austragsrohr durch einen Druck auf die dem Kartuschenboden zugewandten Seite des Knochenzementteigs in Richtung vom Kartuschenboden weg drückbar ist und sich dabei der Stopfen aus dem Austragsrohr löst und dadurch sich die Kartusche öffnet.

Durch beide Maßnahmen wird eine besonders gut einzusetzende Vorrichtung bereitgestellt, der den Vorteil hat, dass der Anwender von außen an der Bewegung des Austragsrohrs gut erkennen kann, dass die Vorrichtung einsatzbereit ist und dass ein Austritt des Knochenzementteigs aus dem Austragsrohr unmittelbar bevorsteht. Letzteres ist auch daran zu erkennen, dass die Bewegung des Austragsrohrs wieder endet. Das Austragsrohr und der Stopfen sind in diesen Fällen Teile des Verschlusssystems. Dadurch kann ein einfaches und für Störungen unanfälliges Verschlusssystem aufgebaut werden, bei dem der Stopfen nicht aus der Vorrichtung herausfällt. Es löst sich dann also kein Teil des Verschlusssystems von der Vorrichtung.

Ferner kann vorgesehen sein, dass das Verschlusssystem eine gasdurchlässige aber für Pulver und Flüssigkeiten undurchlässige Wandung umfasst, wobei die Wandung derart in der Kartusche angeordnet ist, dass der Druck des Knochenzementteigs auf die Wandung wirkt und dadurch einen Stopfen oder einen Deckel mit der Wandung gegen die Kartusche bewegt und dadurch die Kartusche öffnet oder dadurch eine Austragsöffnung mit der Wandung gegen die Kartusche bewegt und dadurch einen Stopfen, der fest mit der Kartusche verbunden ist, aus der Austragsöffnung entfernt, wobei vorzugsweise die Wandung eine Porenscheibe umfasst.

Durch diesen Aufbau kann vor der Anwendung der Vorrichtung das Innere der Kartusche mit einem sterilisierenden Gas wie Ethylenoxid sterilisiert werden, indem Luft aus dem Innenraum der Kartusche evakuiert wird und anschließend das sterilisierende Gas eingespeist wird, auch wenn das Verschlusssystem verschlossen ist.

Des Weiteren kann vorgesehen sein, dass in dem Pulver ein hydrophiles Additiv verteilt ist, mit dem die Monomerflüssigkeit in dem gesamten Pulver verteilbar ist, bevorzugt ohne dass zuvor eine Polymerisation des Knochenzements die weitere Verteilung der Monomerflüssigkeit in dem Pulver verhindert.

Hiermit wird erreicht, dass die Monomerflüssigkeit in dem Pulver schnell verteilt wird, bevor eine Polymerisation des in dem Pulver enthaltenen Zementpulvers mit der Monomerflüssigkeit stattfindet und dadurch eine weitere Verteilung der Monomerflüssigkeit unterbunden wird. Nur hierdurch ist der erfindungsgemäß erreichte Aufbau überhaupt möglich, dass nämlich die Monomerflüssigkeit von einer Seite in das Pulver gepresst wird und sich dennoch in dem gesamten Pulver verteilen kann, bevor die Polymerisierung eine weitere Verteilung der Monomerflüssigkeit in dem Pulver unterbindet.

Gemäß einer besonders bevorzugten Weiterbildung der vorliegenden Erfindung kann vorgesehen sein, dass der vordere Teil des Innenraums der Kartusche über eine für Gase durchlässige aber für das Pulver und für die Monomerflüssigkeit undurchlässige Verbindung mit der Umgebung der Vorrichtung verbunden ist, wobei bevorzugt die Verbindung in einem Verschlusssystem oder einem Stopfen zum Verschließen einer Austragsöffnung angeordnet ist.

Dadurch kann der Inhalt der Vorrichtung mit Hilfe eines sterilisierenden Gases wie Ethylenoxid sterilisiert werden, ohne dass das Pulver oder die Monomerflüssigkeit bei der Anwendung austreten können. Wenn die gasdurchlässige und für Pulver und Monomerflüssigkeit undurchlässige Verbindung als Teil des Verschlusssystems vorgesehen ist, kann die Austragsöffnung gleichzeitig auch zum Begasen des Inhalts der Kartusche verwendet werden. Zudem muss dann keine zusätzliche Öffnung in der Kartusche vorgesehen werden.

Mit einer mit dem erfindungsgemäßen Verfahren hergestellten Vorrichtung kann ein Verfahren zum Mischen und Applizieren eines Knochenzements mit einer solchen Vorrichtung umgesetzt werden, bei dem der Förderkolben in Richtung eines Kartuschenkopfs an der Vorderseite der Kartusche gedrückt wird, wobei durch die Bewegung des Förderkolbens im Innenraum der Kartusche der Behälter geöffnet wird, anschließend die Monomerflüssigkeit in das Pulver gepresst wird und sich dadurch ein Knochenzementteig bildet, anschließend der Austragskolben in Richtung des Kartuschenkopfs gedrückt wird, wobei durch die Bewegung des Austragskolbens der Knochenzementteig durch den Kartuschenkopf aus der Kartusche ausgetrieben wird.

Neben der Monomerflüssigkeit wird auch in dem hinteren Teil des Innenraums der Kartusche enthaltene Luft aus dem hinteren Teil des Innenraums in den vorderen Teil der Kartusche gedrückt. Ferner wird die im vorderen Teil des Innenraums der Kartusche zwischen den Pulverpartikeln enthaltene Luft aus dem vorderen Teil der Kartusche durch die eindringende Monomerflüssigkeit verdrängt. Die Luft kann durch einen für das Pulver undurchlässigen aber für Gase durchlässigen Filter nach außen entweichen, so dass durch die Luft kein Überdruck im vorderen Teil des Innenraums aufgebaut wird.

Der Knochenzementteig bildet sich durch die Benetzung von in dem Pulver enthaltenen Zementpulverpartikeln mit der Monomerflüssigkeit. Anschließend erfolgt ein Anquellen der Zementpulverpartikel durch die Monomerflüssigkeit und Auslösung der radikalischen Polymerisation der Monomerflüssigkeit durch Reaktion des Beschleunigers mit dem Initiator. Der Beschleuniger und der Initiator sind Teil des Pulver-Monomerflüssigkeits-Systems. Durch diese chemischen Reaktionen wird der Knochenzementteig gebildet.

Es kann erfindungsgemäß vorgesehen sein, dass das Mischen des Knochenzementteigs ohne Anwendung von Scherkräften erfolgt. Mit Hilfe des Pulvers mit dem Additiv kann dies erreicht werden.

Es kann erfindungsgemäß vorgesehen sein, dass die Masse der in das Pulver transferierten Monomerflüssigkeit zwischen 1,5-mal und 2,5-mal so groß ist, wie die Masse des Pulvers.

Um das gewünschte Mischungsverhältnis zwischen Pulver und Monomerflüssigkeit im Knochenzementteig zu erhalten, kann erfindungsgemäß vorgesehen sein, dass überschüssige Monomerflüssigkeit an der Vorderseite der Kartusche zwischen einem porösen Filter der Wandung und einem Kartuschenkopf aufgenommen wird. Die Monomerflüssigkeit wird dazu durch den porösen Filter gedrückt, der für das Pulver und den Knochenzementteig undurchlässig ist. Durch Aufnahme der überschüssigen Monomerflüssigkeit nach dem Durchtritt der Monomerflüssigkeit durch das Pulver bis zur Wandung kann verhindert werden, dass der Knochenzementteig zu dünnflüssig wird und dadurch eine unerwünschte Konsistenz erhält. Zudem kann zur Vermeidung einer zu dickflüssigen Konsistenz des Knochenzementteigs vorgesehen sein, dass die Monomerflüssigkeit im Überschuss vorhanden ist, um die Verluste durch die zwischen dem Austragskolben und dem Förderkolben sowie in den Durchgängen des Austragskolbens verbleibende Reste der Monomerflüssigkeit auszugleichen.

Es kann vorgesehen sein, dass der Austragskolben während des Öffnens des Behälters und während des Einpressens der Monomerflüssigkeit in das Pulver mit der Innenwand der Kartusche gerastet ist, wobei die Rastung des Austragskolbens mit der Innenwand der Kartusche durch den Druck des Förderkolbens auf den Austragskolben gelöst wird und anschließend der Förderkolben den Austragskolben in Richtung des Kartuschenkopfs drückt.

Hierdurch kann sichergestellt werden, dass zunächst der Behälter für die Monomerflüssigkeit geöffnet wird und anschließend die Monomerflüssigkeit vollständig oder zumindest die gewünschte Menge der Monomerflüssigkeit in das Pulver gepresst wird, bevor der Austragskolben von dem Förderkolben in Richtung des Kartuschenkopf gedrückt wird, um den Knochenzementteig aus der Kartusche auszupressen.

Ferner kann vorgesehen sein, dass die Vorrichtung in eine Auspresseinrichtung eingesetzt wird und ein Stößel der Auspresseinrichtung vorgetrieben wird, mit dem der Förderkolben in Richtung des Kartuschenkopfs getrieben wird.

Gemäß einer Weiterentwicklung kann vorgesehen sein, dass die Monomerflüssigkeit mit Hilfe eines hydrophilen Additivs in dem Pulver verteilt wird, wobei bevorzugt das Pulver komprimiert oder unter einem mechanischen Druck in dem vorderen Teil des Innenraums enthalten ist.

Es kann erfindungsgemäß vorgesehen sein, dass das Pulver in den vorderen Teil des Innenraums der Kartusche eingepresst wird, wobei bevorzugt der vordere Teil des Innenraums der Kartusche derart verschlossen wird, dass das Pulver im vorderen Teil des Innenraums unter einem mechanischen Druck steht und dadurch komprimiert gehalten wird. Der mechanische Druck wird von der Kartusche, dem Austragskolben sowie von dem Kartuschenkopf aufrechterhalten, die dadurch unter Spannung stehen. Die Kraft zum Aufbringen des Drucks werden also durch die Elastizität beziehungsweise eine elastische Verformung der Kartusche, des Austragskolbens und des Kartuschenkopfs aufgebracht.

Des Weiteren kann vorgesehen sein, dass der Behälter durch die Bewegung des Förderkolbens komprimiert wird und vorzugsweise dabei zerstört wird.

Wenn der Behälter eine Glasampulle oder eine Kunststoffampulle ist wird diese vorzugsweise zersplittert.

Gemäß einer Weiterentwicklung des Verfahrens kann vorgesehen sein, dass eine Austragsöffnung am Kartuschenkopf zunächst mit Hilfe eines Verschlusssystems verschlossen ist, wobei durch den Druck, der mit dem Förderkolben und über den Austragskolben auf den Knochenzementteig ausgeübt wird, das Verschlusssystem geöffnet wird, wobei anschließend der Knochenzementteig mit dem gleichen Druck durch die Austragsöffnung herausgepresst wird.

Die Austragsöffnung wird bevorzugt durch ein Austragsrohr realisiert.

Dabei kann vorgesehen sein, dass ein Teil des Verschlusssystems ein Stopfen ist, der die Austragsöffnung verschließt, wobei der Stopfen mit dem Zementteig aus der Austragsöffnung herausgedrückt wird oder eine gegen die Kartusche Wandung mit der Austragsöffnung von dem Knochenzementteig in Richtung der Vorderseite der Kartusche gedrückt wird und sich dabei ein Stopfen oder ein Deckel, der mit der Kartusche fixiert ist, von der Austragsöffnung löst.

Bevorzugt ist die Wandung axial im Innenraum der Kartusche beweglich.

Die der Erfindung zugrundeliegenden Aufgaben werden gelöst durch ein Verfahren zur Herstellung eines Prepacked-PMMA-Zementkartuschensystems, das zum Mischen und Applizieren eines PMMA-Knochenzementteigs geeignet ist und in dem ein Pulver als eine Ausgangskomponente des Knochenzements gelagert ist, mit den Schritten:
A) Bereitstellen einer Kartusche mit einem zylindrischen Innenraum und eines Austragskolbens mit einem zum zylindrischen Innenraum passenden Außenumfang,
B) Einfüllen des Pulvers in den Innenraum der Kartusche, wobei das Pulver ein Zementpulver zur Herstellung des PMMA-Knochenzementteigs als Hauptkomponente aufweist und das Pulver zusätzlich ein hydrophiles Additiv umfasst, das in dem Zementpulver verteilt ist,
C) Komprimieren des Pulvers in dem Innenraum der Kartusche zwischen dem Austragskolben und einer Wandung, die den zylindrischen Innenraum an einer Vorderseite begrenzt, durch Reduzieren des Abstands zwischen dem Austragskolben und der Wandung im Innenraum,
D) Arretieren, Abstützen oder Befestigen der Wandung und/oder des Austragskolbens, so dass während der Lagerung des Pulvers von der Wandung und dem Austragskolben ein mechanischer Druck auf das Pulver ausgeübt wird und das Pulver zusammengepresst bleibt.

Beim Komprimieren des Pulvers wird das Pulver zusammengepresst und dabei der mittlere Abstand der Pulverpartikel zueinander reduziert. Dabei kann es auch zu einer elastischen Verformung der Partikel kommen.

Dabei kann vorgesehen sein, dass der Austragskolben durch Presspassung, ein Rastmittel und/oder durch Abstützen gegen die Kartusche gehalten wird und die Wandung einteilig mit der Kartusche ausgeführt ist oder die Wandung an der Innenseite der Kartusche anliegt oder die Wandung durch eine Arretierung oder durch eine Presspassung in der Kartusche gehalten wird.

Des Weiteren kann vorgesehen sein, dass in dem Innenraum der Kartusche zumindest ein Behälter enthaltend eine Monomerflüssigkeit als weitere Ausgangskomponente des Knochenzements und am Kartuschenboden der Kartusche ein Förderkolben angeordnet wird, wobei der zumindest eine Behälter zwischen dem Förderkolben und dem Austragskolben angeordnet wird und vorzugsweise in dem hinteren Teil des Innenraums der Kartusche, in dem der zumindest eine Behälter angeordnet ist, zusätzlich ein Füllmaterial angeordnet ist, insbesondere zumindest ein Schaumstoffkörper und/oder Kunststoffkörner angeordnet sind.

Ferner kann vorgesehen sein, dass der Austragskolben von dem zumindest einen Behälter und dem am Kartuschenboden in dem Innenraum der Kartusche angeordneten Förderkolben abgestützt wird.

Der Erfindung liegt die überraschende Erkenntnis zugrunde, dass es mit der mit dem erfindungsgemäßen Verfahren hergestellten Vorrichtung gelingt, einen linearen Antrieb, wie von einer herkömmlichen Auspresseinrichtung mit linear vorgetriebenem Stößel bereitgestellt wird, dazu zu nutzen, die voneinander getrennt gelagerten Ausgangskomponenten, nämlich die Monomerflüssigkeit und das Pulver enthaltend das Zementpulver, miteinander zu mischen und den so gemischten Knochenzementteig anschließend aus der Vorrichtung auszutragen. Hierdurch wird die Anwendung der Vorrichtung maximal vereinfacht. Zudem kann auch ein Verschlusssystem, das zum Verschließen des Innenraums der Kartusche dient, in dem das Pulver enthalten ist, mit dem gleichen Antrieb geöffnet werden. Besonders bevorzugt, werden alle Arbeitsschritte mit nur einer andauernden Krafteinwirkung umgesetzt. Durch ein in dem Pulver verteiltes hydrophiles Additiv und/oder durch die Verwendung eines komprimierten Pulvers kann die Monomerflüssigkeit, auch wenn sie über eine kleine Fläche in das Pulver eingepresst wird, gut in dem Pulver verteilt werden, ohne dass der anquellende Knochenzementteig eine weitere Verteilung der Monomerflüssigkeit in dem Pulver verhindert.

Die Erfindung beruht auf der Idee, die an sich bekannte lineare Vorwärtsbewegung von Stößeln von manuell betriebenen Auspresseinrichtungen so zu nutzen, dass durch kontinuierliche Einwirkung der Druckraft der linearen Vorwärtsbewegung des Stößels zuerst ein Behälter für die Monomerflüssigkeit geöffnet wird, der Behälter anschließend zusammengepresst wird, wodurch die Monomerflüssigkeit aus dem Behälter austritt und in ein verdichtetes Zementpulver eingepresst wird, wobei die zwischen den Zementpulverpartikeln vorhandene Luft durch die eingepresste Monomerflüssigkeit verdrängt wird und nach Benetzung der Zementpulverpartikel durch die Monomerflüssigkeit ein Zementteig entsteht. Voraussetzung dafür ist die Verwendung eines Zementpulvers, das so eingestellt ist, dass es sehr gut von der Monomerflüssigkeit benetzt wird und diese durch Kapillarwirkung einsaugen kann.

Die Idee der mit dem erfindungsgemäßen Verfahren hergestellten Vorrichtung besteht darin, mindestens Behälter für die Monomerflüssigkeit hinter einem Reservoir des Pulvers enthaltend das Zementpulver so anzuordnen, dass hinter dem Behälter der axial bewegliche Förderkolben angeordnet ist und zwischen dem Behälter und dem Reservoir für das Zementpulver der nur für Flüssigkeiten und Gase durchlässige Austragskolben angeordnet ist. Vor dem Reservoir des Zementpulvers kann ein axial beweglicher Sterilisationskolben (eine bewegliche Wandung mit einem für das Pulver undurchlässigen aber für die Gase durchlässigen Filter) angeordnet, der nur für Gase und Flüssigkeiten durchlässig ist. Der Sterilisationskolben ist mit einem Austragsrohr verbunden.

Bevorzugt ist eine Austragsöffnung ist in einer ersten Ausgestaltungsform mit einem nicht axial beweglichen Stopfen verschlossen, der über Stege mit der Kartusche verbunden ist. Die radialen Stege sind mit einem Ring verbunden, dieser kann entweder zwischen dem Kartuschenkopf und der Kartusche eingeklemmt sein oder alternativ kann der Ring auf mit der Kartusche verklemmt sein. Dabei hat der Ring einen geringfügig größeren Außendurchmesser als der Innendurchmesser der Kartusche. Nach der Vermischung des Zementpulvers mit der Monomerflüssigkeit schiebt der Zementteig infolge der linearen Vorwärtsbewegung der Austragskolben den Sterilisationskolben beziehungsweise die Wandung in Richtung des Kartuschenkopfs. Dadurch wird das Austragsrohr vom Stopfen abgezogen und die Austragsöffnung wird frei und der gebildete Zementteig kann durch das geöffnete Austragsrohr nach außen gepresst werden.

In einer alternativen Ausgestaltungsform ist das Austragsrohr durch einen axial verschiebbaren Stopfen verschlossen, der in dem Austragsrohr angeordnet ist. Der axial verschiebbare Kolben im Austragsrohr bildet mit der Wandung ein durch axialen Druck zu öffnendes Verschlusssystem der Kartusche. Das bedeutet, der nach Vermischung der Monomerflüssigkeit mit dem Zementpulver entstandene Zementteig drückt den Stopfen aus dem Austragsrohr. Dadurch wird das Austragsrohr frei und der Zementteig kann durch die axiale Bewegung des Förderkolbens und des Austragskolbens ausgetrieben und appliziert werden.

Es kann vorgesehen sein, dass im Sterilisationskolben beziehungsweise in der Wandung eine gasdurchlässige, aber für Pulverpartikel undurchlässige offen-poröse Kunststoffschicht angeordnet ist, welche die Oberseite des Sterilisationskolbens mit der Unterseite des Sterilisationskolbens gasdurchlässig verbindet, wobei die Kunststoffschicht bevorzugt als Scheibe ausgebildet ist. Das Gas Ethylenoxid kann zur Sterilisation durch die Durchführung des Kartuschenkopfs zum Sterilisationskolben gelangen und dann durch die offen-poröse Kunststoffschicht in den vorderen Teil des Innenraums der Kartusche eindringen und das Zementpulver sterilisieren. Partikel des Pulvers werden durch die für Partikel undurchlässige offen-poröse Kunststoffschicht des Sterilisationskolbens beziehungsweise der Wandung oder der Dichtung am Austritt aus dem vorderen Teil des Innenraums während der Sterilisation und der Lagerung sowie des Transports der Vorrichtung gehindert. Nach der erfolgten Sterilisation kann das Ethylenoxid während der Entgasung aus dem Innenraum durch die offen-poröse Kunststoffschicht des Sterilisationskolbens beziehungsweise der Wandung oder der Dichtung und gegebenenfalls einer Durchführung im Kartuschenkopf in die Umgebung entweichen.

Der besondere Vorteil der mit dem erfindungsgemäßen Verfahren hergestellten Vorrichtung besteht in der vereinfachten Handhabung. Der Anwender muss nur die mit Pulver und Monomerflüssigkeit befüllte Vorrichtung in einem ersten Schritt mit einer manuellen Auspressvorrichtung verbinden und in einem zweiten Schritt die mit dem Kartuschenkopf nach oben zeigende Auspressvorrichtung so lange betätigen, bis der Zementteig aus dem Austragsrohr austritt. Der Zementteig kann danach wie bei bisher üblichen Mischsystemen durch weitere Betätigung der Auspressvorrichtung ausgepresst werden. Es handelt sich bei dem Mischsystem beziehungsweise bei der Vorrichtung um ein Full-Prepacked-Mischsystem, das bei Verwendung eines geeigneten Zementpulvers als Ready-to-use-System eingesetzt werden kann.

Komplexe Montageschritte und ein manuelles Mischen mit Mischstäben mit daran befestigten Mischelementen sind im Gegensatz zu den bisherigen Full-Prepacked-Mischsystemen nicht mehr notwendig. Dadurch sind Anwendungsfehler durch fehlerhafte Montageschritte und durch fehlerhaftes manuelles Mischen konstruktiv ausgeschlossen.

Vorteilhaft ist es, wenn an der dem Kartuschenboden zugewandten Seite des Austragskolbens mindestens eine Öffnungshilfe zur Öffnung des Behälters für die Monomerflüssigkeit angeordnet ist, wobei als Öffnungshilfe scharfkantige Schneiden oder Stifte bevorzugt sind. Dadurch wird das Öffnen des Monomerflüssigkeitsbehälters erleichtert.

Eine beispielhafte Vorrichtung zum Lagern, Vermischen und Austragen von Polymethylmethacrylat-Knochenzement, die mit einem erfindungsgemäßen Verfahren hergestellt wurde, ist zusammengesetzt aus
a) einer hohlzylinderförmigen Kartusche mit einem am Kartuschenende angeordneten Element zur Verbindung mit einer Auspressvorrichtung,
b) einem Kartuschenkopf, der die hohlzylinderförmige Kartusche abschließt, wobei eine Durchführung zur Aufnahme des Austragsrohrs im Kartuschenkopf angeordnet ist, und wobei mindestens eine Durchführung die Außenseite des Kartuschenkopfs mit der Innenseite des Kartuschenkopfs gasdurchlässig verbindet,
c) einem Austragsrohr,
d) einem axial im Kartuschenkopf beweglichen Sterilisationskolben (als Wandung), der gasdurchlässig aber für Pulverpartikel undurchlässig ist, wobei der Sterilisationskolben beziehungsweise die Wandung eine Durchführung besitzt, die an der Oberseite mit dem Austragsrohr flüssigkeitsdurchlässig verbunden ist,
e) einem durch axiale Druckbeanspruchung zu öffnender Verschluss des Austragsrohrs, der das Austragsrohr für Zementpulverpartikel verschließt,
f) einem Förderkolben, der in der Kartusche axial beweglich angeordnet ist, der den Kartuschenboden flüssigkeitsundurchlässig verschließt,
g) einem Austragskolben, der in der Kartusche axial beweglich zwischen dem Sterilisationskolben und dem Förderkolben angeordnet ist, wobei der Austragskolben mindestens eine flüssigkeitsdurchlässige und für Pulverpartikel undurchlässige Durchführung zwischen den beiden Stirnseiten besitzt,
h) mindestens einem Monomerflüssigkeitsbehälter,
i) einem hinteren Hohlraum zur Lagerung des Monomerflüssigkeitsbehälters, der durch die Innenwand der Kartusche, den Förderkolben und den Austragskolben begrenzt ist,
j) einem Pulver enthaltend ein Zementpulver, und
k) einem vorderen Hohlraum, in dem das Pulver angeordnet ist, wobei der vordere Hohlraum durch die Innenwand der Kartusche, den Sterilisationskolben und den Austragskolben begrenzt ist.

Die Durchführung in dem Sterilisationskolben bildet dabei die Austragsöffnung in der Wandung im Sinne der vorliegenden Erfindung.

Beispielhaft kann ein Verfahren zur Vermischung des Pulvers mit der Monomerflüssigkeit unter Bildung von Knochenzementteig mit einer solchen Vorrichtung, die mit einem erfindungsgemäßen Verfahren hergestellt wurde, umgesetzt werden mit den folgenden aufeinanderfolgenden Schritten:
a) Verbinden einer Auspresseinrichtung mit dem Verbindungselement der Kartusche,
b) Vortreiben eines Stößels der Auspresseinrichtung,
c) Verschieben des Förderkolbens in Richtung des Kartuschenkopfs,
d) Komprimierung des mindestens einen Behälters für die Monomerflüssigkeit zwischen dem Förderkolben und dem Austragskolben,
e) Bersten des Behälters,
f) Zusammenschieben des Behälters und Auspressen der Luft aus dem hinteren Hohlraum und der Monomerflüssigkeit mit dem Förderkolben durch die mindestens eine Durchführung des Austragskolbens in das Pulver in dem vorderen Hohlraum,
g) Ausbreitung der Monomerflüssigkeit in dem Pulver unter gleichzeitiger Verdrängung der Luft aus den Zwischenräumen der Pulverpartikel,
h) Benetzung der Zementpulverpartikel mit der Monomerflüssigkeit,
i) Entweichen der Luft aus dem Pulver durch den Sterilisationskolben beziehungsweise die Wandung,
j) Anquellen der Zementpulverpartikel durch die Monomerflüssigkeit und Auslösung der radikalischen Polymerisation der Monomerflüssigkeit durch Reaktion des Beschleunigers mit dem Initiator,
k) Bildung des Zementteigs aus dem Zementpulver und der Monomerflüssigkeit,
l) Öffnung des Verschlusses an der Austragsöffnung durch axiale Druckbeanspruchung durch den axial in Richtung Kartuschenkopf gepressten Zementteig, und
m) Auspressen des Zementteigs durch das Austragsrohr infolge der Vorwärtsbewegung des Förderkolbens und des Austragskolbens.

Zur Bestimmung des Aufsaugvermögens der Additive wurde eine aus der Pharmazie bekannte Enslin-Apparatur (C.-D. Herzfeldt, J. Kreuter (Hrsg.): Grundlagen der Arzneiformenlehre. Galenik 2, Springer Verlag Berlin Heidelberg New York, 1999, S. 79-80.) vereinfacht. Es wurde ein Glasfiltertiegel 1D3 der Firma Schott verwendet. Es wurde zuerst die Taramasse des Glasfiltertiegels bestimmt. Dann wurden jeweils 3,000 g bzw. 1,000 g des Additivs in den Glasfiltertiegel eingewogen. Der Glasfiltertiegel wurde auf eine Saugflasche aufgesetzt. Zum Additiv wurden 20 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert bestimmt. Als Referenz wurde der Glasfiltertiegel mit Methylmethacrylat ohne zugesetztes Additiv in gleicher Weise behandelt.

### Beispiel 1: Bestimmung des Aufsaugvermögens des Additivs

Für die Bestimmung des Aufsaugvermögens des Additivs wurden folgende Ausgangsstoffe verwendet:
Methylmethacrylat (Sigma-Aldrich)
Stärke (Sigma-Aldrich, Siebfraktion <100 µm)
Cellulose (Sigma-Aldrich, Siebfraktion <100 µm)
Aerosil® 380 (Evonik, Partikelgröße ∼ 7 nm)

Für die Bestimmung des Aufsaugvermögens der Additive Stärke, Cellulose und Aerosil®380 wurde ein Glasfiltertiegel 1D3 der Firma Schott Mainz verwendet. Es wurde zuerst die Taramasse des Glasfiltertiegels bestimmt. Dann wurden jeweils 3,000 g Additiv und beim Aerosil® 380 1,000g in den Glasfiltertiegel eingewogen. Der Glasfiltertiegel mit dem eingewogenen Additiv wurde auf eine Saugflasche aufgesetzt. Zum Additiv wurden 10 ml Methylmethacrylat gegeben, so dass das Additiv vollständig bedeckt war. Das nicht vom Additiv aufgesaugte Methylmethacrylat lief durch den Glasfiltertiegel nach unten ab. Nach 15 Minuten wurde der Glasfiltertiegel mit dem Additiv und dem aufgesaugten Methylmethacrylat ausgewogen und die Masse des aufgesaugten Methylmethacrylats bestimmt. Die Bestimmung wurde jeweils dreimal wiederholt und der Mittelwert ermittelt. Als Referenz wurde der Glasfiltertiefgel mit Methylmethacrylat ohne zugesetztes Additiv in gleicher Weise behandelt.

| Additiv | Aufsaugvermögen [g Methylmethacrylat/g Additiv] |
|---|---|
| Stärke | 0,7 |
| Cellulose | 1,8 |
| Aerosil® 380 | 9,4 |

Im Folgenden werden weitere Ausführungsbeispiele der Erfindung anhand von einundzwanzig schematisch dargestellten Figuren erläutert, ohne jedoch dabei die Erfindung zu beschränken. Dabei zeigt:
Figur 1: eine schematische Querschnittansicht einer ersten beispielhaften, mit einem erfindungsgemäßen Verfahren hergestellten Vorrichtung mit zweiteiliger Kartusche im Ausgangszustand;
Figur 2: eine schematische perspektivische Ansicht der Vorrichtung nach Figur 1;
Figur 3: eine perspektivische Explosionsdarstellung der Teile der Vorrichtung nach den Figuren 1 und 2;
Figur 4: fünf schematische Querschnittansichten A bis E der ersten beispielhaften Vorrichtung, die den Ablauf der Anwendung der Vorrichtung während der Herstellung und der Applikation des Knochenzementteigs zeigen;
Figur 5: eine Ausschnittvergrößerung des Verschlusssystems als schematische Teilquerschnittansicht der ersten beispielhaften Vorrichtung;
Figur 6: sechs schematische Querschnittansichten A bis F einer zweiten beispielhaften Vorrichtung, die mit einem erfindungsgemäßen Verfahren hergestellt wurde, mit einteiliger Kartusche, die den Ablauf der Anwendung der Vorrichtung während der Herstellung und der Applikation des Knochenzementteigs zeigen;
Figur 7: eine perspektivische Außenansicht der zweiten beispielhaften Vorrichtung nach Figur 6;
Figur 8: eine perspektivische Explosionsdarstellung der Teile der zweiten Vorrichtung nach den Figuren 6 und 7;
Figur 9: sechs schematische Querschnittansichten A bis F einer dritten beispielhaften Vorrichtung, die mit einem erfindungsgemäßen Verfahren hergestellt wurde und die den Ablauf der Anwendung der Vorrichtung während der Herstellung und der Applikation des Knochenzementteigs zeigen;
Figur 10: eine perspektivische Außenansicht der dritten beispielhaften Vorrichtung nach Figur 9;
Figur 11: eine perspektivische Querschnittansicht der dritten beispielhaften Vorrichtung nach den Figuren 9 und 10 im Ausgangszustand;
Figur 12: eine Ausschnittvergrößerung als schematische Teilquerschnittansicht der dritten beispielhaften Vorrichtung im Ausgangszustand;
Figur 13: eine Ausschnittvergrößerung als schematische Teilquerschnittansicht der dritten beispielhaften Vorrichtung nach dem Auspressen des Knochenzementteigs;
Figur 14: eine schematische perspektivische Querschnittansicht einer vierten beispielhaften Vorrichtung, die mit einem erfindungsgemäßen Verfahren hergestellt wurde, im Ausgangszustand;
Figur 15: fünf schematische Querschnittansichten A bis E der vierten beispielhaften Vorrichtung, die den Ablauf der Anwendung der Vorrichtung während der Herstellung und der Applikation des Knochenzementteigs zeigen;
Figur 16: eine schematische Querschnittansicht der vierten beispielhaften Vorrichtung nach den Figuren 14 und 15 mit einteiliger Kartusche und Verlängerungsrohr im Ausgangszustand;
Figur 17: sechs schematische Querschnittansichten A bis F einer fünften beispielhaften Vorrichtung, die mit einem erfindungsgemäßen Verfahren hergestellt wurde und die den Ablauf der Anwendung der Vorrichtung während der Herstellung und der Applikation des Knochenzementteigs zeigen;
Figur 18: zwei schematische perspektivische Querschnittansichten der fünften beispielhaften Vorrichtung im Ausgangszustand;
Figur 19: eine Ausschnittvergrößerung der Vorderseite einer sechsten beispielhaften Vorrichtung, die mit einem erfindungsgemäßen Verfahren hergestellt wurde, in einer schematischen Querschnittansicht;
Figur 20: eine perspektivische Querschnittansicht des vorderen Teils der Kartusche der sechsten beispielhaften Vorrichtung; und
Figur 21: eine weitere perspektivische Querschnittansicht des vorderen Teils der Kartusche der sechsten beispielhaften Vorrichtung mit einer Austragsrohrverlängerung.

In den Figuren werden auch für unterschiedliche Ausführungsbeispiele bei gleichen oder gleichartigen Bauteilen die gleichen Bezugszeichen verwendet, um die Übersichtlichkeit zu erhalten und die Vergleichbarkeit der Ausführungsbeispiele zu ermöglichen.

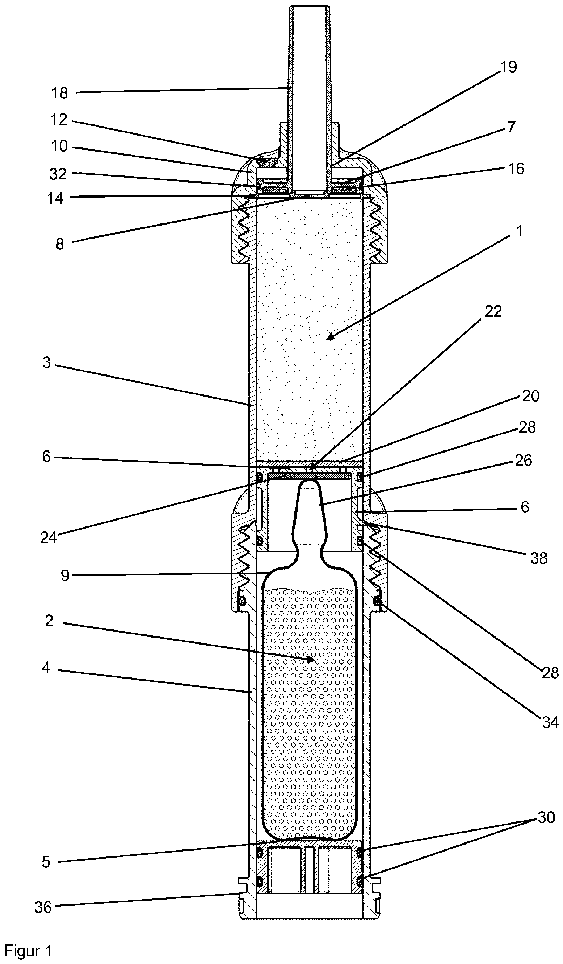

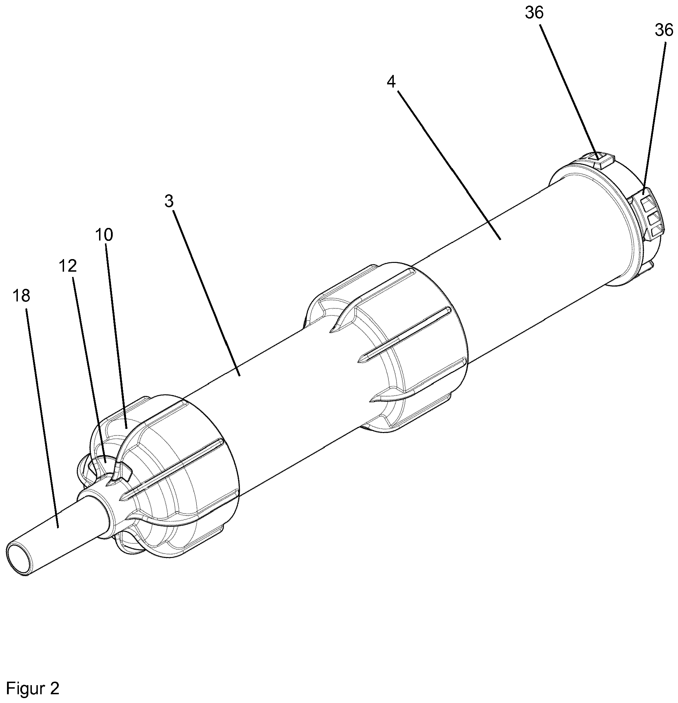

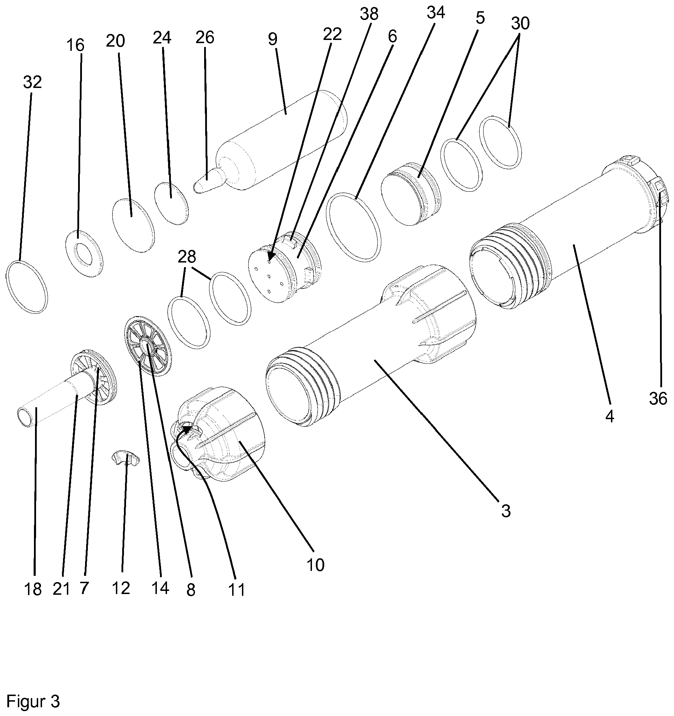

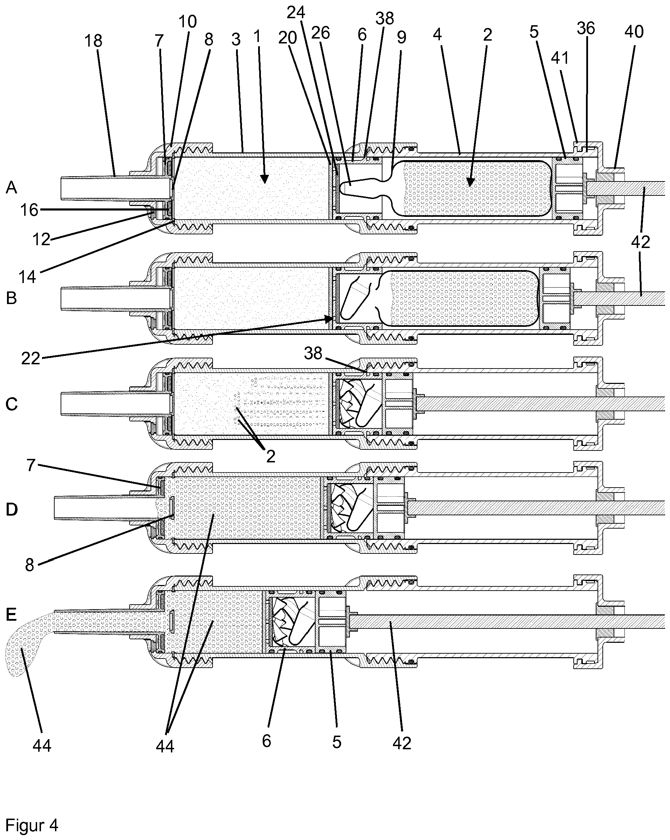

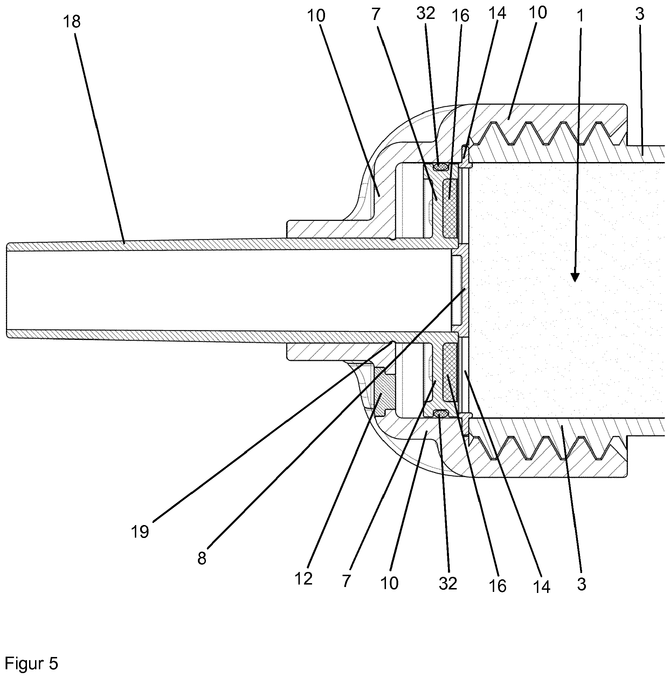

Die Figuren 1 bis 5 zeigen eine beispielhafte Ausführung einer ersten Vorrichtung, die mit einem erfindungsgemäßen Verfahren hergestellt wurde. Dabei zeigt Figur 1 eine schematische Querschnittansicht der ersten beispielhaften Vorrichtung im Ausgangszustand, Figur 2 eine schematische perspektivische Ansicht der Vorrichtung, Figur 3 eine perspektivische Explosionsdarstellung der Teile der Vorrichtung, Figur 4 fünf schematische Querschnittansichten A bis E der Vorrichtung, die den Ablauf der Anwendung der Vorrichtung während der Herstellung und der Applikation des Knochenzementteigs zeigen und Figur 5 eine Ausschnittvergrößerung als schematische Teilquerschnittansicht der Vorrichtung.

Im Ausgangszustand der Vorrichtung sind ein Pulver 1 als eine Ausgangskomponente eines PMMA-Knochenzements und eine Monomerflüssigkeit 2 als weitere Ausgangskomponente des PMMA-Knochenzements in der Vorrichtung enthalten. Das Pulver 1 enthält ein Knochenzementpulver als Hauptbestandteil sowie ein hydrophiles Additiv, mit dem die Monomerflüssigkeit 2 in dem Pulver 1 verteilbar ist. Das Pulver 1 und die Monomerflüssigkeit 2 sind in einer zweiteiligen Kartusche 3, 4 enthalten, wobei das Pulver 1 in einem vorderen Kartuschenteil 3 mit einem vorderen Innenraum und die Monomerflüssigkeit 2 in einem hinteren Kartuschenteil 4 mit einem hinteren Innenraum der Kartusche 3, 4 angeordnet ist. Gemeinsam begrenzt der hintere Innenraum und der vordere Innenraum einen zylindrischen Innenraum der Kartusche 3,4.

An der Rückseite der Vorrichtung (in Figur 1 unten, in Figur 2 nach rechts oben hinten und in Figur 4 rechts) ist ein Förderkolben 5 angeordnet, der in axialer Richtung linear in Richtung der Vorderseite der Vorrichtung (in Figur 1 oben, in Figur 2 nach links unten vorne und in Figur 4 links) im Innenraum der Kartusche 3, 4 vortreibbar ist, beziehungsweise beweglich gelagert ist. Der Förderkolben 5 schließt die Rückseite des Innenraums der Kartusche 3, 4 ab. Im hinteren Ende des vorderen Innenraums, beziehungsweise in der Verbindung vom vorderen Innenraum zum hinteren Innenraum der Kartusche 3, 4 ist ein Austragskolben 6 angeordnet, der in axialer Richtung linear in Richtung der Vorderseite der Vorrichtung im Innenraum der Kartusche 3, 4 vortreibbar ist, beziehungsweise beweglich gelagert ist. Der Austragskolben 6 ist also zwischen dem Pulver 1 und der Monomerflüssigkeit 2 im Innenraum der Kartusche 3, 4 angeordnet.

An der Vorderseite der Vorrichtung ist ein Verschlusssystem angeordnet, mit dem der Innenraum der Kartusche 3, 4 nach vorne abgeschlossen ist, das sich aber zum Austragen eines aus den Ausgangskomponenten 1, 2 gemischten Knochenzementteigs 44 (siehe Figuren 4 D und 4 E) öffnen lässt. Eine Wandung 7 des Verschlusssystems weist eine zentrale kreisrunde Austragsöffnung auf und ist in axialer Richtung des vorderen Innenraums des vorderen Kartuschenteils 3 beweglich angeordnet. Die Austragsöffnung ist durch einen inneren Ring begrenzt. Die Wandung 7 ist nach Art eines Rads mit einem äußeren Ring aufgebaut, der mit dem die Austragsöffnung begrenzenden inneren Ring über eine Mehrzahl von Streben verbunden ist. Der innere Ring, der äußere Ring und die Streben sind einteilig aus Kunststoff gefertigt. Ferner umfasst das Verschlusssystem einen Stopfen 8, mit dem die Austragsöffnung im Ausgangszustand, wie er in Figur 1 gezeigt ist, verschlossen ist.

Die Monomerflüssigkeit 2 ist in einer geschlossenen Glasampulle 9 als Behälter 9 für die Monomerflüssigkeit 2 enthalten. Innerhalb der Glasampulle 9 kann die Monomerflüssigkeit 2 über lange Zeit in der Vorrichtung gelagert werden.

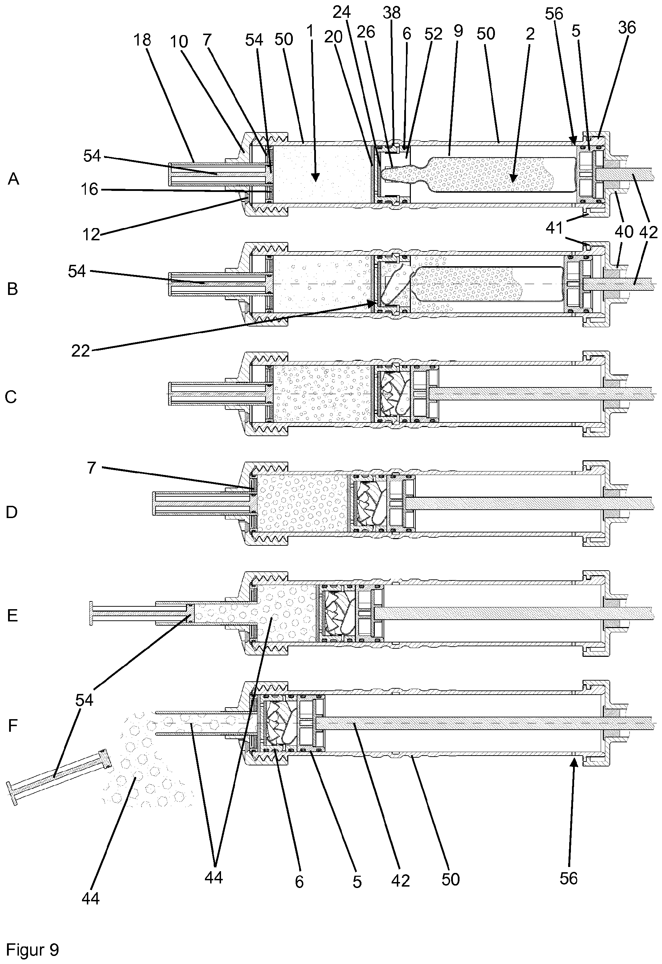

Das Verschlusssystem der Vorrichtung ist in einem Kartuschenkopf 10 angeordnet, der den vorderen Bereich des vorderen Innenraums beziehungsweise den vorderen Kartuschenteil 3 der Kartusche 3, 4 begrenzt. Genaugenommen ist der Kartuschenkopf 10 ein Teil der Kartusche 3, 4. In dem Kartuschenkopf 10 ist an der Fronseite eine Begasungsöffnung 11 vorgesehen, die im Lagerungszustand beziehungsweise im Ausgangszustand der Vorrichtung mit einer Kappe 12 verschlossen ist. Durch die Begasungsöffnung 11 kann der Innenraum der Kartusche 3, 4 mit einem sterilisierenden Gas, wie Ethylenoxid, begast werden und so der Inhalt der Kartusche 3, 4 sterilisiert werden. Vorzugsweise sind in der Wandung des hinteren Kartuschenteils 4 direkt neben der Position des Förderkolbens 5 noch weitere Begasungsöffnungen (nicht gezeigt) analog den Ausführungsbeispielen nach den Figuren 9 bis 18 vorgesehen, die das Innere der Kartusche 3, 4 mit der Umgebung verbinden und durch die der Innenraum der Kartusche 3, 4 von außen mit dem sterilisierenden Gas begast werden kann. Bei einer Bewegung des Förderkolbens 5 in Richtung des Kartuschenkopfs 10 werden diese rückseitigen Begasungsöffnungen durch den Förderkolben 5 verschlossen, so dass keine aus der Ampulle 9 austretende Monomerflüssigkeit 2 aus dem hinteren Teil des Innenraums nach außen dringen kann. Dadurch kann das sterilisierende Gas durch die Kartusche 3, 4 geleitet werden.

Der Stopfen 8 des Verschlusssystems wird von einem Halterungsring 14 gehalten, wobei der Stopfen 8 über Streben des Halterungsrings 14 mit einem äußeren Ring des Halterungsrings 14 verbunden ist. Der Halterungsring 14 ist außen in der Verbindung zwischen dem Kartuschenkopf 10 und dem vorderen Kartuschenteil 3 fixiert. Dadurch ist auch der Stopfen 8 gegen die Kartusche 3, 4 fixiert.

Die zwischen den Streben der Wandung 7 des Verschlusssystems offenen Zwischenräume sind durch einen gasdurchlässigen aber für das Pulver 1 und den Knochenzementteig 44 undurchlässigen Filter 16 in Form einer Lochscheibe abgedeckt. Dadurch ist die Wandung 7 für Gase wie Ethylenoxid durchlässig aber für das Pulver 1 und den Knochenzementteig 44 undurchlässig. Analog dem Halterungsring 14 ist die Wandung 7 nämlich durch ein Gerüst in Form des äußeren Rings mit den sich nach Innen zur Austragsöffnung hin radial erstreckenden Speichen geformt und somit durchlässig, wenn deren Zwischenräume nicht durch den Filter 16 abgedeckt wären. Der Aufbau der Wandung 7 und des Halterungsrings 14 ist am besten in Figur 3 zu erkennen. Die Wandung 7 mit dem Filter 16 sowie dem gegen die Kartusche 3, 4 unbeweglich gehaltenen Stopfen 8 und dem Halterungsring 14 bilden ein Verschlusssystem für die Vorrichtung.

Einteilig mit der Wandung 7 ausgeführt, ist an der Austragsöffnung, beziehungsweise an dem Inneren Ring der Wandung 7, der die Austragsöffnung begrenzt, ein Austragsrohr 18 angeordnet, durch das bei der Anwendung der Vorrichtung eine Applikation des Knochenzementteigs 44 erfolgt (siehe Figur 4 E). Das Austragsrohr 18 mündet also in die Austragsöffnung im Inneren der Kartusche 3, 4. Mit der Wandung 7 wird also auch das Austragsrohr 18 bewegt. Dazu ist das Austragsrohr 18 durch eine Durchführung in dem Kartuschenkopf 10 in Längsrichtung (also in axialer Richtung des zylindrischen Innenraums der Kartusche 3, 4) beweglich gelagert. In der Durchführung sind Nocken 19 vorgesehen, so dass das Austragsrohr 18 und damit die Wandung 7 mit dem Filter 16 nur gegen einen Widerstand gegen den Kartuschenkopf 10 bewegt werden kann. In dem Austragsrohr 18 ist eine umlaufende Nut 21 vorgesehen, die mit den Nocken 19 eine lösbare Arretierung des Austragsrohrs 18 und damit der Wandung 7 gegen den Kartuschenkopf 10 und damit gegen die Kartusche 3, 4 bildet. Die Wandung 7 mit dem Filter 16 kann auch als Sterilisationskolben bezeichnet werden. Das Pulver 1 ist unter Druck in den vorderen Teil des Innenraums der Kartusche 3, 4 zwischen der Wandung 7 mit dem Filter 16 und dem Austragskolben 6 eingepresst und steht unter einem elastischen mechanischen Druck. Durch die Arretierung der Nocken 19 mit der umlaufenden Nut 21 in der Durchführung soll vermieden werden, dass der von dem zusammengepressten Pulver 1 auf die Wandung 7 mit dem Filter 16 ausgeübte elastische Druck ausreicht, um die Wandung 7 mit dem Filter 16 in Richtung der Vorderseite der Kartusche 3, 4 beziehungsweise an die Frontseite des durch den Kartuschenkopf 10 begrenzten Innenraums zu drücken und dadurch das Pulver 1 wieder zu entspannen.

An der zum Pulver 1 weisenden Seite des Austragskolbens 6 ist ein für das Pulver 1 undurchlässiger aber für die Monomerflüssigkeit 2 durchlässiger Porenfilter 20 angeordnet. Damit soll verhindert werden, dass das Pulver 1 durch Durchgänge 22, die in dem Austragskolben 6 vorgesehen sind, in den hinteren Teil des Innenraums der Kartusche 3, 4 vordringen kann. Der Porenfilter 20 deckt dabei die Durchgänge 22 ab, so dass das Pulver 1 auch nicht in die Durchgänge 22 vordringen kann. Hierdurch wird vermieden, dass die Monomerflüssigkeit 2 nach dem Öffnen der Ampulle 9 bereits vorzeitig mit Zementpulverpartikeln des Pulvers 1 reagiert, also bevor die Monomerflüssigkeit 2 in den vorderen Teil des Innenraums eingepresst wurde. Dadurch kann verhindert werden, dass die Durchgänge 22 durch anquellenden Knochenzement blockiert werden und dadurch keine weitere Einleitung von Monomerflüssigkeit 2 in das Pulver 1 mehr möglich ist.

Auf der dem Porenfilter 20 gegenüberliegenden Seite des Austragskolbens 6 ist ein Gitter 24 oder Sieb 24 angeordnet, mit dem das Vordringen von Splittern der aufgebrochenen Glasampulle 9 in die Durchgänge 22 verhindert wird. Auch hiermit soll sichergestellt werden, dass die Monomerflüssigkeit 2 aus dem hinteren Teil des Innenraums der Kartusche 3, 4 problemlos in das Pulver 1 gedrückt werden kann. Beim Aufbrechen der Ampulle 9 wird zunächst ein Ampullenkopf 26 abgebrochen und dadurch die Ampulle 9 geöffnet (siehe Figur 4 B). Die Monomerflüssigkeit 2 aus der Ampulle 9 kann dann in den vorderen Teil des Innenraums der Kartusche 3, 4 ausfließen und anschließend durch die Durchgänge 22 in das Pulver 1 gepresst werden (siehe Figur 4 C). Dabei wird die Ampulle 9 in derart kleine Splitter zersplittert, dass diese in einen Hohlraum passen, der auf der dem Kartuschenboden zugewandten Seite (in Figur 1 nach unten, in Figur 2 nach oben rechts hinten und in Figur 4 nach rechts) des Austragskolbens 6 gebildet ist.

Damit die Monomerflüssigkeit 2 nicht am Austragskolben 6 vorbei in den vorderen Teil des Innenraums der Kartusche 3, 4 gedrückt wird, sind zwei umlaufende Dichtungsringe 28 aus Gummi an dem Austragskolben 6 vorgesehen, mit denen der Austragskolben 6 gegen die Wand des Innenraums der Kartusche 3, 4 abgedichtet ist. Ebenso sind am Förderkolben 5 zwei umlaufende Dichtungsringe 30 aus Gummi vorgesehen, mit denen ein Austritt der Monomerflüssigkeit 2 am Kartuschenboden nach außen vermieden wird. Die Dichtungswirkung der Dichtungen 30 muss zumindest dafür ausreichen, damit keine Monomerflüssigkeit 2 austritt, auch wenn mit dem Förderkolben 5 ein derart großer Druck auf die Monomerflüssigkeit 2 ausgeübt wird, dass diese durch das Gitter 24, die Durchgänge 22 und den Porenfilter 20 in das Pulver 1 eingepresst wird.

Des Weiteren ist die Wandung 7 mit einer umlaufenden Dichtung 32 aus Gummi gegen die Wand des vorderen Innenraums im Bereich des Kartuschenkopfs 10 abgedichtet. Die Dichtung 32 läuft außen um den äußeren Ring der Wandung 7. Ferner sind auch die beiden Kartuschenteile 3, 4 gegeneinander mit einer umlaufenden Dichtung 34 aus Gummi gegeneinander abgedichtet. Die beiden Kartuschenteile 3, 4 sind über ein Gewinde miteinander verschraubt. Dazu ist am vorderen Kartuschenteil 3 ein Innengewinde und am hinteren Kartuschenteil 4 ein Außengewinde vorgesehen. In gleicher Weise sind der Kartuschenkopf 10 und der restliche vordere Kartuschenteil 3 miteinander verbunden, wobei hier die Abdichtung durch das Gewinde selbst oder durch den dazwischen eingeklemmten Halterungsring 14 erfolgt.

Am Kartuschenboden ist außen an dem hinteren Kartuschenteil 4 eine Halterung 36 zur Befestigung einer Auspresseinrichtung 40 (siehe Figur 4) vorgesehen.

An dem Austragskolben 6 sind seitlich mehrere Haken 38 als Rastmittel vorgesehen, die in dazu passende Ausnehmungen an der Verbindung des vorderen Kartuschenteils 3 zum hinteren Kartuschenteil 4 an der Wand des Innenraums greifen. Die dadurch hervorgerufene Rastung des Austragskolbens 6 mit der Kartusche 3, 4 ist stark genug, um dem Druck der beim Zersplittern der Glasampulle 9 auftretenden Kräfte und dem Druck der Monomerflüssigkeit 2 während des Vortriebs des Förderkolbens 5 zu wiederstehen. Erst wenn der Förderkolben 5 unmittelbar an dem Austragskolben 6 anliegt (siehe Figur 4 C), wird die Rastung gelöst, beziehungsweise werden die Haken 38 verformt und gleiten aus den Ausnehmungen in der Wand des Innenraums der Kartusche 3, 4, und anschließend der Austragskolben 6 von dem Förderkolben 5 in Richtung des Kartuschenkopfs 10 an der Vorderseite der Vorrichtung gedrückt.

In dem in der Rückseite des Austragskolbens 6 gebildeten Hohlraum im Austragskolben 6 kann vorzugsweise ein Füllmaterial (nicht gezeigt), wie beispielsweise ein Schaumstoffeinsatz und/oder Kunststoffkörner, vorgesehen sein. Damit soll das Volumen der Monomerflüssigkeit 2, die in diesem Hohlraum zurückbleibt und nicht von dem Förderkolben 5 in das Pulver 1 gepresst werden kann, möglichst klein gehalten werden. Des Weiteren kann dieses Füllmaterial als Transportschutz und Stoßschutz für die Glasampulle 9 verwendet werden, damit die Glasampulle 9 beim Transport der Vorrichtung im Ausgangszustand (siehe Figuren 1 und 2) nicht aus Versehen aufbricht. Hierzu kann ein komprimierbarer Schaumstoff zusätzlich um die Glasampulle 9 herum im hinteren Teil des Innenraums der Kartusche 3, 4 angeordnet sein. Alternativ können auch aus elastischem Kunststoff gebildete, mechanisch deformierbare Abstandhalter als Transportschutz verwendet werden.

Der Ablauf eines beispielhaften Verfahrens zur Herstellung eines Knochenzementteigs ist in Figur 4 durch fünf übereinander gezeichnete Querschnittansichten Figur 4 A bis Figur 4 E dargestellt. Zunächst wird die Vorrichtung in eine Auspresseinrichtung 40 eingesetzt, dazu wird die Kartusche 3, 4 mit der Halterung 36 an einem passenden Gegenstück 41 der Auspresseinrichtung 40 befestigt (siehe Figur 4 A).

Anschließend wird ein Stößel 42 der Auspresseinrichtung 40 gegen das Gegenstück 41 vorgetrieben. Der Stößel 42 liegt an dem Förderkolben 5 an. Dadurch wird der Förderkolben 5 von dem Stößel 42 in Richtung des Austragskolbens 6 gedrückt. Durch die Bewegung des Förderkolbens 5 wird die Ampulle 9 gegen den mit dem Rastmittel 38 arretierten Austragskolben 6 gedrückt. Der Ampullenkopf 26 bricht ab und die Ampulle 9 ist geöffnet (siehe Figur 4 B).

Die Vorrichtung in der Auspresseinrichtung 40 wird dabei bevorzugt mit dem Kartuschenkopf 10 nach oben gehalten, so dass bei einem nachfolgenden weiteren Vortreiben des Förderkolbens 5 zunächst die überstehende Luft aus dem hinteren Teil des Innenraums nach oben durch das Pulver 1 und durch den gasdurchlässigen Filter 16 nach außen gedrückt wird. Irgendwann wird die Monomerflüssigkeit 2 aus der Ampulle 9 von dem Förderkolben 5 durch das Gitter 24 und/oder das Sieb 24, durch die Durchgänge 22 und durch den Porenfilter 20 in den vorderen Teil des Innenraums in das Pulver 1 gedrückt. Dabei wird die Ampulle 9 weiter zusammengedrückt und zersplittert dabei in kleinere Splitter, die sich schließlich in dem rückseitigen Hohlraum des Austragskolbens 6 sammeln. Das Pulver 1 enthält ein hydrophiles Additiv, das eine große Oberflächenenergie bezüglich der wässrigen Monomerflüssigkeit 2 aufweist, die größer ist als die des Knochenzementpulvers. Gleichzeitig sind die Kapillarkräfte aufgrund des komprimierten Pulvers 1 groß, da die Zwischenräume zwischen den Pulverpartikeln klein sind. Zudem wird die Monomerflüssigkeit 2 mit Druck in das Pulver 1 gepresst. Durch all diese Maßnahmen wird die Monomerflüssigkeit 2 schnell in und durch das Pulver 1 geleitet und kann sich vollständig in dem Pulver 1 ausbreiten und verteilen, bevor die anquellenden Zementpulverpartikel eine weitere Ausbreitung der Monomerflüssigkeit 2 im Pulver 1 unterbinden. Schließlich trifft der Förderkolben 5 auf den Austragskolben 6 (siehe Figur 4 C).

Das Zementpulver in dem Pulver 1 reagiert mit der Monomerflüssigkeit 2 und bildet dort den Knochenzementteig 44. Um dabei das gewünschte Mischungsverhältnis zwischen Pulver 1 und Monomerflüssigkeit 2 im Knochenzementteig 44 zu erhalten, kann überschüssige Monomerflüssigkeit 2 an der Vorderseite der Kartusche 3, 4 zwischen dem porösen Filter 16 der Wandung 7 und dem Kartuschenkopf 10 aufgenommen werden. Die Monomerflüssigkeit 2 wird dazu durch den porösen Filter 16 gedrückt, der für das Pulver 1 und den Knochenzementteig 44 undurchlässig ist. Durch Aufnahme der überschüssigen Monomerflüssigkeit 2 nach dem Durchtritt der Monomerflüssigkeit 2 durch das Pulver 1 bis zur Wandung 7 wird verhindert, dass der Knochenzementteig 44 zu dünnflüssig wird und dadurch eine unerwünschte Konsistenz erhält. Zudem wird zur Vermeidung einer zu dickflüssigen Konsistenz des Knochenzementteigs 44 die Monomerflüssigkeit 2 im Überschuss verwendet, so dass die Verluste durch die zwischen dem Austragskolben 6 und dem Förderkolben 5 sowie in den Durchgängen 22 des Austragskolbens 6 verbleibende Reste der Monomerflüssigkeit 2 ausgeglichen werden.

Durch einen weiteren Vortrieb des Förderkolbens 5 wird der Austragskolben 6 in Richtung des Kartuschenkopfs 10 getrieben und die Rastung beziehungsweise das Rastmittel 38 gelöst. Durch die Bewegung des Austragskolbens 6 in Richtung des Kartuschenkopfs 10 wird vom Knochenzementteig 44 ein Druck auf die Wandung 7 und den Filter 16 des Verschlusssystems ausgeübt. Der Knochenzementteig 44 kann nicht durch den Filter 16 fließen, so dass der Druck des Knochenzementteigs 44 auf den Filter 16 und die Wandung 7 wirkt. Die Arretierung durch die Nocken 19 und die umlaufende Nut 21 wird gelöst und dadurch die Wandung 7, der Filter 16 und das Austragsrohr 18 beweglich gegen die Kartusche 3, 4 beziehungsweise den Kartuschenkopf 10, so dass diese Teile des Verschlusssystems nach vorne gedrückt werden, während der Stopfen 8, der über den Halterungsring 14 fest mit der Kartusche 3, 4 verbunden ist, nicht mit bewegt wird. Dabei fließt der Knochenzementteig 44 durch die Öffnungen zwischen den Speichen des Halterungsrings 14 hindurch. Währenddessen wird die Austragsöffnung in der Wandung 7 geöffnet. Die Wandung 7, der Filter 16 und das Austragsrohr 18 werden so weit nach vorne getrieben, bis die Vorderseite der Wandung 7 an der frontalen Innenseite des Kartuschenkopfs 10 anliegt. Diese Situation ist in Figur 4 D dargestellt. Die Kartusche 3, 4 ist nun nach außen geöffnet. Der Abstand zwischen der Wandung 7 und dem Stopfen 8 ist, wenn die Wandung 7 an der Frontseite des Innenraums anliegt, so groß, dass die freie Fläche, durch die der Knochenzementteig 44 ausfließt, mindestens so groß ist, wie die Querschnittsfläche des Austragsrohrs 18, damit der Fluss des Knochenzementteigs 44 möglichst wenig behindert wird.

Durch weiteres Vortreiben des Förderkolbens 5 und damit des Austragskolbens 6 wird der fertige Knochenzementteig 44 durch die Austragsöffnung und das Austragsrohr 18 nach außen gepresst und kann appliziert werden (siehe Figur 4 E).

Aufgrund des in dem Pulver 1 vorhandenen Additivs gelingt es, die Monomerflüssigkeit 2 an einer Grundfläche des vorderen Teils des zylindrischen Innenraums der Kartusche 3, 4 einzupressen und dennoch eine vollständige Verteilung der Monomerflüssigkeit 2 in dem Pulver 1 zu erreichen. Durch den Aufbau der Vorrichtung gelingt es, eine herkömmliche Auspresseinrichtung 40 verwenden zu können und durch eine unidirektionale lineare Bewegung des Stößels 42 sowohl den Behälter 9 für die Monomerflüssigkeit 2 zu öffnen, die Monomerflüssigkeit 2 in das Pulver 1 zu pressen und dadurch den Knochenzementteig 44 zu mischen, als auch das Verschlusssystem zu öffnen und den gemischten Knochenzementteig 44 auszutreiben und zu applizieren. Mit dem Aufbau des Verschlusssystems gelingt es, die Kraft, die durch den Stößel 42 auf den Förderkolben 5 ausgeübt wird, zum Öffnen der Austragsöffnung nutzen können.

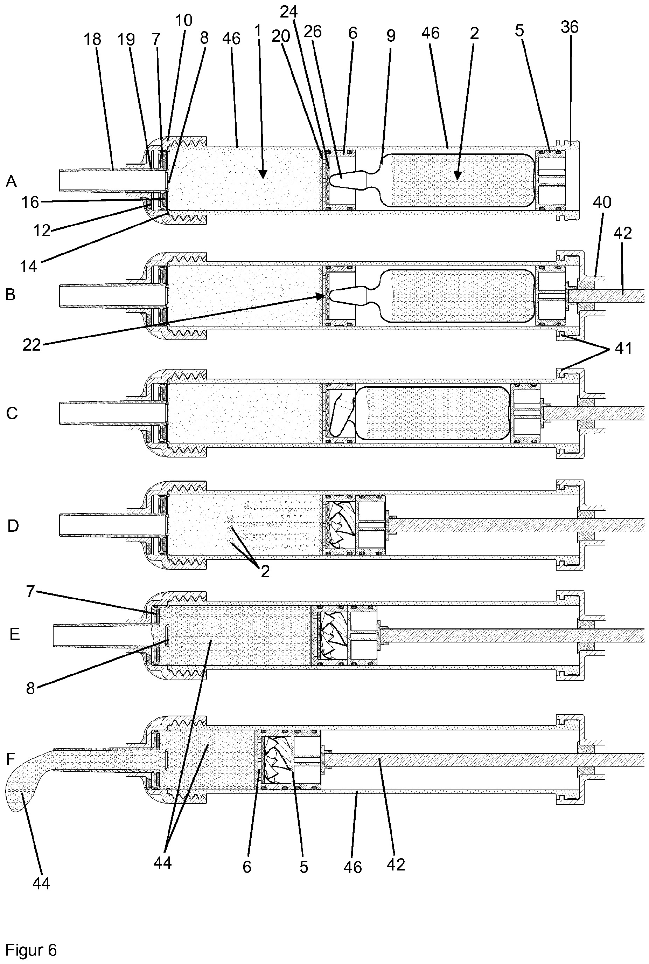

In den Figuren 6 bis 8 ist eine zweite beispielhafte Vorrichtung gezeigt, die mit einem erfindungsgemäßen Verfahren hergestellt wurde, wobei die Vorrichtung sich von der ersten beispielhaften Vorrichtung nach den Figuren 1 bis 5 dadurch unterscheidet, dass die zweite beispielhafte Vorrichtung eine Kartusche 46 aufweist, die nicht aus zwei Kartuschenteilen besteht, wobei der Kartuschen kopf 10 noch immer aufgeschraubt ist, und dass der ein Austragskolben 6 im Innenraum der Kartusche 46 nicht über ein Rastmittel mit der Kartusche 46 verbunden ist.

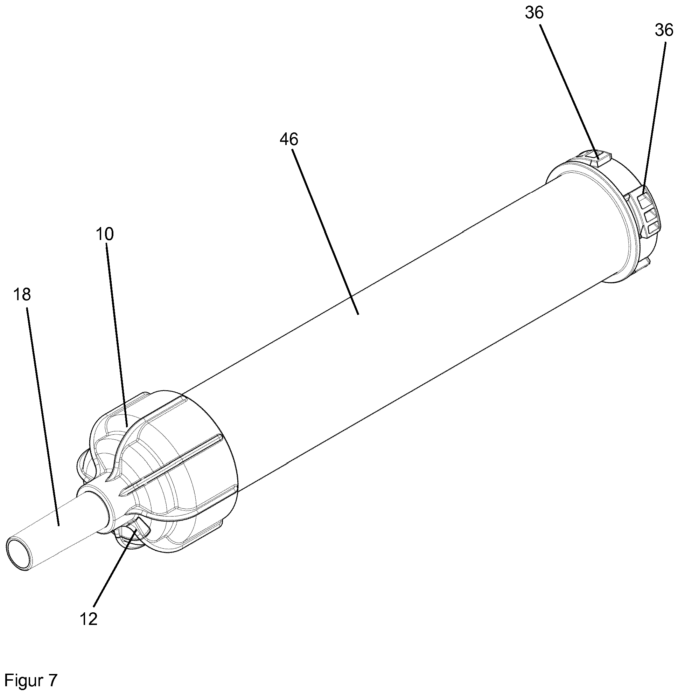

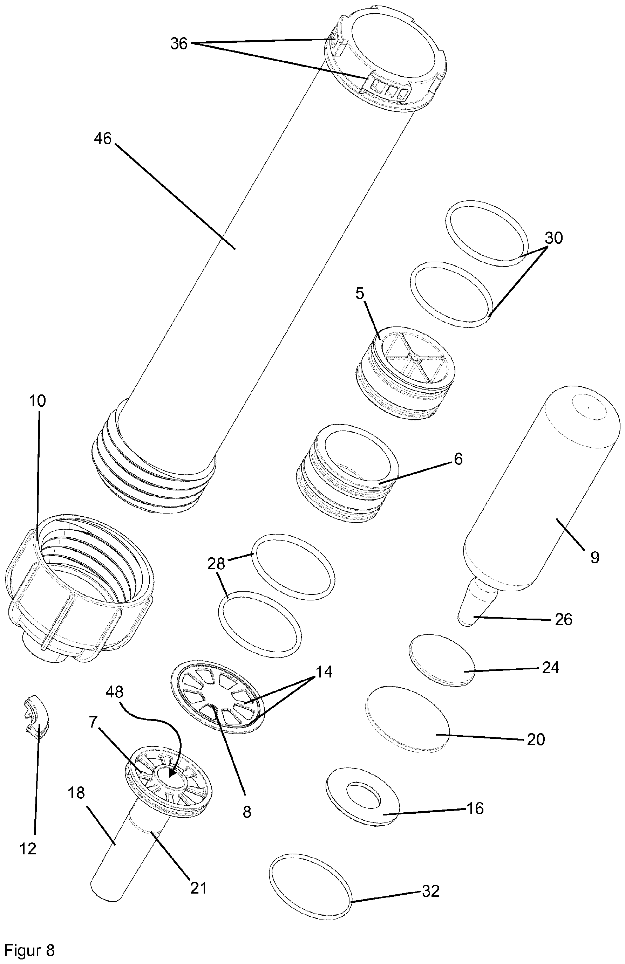

Dabei zeigt Figur 6 sechs schematische Querschnittansichten A bis F der zweiten beispielhaften Vorrichtung mit einteiliger Kartusche 46, die den Ablauf der Anwendung der Vorrichtung während der Herstellung und der Applikation eines Knochenzementteigs 44 zeigen, Figur 7 eine perspektivische Außenansicht der zweiten beispielhaften Vorrichtung und Figur 8 eine perspektivische Explosionsdarstellung der zweiten beispielhaften Vorrichtung.

Der Aufbau und die Funktionsweise der zweiten beispielhaften Vorrichtung entsprechen weitgehend denen der ersten beispielhaften Vorrichtung, so dass auch weitgehend auf die Figurenbeschreibung des ersten Ausführungsbeispiels verwiesen werden kann. Insbesondere sind das verwendete Pulver 1 und die Wirkungsweise der Kolben 5, 6 ist bis auf die Rastung mit der Innenseite der Kartusche bei beiden Ausführungsbeispielen identisch.

Im Ausgangszustand der Vorrichtung sind das Pulver 1 als eine Ausgangskomponente eines PMMA-Knochenzements und eine Monomerflüssigkeit 2 als weitere Ausgangskomponente des PMMA-Knochenzements in der Vorrichtung enthalten. Das Pulver 1 enthält ein Knochenzementpulver als Hauptbestandteil sowie ein hydrophiles Additiv, mit dem die Monomerflüssigkeit 2 in dem Pulver 1 verteilbar ist. Das Pulver 1 und die Monomerflüssigkeit 2 sind in der Kartusche 46 enthalten, wobei das Pulver 1 in einem vorderen Kartuschenteil mit einem vorderen Innenraum und die Monomerflüssigkeit 2 in einem hinteren Kartuschenteil mit einem hinteren Innenraum der Kartusche 46 angeordnet ist. Gemeinsam begrenzt der hintere Innenraum und der vordere Innenraum einen zylindrischen Innenraum der Kartusche 46.

An der Rückseite der Vorrichtung (in Figur 6 rechts und in Figur 7 nach rechts oben hinten) ist ein Förderkolben 5 angeordnet, der in axialer Richtung linear in Richtung der Vorderseite der Vorrichtung (in Figur 6 links und in Figur 7 nach links unten vorne) im Innenraum der Kartusche 46 vortreibbar ist, beziehungsweise beweglich gelagert ist. Der Förderkolben 5 schließt die Rückseite des Innenraums der Kartusche 46 ab. Im hinteren Ende des vorderen Innenraums, beziehungsweise in der Verbindung vom vorderen Innenraum zum hinteren Innenraum der Kartusche 46 ist ein Austragskolben 6 angeordnet, der in axialer Richtung linear in Richtung der Vorderseite der Vorrichtung im Innenraum der Kartusche 46 vortreibbar ist, beziehungsweise beweglich gelagert ist. Der Austragskolben 6 ist also auch bei dieser Ausführung zwischen dem Pulver 1 und der Monomerflüssigkeit 2 im Innenraum der Kartusche 46 angeordnet.

An der Vorderseite der Vorrichtung ist ein Verschlusssystem angeordnet, mit dem der Innenraum der Kartusche 46 nach vorne abgeschlossen ist, das sich aber zum Austragen eines aus den Ausgangskomponenten 1, 2 gemischten Knochenzementteigs 44 (siehe Figuren 6 E und 6 F) öffnen lässt. Eine Wandung 7 des Verschlusssystems weist eine zentrale kreisrunde Austragsöffnung 48 (siehe Figur 8) auf und ist in axialer Richtung des Innenraums der Kartusche 46 beweglich angeordnet. Die Austragsöffnung 48 ist durch einen inneren Ring begrenzt (siehe Figur 8). Die Wandung 7 ist nach Art eines Rads mit einem äußeren Ring aufgebaut, der mit dem die Austragsöffnung 48 begrenzenden inneren Ring über eine Mehrzahl von Streben verbunden ist. Der innere Ring, der äußere Ring und die Streben sind einteilig aus Kunststoff gefertigt. Ferner umfasst das Verschlusssystem einen Stopfen 8, mit dem die Austragsöffnung 48 im Ausgangszustand, wie er in Figur 6 A gezeigt ist, verschlossen ist.

Die Monomerflüssigkeit 2 ist in einer geschlossenen Ampulle 9 aus Kunststoff oder aus Glas als Behälter 9 für die Monomerflüssigkeit 2 enthalten. Die Ampulle 9 besteht aus Glas oder aus einem Kunststoff, der chemisch gegen die Monomerflüssigkeit 2 beständig ist. Innerhalb der Ampulle 9 kann die Monomerflüssigkeit 2 über lange Zeit in der Vorrichtung gelagert werden.

Das Verschlusssystem der Vorrichtung ist in einem Kartuschenkopf 10 angeordnet, der den vorderen Bereich des Innenraums der Kartusche 46 begrenzt. Genaugenommen ist der Kartuschenkopf 10 ein Teil der Kartusche 46. In dem Kartuschenkopf 10 ist an der Fronseite eine Begasungsöffnung vorgesehen, die im Lagerungszustand beziehungsweise im Ausgangszustand der Vorrichtung mit einer Kappe 12 verschlossen ist. Durch die Begasungsöffnung kann der Innenraum der Kartusche 46 mit einem sterilisierenden Gas, wie Ethylenoxid, begast werden und so der Inhalt der Kartusche 46 sterilisiert werden. Vorzugsweise sind in der Wandung der Kartusche 46 direkt neben der Position des Förderkolbens 5 noch weitere Begasungsöffnungen (nicht gezeigt) analog den Ausführungsbeispielen nach den Figuren 9 bis 18 vorgesehen, die das Innere der Kartusche 46 mit der Umgebung verbinden und durch die der Innenraum der Kartusche 46 von außen mit dem sterilisierenden Gas begast werden kann. Bei einer Bewegung des Förderkolbens 5 in Richtung des Kartuschenkopfs 10 werden diese rückseitigen Begasungsöffnungen durch den Förderkolben 5 verschlossen, so dass keine aus der Ampulle 9 austretende Monomerflüssigkeit 2 aus dem hinteren Teil des Innenraums nach außen dringen kann. Dadurch kann das sterilisierende Gas durch die Kartusche 46 geleitet werden.

Der Stopfen 8 des Verschlusssystems wird von einem Halterungsring 14 gehalten, wobei der Stopfen 8 über Streben des Halterungsrings 14 mit einem äußeren Ring des Halterungsrings 14 verbunden ist. Der Halterungsring 14 ist außen in der Verbindung zwischen dem Kartuschenkopf 10 und der restlichen Kartusche 46 fixiert. Dadurch ist auch der Stopfen 8 gegen die Kartusche 46 fixiert.

Die zwischen den Streben der Wandung 7 des Verschlusssystems offenen Zwischenräume sind durch einen gasdurchlässigen aber für das Pulver 1 und den Knochenzementteig 44 undurchlässigen Filter 16 in Form einer Lochscheibe abgedeckt. Dadurch ist die Wandung 7 für Gase wie Ethylenoxid durchlässig aber für das Pulver 1 und den Knochenzementteig 44 undurchlässig. Analog dem Halterungsring 14 ist die Wandung 7 nämlich durch ein Gerüst in Form des äußeren Rings mit den sich nach Innen zur Austragsöffnung 48 hin radial erstreckenden Speichen geformt und somit durchlässig, wenn deren Zwischenräume nicht durch den Filter 16 abgedeckt wären. Der Aufbau der Wandung 7 und des Halterungsrings 14 ist am besten in Figur 8 zu erkennen. Die Wandung 7 mit dem Filter 16 sowie dem gegen die Kartusche 46 unbeweglich gehaltenen Stopfen 8 und dem Halterungsring 14 bilden ein Verschlusssystem für die Vorrichtung.

Einteilig mit der Wandung 7 ausgeführt, ist an der Austragsöffnung 48, beziehungsweise an dem Inneren Ring der Wandung 7, der die Austragsöffnung 48 begrenzt, ein Austragsrohr 18 angeordnet, durch das bei der Anwendung der Vorrichtung eine Applikation des Knochenzementteigs 44 erfolgt (siehe Figur 6 F). Mit der Wandung 7 wird also auch das Austragsrohr 18 bewegt. Dazu ist das Austragsrohr 18 durch eine Durchführung in dem Kartuschenkopf 10 in Längsrichtung (also in axialer Richtung des zylindrischen Innenraums der Kartusche 46) beweglich gelagert. In der Durchführung sind Nocken 19 als Verengung vorgesehen, so dass das Austragsrohr 18 und damit die Wandung 7 mit dem Filter 16 nur gegen einen Widerstand gegen den Kartuschenkopf 10 bewegt werden kann. In dem Austragsrohr 18 ist eine umlaufende Nut 21 vorgesehen, die mit den Nocken 19 eine lösbare Arretierung des Austragsrohrs 18 und damit der Wandung 7 gegen den Kartuschenkopf 10 und damit gegen die Kartusche 3, 4 bildet. Das Pulver 1 ist unter Druck in den vorderen Teil des Innenraums der Kartusche 46 zwischen der Wandung 7 mit dem Filter 16 und dem Austragskolben 6 eingepresst und steht unter einem elastischen mechanischen Druck. Durch die Arretierung der Nocken 19 mit der umlaufenden Nut 21 in der Durchführung soll vermieden werden, dass der von dem zusammengepressten Pulver 1 auf die Wandung 7 mit dem Filter 16 ausgeübte elastische Druck ausreicht, um die Wandung 7 mit dem Filter 16 in Richtung der Vorderseite der Kartusche 46 beziehungsweise an die Frontseite des durch den Kartuschenkopf 10 begrenzten Innenraums zu drücken und dadurch das Pulver 1 wieder zu entspannen.

An der zum Pulver 1 weisenden Seite des Austragskolbens 6 ist ein für das Pulver 1 undurchlässiger aber für die Monomerflüssigkeit 2 durchlässiger Porenfilter 20 angeordnet. Damit soll verhindert werden, dass das Pulver 1 durch Durchgänge 22, die in dem Austragskolben 6 vorgesehen sind, in den hinteren Teil des Innenraums der Kartusche 3, 4 vordringen kann. Der Porenfilter 20 deckt dabei die Durchgänge 22 ab, so dass das Pulver 1 auch nicht in die Durchgänge 22 vordringen kann. Hierdurch wird vermieden, dass die Monomerflüssigkeit 2 nach dem Öffnen der Ampulle 9 bereits vorzeitig mit Zementpulverpartikeln des Pulvers 1 reagiert, also bevor die Monomerflüssigkeit 2 in den vorderen Teil des Innenraums eingepresst wurde. Dadurch kann verhindert werden, dass die Durchgänge 22 durch anquellenden Knochenzement blockiert werden und dadurch keine weitere Einleitung von Monomerflüssigkeit 2 in das Pulver 1 mehr möglich ist.

Auf der dem Porenfilter 20 gegenüberliegenden Seite des Austragskolbens 6 ist ein Gitter 24 oder Sieb 24 angeordnet, mit dem das Vordringen von Splittern der aufgebrochenen Ampulle 9 in die Durchgänge 22 verhindert wird. Auch hiermit soll sichergestellt werden, dass die Monomerflüssigkeit 2 aus dem hinteren Teil des Innenraums der Kartusche 46 problemlos in das Pulver 1 gedrückt werden kann. Beim Aufbrechen der Ampulle 9 wird zunächst ein Ampullenkopf 26 abgebrochen und dadurch die Ampulle 9 geöffnet (siehe Figur 6 C). Die Monomerflüssigkeit 2 aus der Ampulle 9 kann dann in den vorderen Teil des Innenraums der Kartusche 46 ausfließen und anschließend durch die Durchgänge 22 in das Pulver 1 gepresst werden (siehe Figur 6 D). Dabei wird die Ampulle 9 in derart kleine Splitter zersplittert, dass diese in einen Hohlraum passen, der auf der dem Kartuschenboden zugewandten Seite (in Figur 6 nach rechts und in Figur 7 nach oben rechts hinten) des Austragskolbens 6 gebildet ist.

Am Kartuschenboden ist außen an dem hinteren Kartuschenteil 4 eine Halterung 36 zur Befestigung einer Auspresseinrichtung 40 (siehe Figur 6) vorgesehen.

Der Austragskolben 6 sitzt in Presspassung in dem Innenraum der Kartusche 46. Die beiden umlaufenden Dichtungsringe 28 werden dabei derart stark komprimiert, dass der Austragskolben 6 zunächst in dem Innenraum der Kartusche 46 festsitzt. Die dadurch hervorgerufene Halterung des Austragskolbens 6 mit der Kartusche 46 ist stark genug, um dem Druck der beim Zersplittern der Ampulle 9 auftretenden Kräfte und dem Druck der Monomerflüssigkeit 2 während des Vortriebs des Förderkolbens 5 zu wiederstehen. Erst wenn der Förderkolben 5 unmittelbar an dem Austragskolben 6 anliegt (siehe Figur 6 D), wird die auf den Austragskolben 6 übertragene Kraft so groß, dass die Haftreibung zwischen dem Austragskolben 6 und der Innenwand der Kartusche 46 überwunden wird, so dass anschließend der Austragskolben 6 von dem Förderkolben 5 in Richtung des Kartuschenkopfs 10 an der Vorderseite der Vorrichtung gedrückt wird.

In dem in der Rückseite des Austragskolbens 6 gebildeten Hohlraum im Austragskolben 6 kann vorzugsweise ein Füllmaterial (nicht gezeigt), wie beispielsweise ein Schaumstoffeinsatz und/oder Kunststoffkugeln oder -körner, vorgesehen sein. Damit soll das Volumen der Monomerflüssigkeit 2, die in diesem Hohlraum zurückbleibt und nicht von dem Förderkolben 5 in das Pulver 1 gepresst werden kann, möglichst klein gehalten werden. Des Weiteren kann dieses Füllmaterial als Transportschutz und Stoßschutz für die Ampulle 9 verwendet werden, damit die Ampulle 9 beim Transport der Vorrichtung im Ausgangszustand (siehe Figur 6 A) nicht aus Versehen aufbricht. Zum gleichen Zweck kann auch der Ampullenkörper der Ampulle 9 im hinteren Teil des Innenraums der Kartusche 46 in einen zusammenpressbaren Schaumstoff gehüllt sein.

Der Ablauf eines beispielhaften Verfahrens ist in Figur 6 durch sechs übereinander gezeichnete Querschnittansichten Figur 6 A bis Figur 6 F dargestellt. Figur 6 A zeigt die Vorrichtung im Ausgangszustand, in dem die Ausgangskomponenten 1, 2 gelagert und aufbewahrt werden. In diesem Zustand kann dieser ausgeliefert werden. Zunächst wird die Vorrichtung in eine Auspresseinrichtung 40 eingesetzt, dazu wird die Kartusche 46 mit der Halterung 36 an einem passenden Gegenstück 41 der Auspresseinrichtung 40 befestigt (siehe Figur 6 B).

Anschließend wird ein Stößel 42 der Auspresseinrichtung 40 gegen das Gegenstück 41 vorgetrieben. Der Stößel 42 liegt an dem Förderkolben 5 an. Dadurch wird der Förderkolben 5 von dem Stößel 42 in Richtung des Austragskolbens 6 gedrückt. Durch die Bewegung des Förderkolbens 5 wird die Ampulle 9 gegen den in Presspassung festsitzenden Austragskolben 6 gedrückt. Der Ampullenkopf 26 bricht ab und die Ampulle 9 ist geöffnet (siehe Figur 6 C).

Die Vorrichtung in der Auspresseinrichtung 40 wird dabei bevorzugt mit dem Kartuschenkopf 10 nach oben gehalten, so dass bei einem nachfolgenden weiteren Vortreiben des Förderkolbens 5 zunächst die überstehende Luft aus dem hinteren Teil des Innenraums nach oben durch das Pulver 1 und durch den gasdurchlässigen Filter 16 nach außen gedrückt wird. Irgendwann wird die Monomerflüssigkeit 2 aus der Ampulle 9 von dem Förderkolben 5 durch das Gitter 24 und/oder das Sieb 24, durch die Durchgänge 22 und durch den Porenfilter 20 in den vorderen Teil des Innenraums in das Pulver 1 gedrückt. Dabei wird die Ampulle 9 weiter zusammengedrückt und zersplittert dabei in kleinere Splitter, die sich schließlich in dem rückseitigen Hohlraum des Austragskolbens 6 sammeln. Das Pulver 1 enthält ein hydrophiles Additiv, das eine große Oberflächenenergie bezüglich der wässrigen Monomerflüssigkeit 2 aufweist, die größer ist als die des Knochenzementpulvers. Gleichzeitig sind die Kapillarkräfte aufgrund des komprimierten Pulvers 1 groß, da die Zwischenräume zwischen den Pulverpartikeln klein sind. Zudem wird die Monomerflüssigkeit 2 mit Druck in das Pulver 1 gepresst. Durch all diese Maßnahmen wird die Monomerflüssigkeit 2 schnell in und durch das Pulver 1 geleitet und kann sich vollständig in dem Pulver 1 ausbreiten und verteilen, bevor die anquellenden Zementpulverpartikel eine weitere Ausbreitung der Monomerflüssigkeit 2 im Pulver 1 unterbinden. Schließlich trifft der Förderkolben 5 auf den Austragskolben 6 (siehe Figur 6 D).

Das Zementpulver in dem Pulver 1 reagiert mit der Monomerflüssigkeit 2 und bildet dort den Knochenzementteig 44. Um dabei das gewünschte Mischungsverhältnis zwischen Pulver 1 und Monomerflüssigkeit 2 im Knochenzementteig 44 zu erhalten, kann überschüssige Monomerflüssigkeit 2 an der Vorderseite der Kartusche 46 zwischen dem porösen Filter 16 der Wandung 7 und dem Kartuschenkopf 10 aufgenommen werden. Die Monomerflüssigkeit 2 wird dazu durch den porösen Filter 16 gedrückt, der für das Pulver 1 und den Knochenzementteig 44 undurchlässig ist. Durch Aufnahme der überschüssigen Monomerflüssigkeit 2 nach dem Durchtritt der Monomerflüssigkeit 2 durch das Pulver 1 bis zur Wandung 7 wird verhindert, dass der Knochenzementteig 44 zu dünnflüssig wird und dadurch eine unerwünschte Konsistenz erhält. Zudem wird zur Vermeidung einer zu dickflüssigen Konsistenz des Knochenzementteigs 44 die Monomerflüssigkeit 2 im Überschuss verwendet, so dass die Verluste durch die zwischen dem Austragskolben 6 und dem Förderkolben 5 sowie in den Durchgängen 22 des Austragskolbens 6 verbleibende Reste der Monomerflüssigkeit 2 ausgeglichen werden.

Durch einen weiteren Vortrieb des Förderkolbens 5 wird der Austragskolben 6 in Richtung des Kartuschenkopfs 10 getrieben, wobei der von dem Förderkolben 5 auf den Austragskolben 6 ausgeübte Druck dazu ausreicht, die Haftreibung des Austragskolbens 6 gegen die Wand des Innenraums zu überwinden und dadurch den Austragskolben 6 in Richtung des Kartuschenkopfs 10 vorzutreiben. Durch die Bewegung des Austragskolbens 6 in Richtung des Kartuschenkopfs 10 wird vom Knochenzementteig 44 ein Druck auf die Wandung 7 und den Filter 16 des Verschlusssystems ausgeübt. Der Knochenzementteig 44 kann nicht durch den Filter 16 fließen, so dass der Druck des Knochenzementteigs 44 auf den Filter 16 und die Wandung 7 wirkt. Durch diesen Druck wird die Arretierung zwischen den Nocken 19 und der umlaufenden Nut 21 in dem Austragsrohr 18 gelöst. Da die Wandung 7, der Filter 16 und das Austragsrohr 18 nun beweglich gegen die Kartusche 46 beziehungsweise den Kartuschenkopf 10 gelagert sind, werden diese Teile des Verschlusssystems nach vorne gedrückt, während der Stopfen 8, der über den Halterungsring 14 fest mit der Kartusche 46 verbunden ist, nicht mit bewegt wird. Dabei fließt der Knochenzementteig 44 durch die Öffnungen zwischen den Speichen des Halterungsrings 14 hindurch. Währenddessen wird die Austragsöffnung 48 in der Wandung 7 geöffnet. Die Wandung 7, der Filter 16 und das Austragsrohr 18 werden so weit nach vorne getrieben, bis die Vorderseite der Wandung 7 an der frontalen Innenseite des Kartuschenkopfs 10 anliegt. Diese Situation ist in Figur 6 E dargestellt. Die Kartusche 46 ist nun nach außen geöffnet. Der Abstand zwischen der Wandung 7 und dem Stopfen 8 ist, wenn die Wandung 7 an der Frontseite des Innenraums anliegt, so groß, dass die freie Fläche, durch die der Knochenzementteig 44 ausfließt, mindestens so groß ist, wie die Querschnittsfläche des Austragsrohrs 18 beziehungsweise der Austragsöffnung 48, damit der Fluss des Knochenzementteigs 44 möglichst wenig behindert wird.

Durch weiteres Vortreiben des Förderkolbens 5 und damit des Austragskolbens 6 wird der fertige Knochenzementteig 44 durch die Austragsöffnung 48 und das Austragsrohr 18 nach außen gepresst und kann appliziert werden (siehe Figur 6 F).

Aufgrund des in dem Pulver 1 vorhandenen Additivs gelingt es, die Monomerflüssigkeit 2 an einer Grundfläche des vorderen Teils des zylindrischen Innenraums der Kartusche 46 einzupressen und dennoch eine vollständige Verteilung der Monomerflüssigkeit 2 in dem Pulver 1 zu erreichen. Durch den Aufbau der Vorrichtung gelingt es, eine herkömmliche Auspresseinrichtung 40 verwenden zu können und durch eine unidirektionale lineare Bewegung des Stößels 42 sowohl den Behälter 9 für die Monomerflüssigkeit 2 zu öffnen, die Monomerflüssigkeit 2 in das Pulver 1 zu pressen und dadurch den Knochenzementteig 44 zu mischen, als auch das Verschlusssystem zu öffnen und den gemischten Knochenzementteig 44 auszutreiben und zu applizieren. Mit dem Aufbau des Verschlusssystems gelingt es, die Kraft, die durch den Stößel 42 auf den Förderkolben 5 ausgeübt wird, zum Öffnen der Austragsöffnung 48 nutzen können.

In den Figuren 9 bis 13 ist eine dritte beispielhafte Vorrichtung gezeigt, die mit einem erfindungsgemäßen Verfahren hergestellt wurde und die sich von der ersten beispielhaften Vorrichtung nach den Figuren 1 bis 5 vor allem dadurch unterscheidet, dass die dritte beispielhafte Vorrichtung eine Kartusche 50 aufweist, die nicht aus zwei Kartuschenteilen besteht, wobei der Kartuschenkopf 10 noch immer aufgeschraubt ist, dass ein Füllmaterial 52 in einem rückseitigen Hohlraum des Austragskolbens 6 vorgesehen ist und dass das Verschlusssystem anders aufgebaut ist.

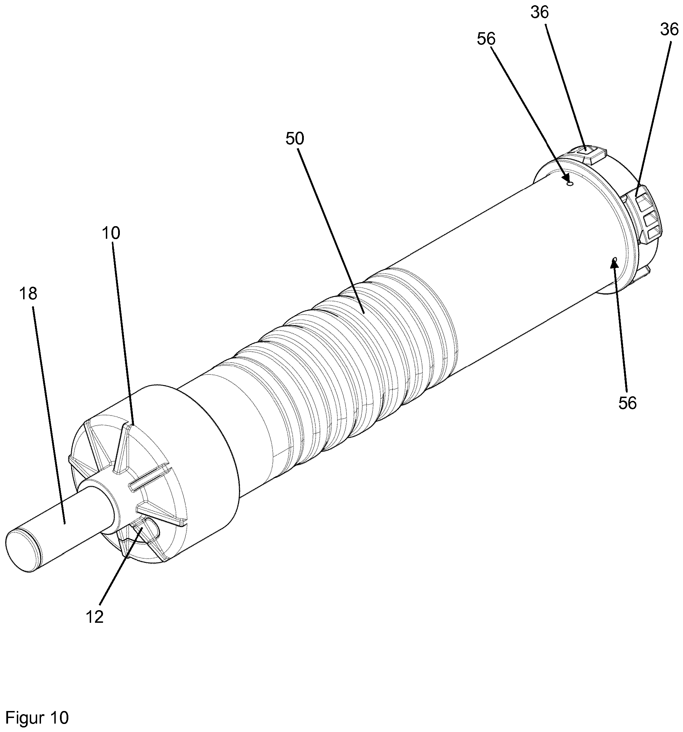

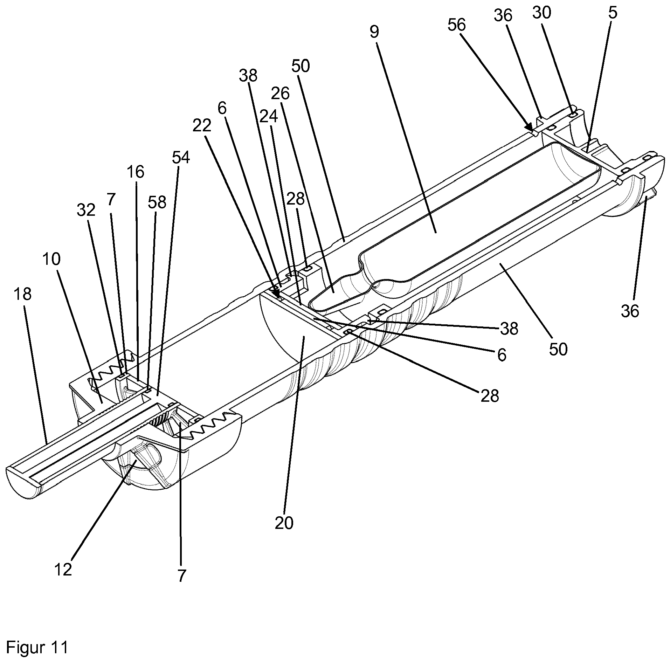

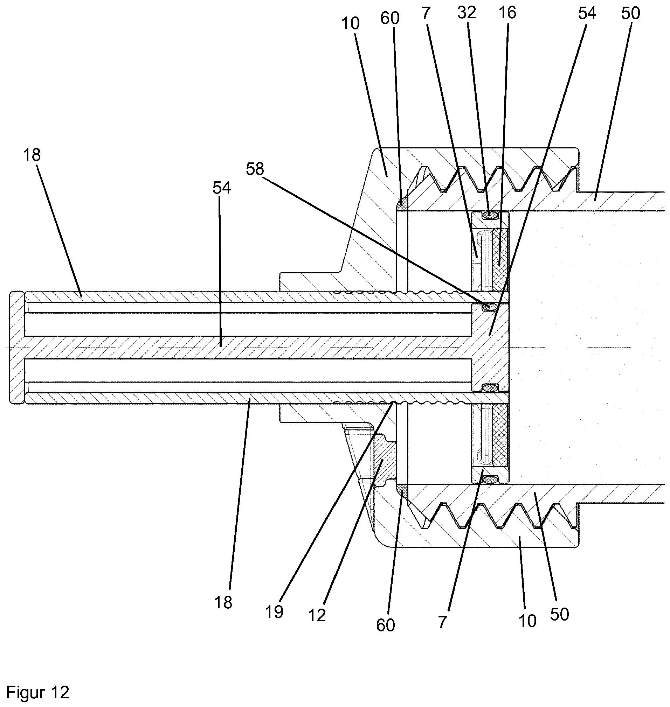

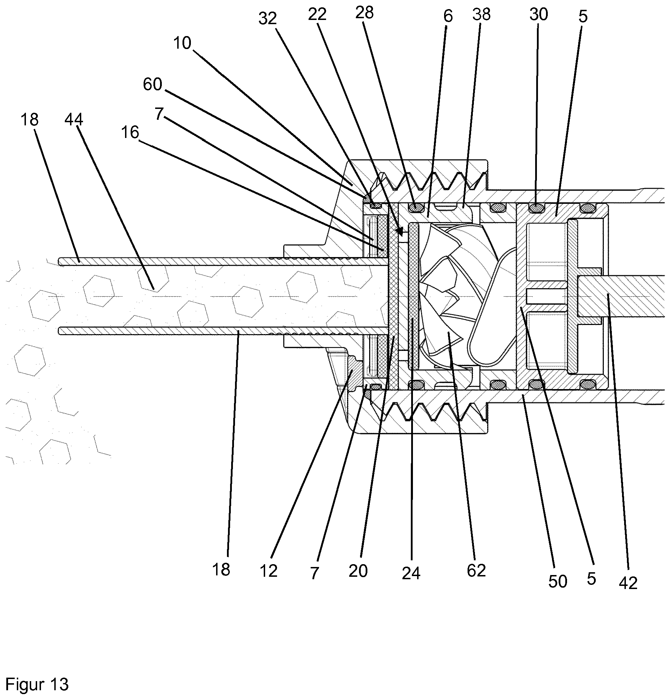

Dabei zeigt Figur 9 sechs schematische Querschnittansichten A bis F der dritten beispielhaften Vorrichtung, die den Ablauf der Anwendung der Vorrichtung während der Herstellung und der Applikation des Knochenzementteigs zeigen, Figur 10 eine perspektivische Außenansicht der Vorrichtung, Figur 11 eine perspektivische Querschnittansicht der Vorrichtung im Ausgangszustand, Figur 12 eine Ausschnittvergrößerung des alternativen Verschlusssystems als schematische Teilquerschnittansicht der Vorrichtung im Ausgangszustand und Figur 13 eine Ausschnittvergrößerung des alternativen Verschlusssystems als schematische Teilquerschnittansicht der dritten beispielhaften Vorrichtung nach dem Auspressen des Knochenzementteigs 44.

Der Aufbau und die Funktionsweise der dritten beispielhaften Vorrichtung entsprechen weitgehend denen der ersten beispielhaften Vorrichtung, so dass auch weitgehend auf die Figurenbeschreibung des ersten Ausführungsbeispiels verwiesen werden kann. Insbesondere sind das verwendete Pulver 1 und im Wesentlichen die Funktionsweise der beiden Kolben 5, 6 identisch.

Im Ausgangszustand der Vorrichtung sind ein Pulver 1 als eine Ausgangskomponente eines PMMA-Knochenzements und eine Monomerflüssigkeit 2 als weitere Ausgangskomponente des PMMA-Knochenzements in der Vorrichtung enthalten. Das Pulver 1 enthält ein Knochenzementpulver als Hauptbestandteil sowie ein hydrophiles Additiv, mit dem die Monomerflüssigkeit 2 in dem Pulver 1 verteilbar ist. Das Pulver 1 und die Monomerflüssigkeit 2 sind in einer Kartusche 50 enthalten, wobei das Pulver 1 in einem vorderen Teil des Innenraums der Kartusche 50 und die Monomerflüssigkeit 2 in einem hinteren Teil des Innenraums der Kartusche 50 angeordnet ist. Gemeinsam begrenzt der hintere Innenraum und der vordere Innenraum einen zylindrischen Innenraum der Kartusche 50.

An der Rückseite der Vorrichtung (in Figur 9 rechts und in den Figuren 10 und 11 nach rechts oben hinten) ist ein Förderkolben 5 angeordnet, der in axialer Richtung linear in Richtung der Vorderseite der Vorrichtung (in Figur 9 links und in den Figuren 10 und 11 nach links unten vorne) im Innenraum der Kartusche 50 vortreibbar ist, beziehungsweise beweglich gelagert ist. Der Förderkolben 5 schließt die Rückseite des Innenraums der Kartusche 50 ab. Im hinteren Ende des vorderen Innenraums, beziehungsweise in der Verbindung vom vorderen Innenraum zum hinteren Innenraum der Kartusche 50 ist ein Austragskolben 6 angeordnet, der in axialer Richtung linear in Richtung der Vorderseite der Vorrichtung im Innenraum der Kartusche 50 vortreibbar ist, beziehungsweise beweglich gelagert ist. Der Austragskolben 6 ist also zwischen dem Pulver 1 und der Monomerflüssigkeit 2 im Innenraum der Kartusche 50 angeordnet.

An der Vorderseite der Vorrichtung ist ein Verschlusssystem angeordnet, mit dem der Innenraum der Kartusche 50 nach vorne abgeschlossen ist, das sich aber zum Austragen eines aus den Ausgangskomponenten 1, 2 gemischten Knochenzementteigs 44 (siehe Figuren 9 E und 9 F) öffnen lässt. Eine Wandung 7 des Verschlusssystems weist eine zentrale kreisrunde Austragsöffnung auf und ist in axialer Richtung des vorderen Innenraums der Kartusche 50 beweglich angeordnet. Die Austragsöffnung ist durch einen inneren Ring begrenzt. Die Wandung 7 ist nach Art eines Rads mit einem äußeren Ring aufgebaut, der mit dem die Austragsöffnung begrenzenden inneren Ring über eine Mehrzahl von Streben verbunden ist, was andeutungsweise in Figur 11 zu erkennen ist. Der innere Ring, der äußere Ring und die Streben sind einteilig aus Kunststoff gefertigt. Ferner umfasst das Verschlusssystem einen Stopfen 54, mit dem die Austragsöffnung im Ausgangszustand, wie er in Figur 9 A und Figur 11 gezeigt ist, verschlossen ist.

Die Monomerflüssigkeit 2 ist in einer geschlossenen Ampulle 9 als Behälter 9 für die Monomerflüssigkeit 2 enthalten. Die Ampulle 9 besteht aus Glas oder aus einem Kunststoff, der chemisch gegen die Monomerflüssigkeit 2 beständig ist. Innerhalb der Ampulle 9 kann die Monomerflüssigkeit 2 über lange Zeit in der Vorrichtung gelagert werden.

Das Verschlusssystem der Vorrichtung ist in einem Kartuschenkopf 10 angeordnet, der den vorderen Bereich des vorderen Innenraums der Kartusche 50 begrenzt. Genaugenommen ist der Kartuschenkopf 10 ein Teil der Kartusche 50. In dem Kartuschenkopf 10 ist an der Fronseite eine Begasungsöffnung vorgesehen, die im Lagerungszustand beziehungsweise im Ausgangszustand der Vorrichtung mit einer Kappe 12 verschlossen ist. Durch die Begasungsöffnung kann der Innenraum der Kartusche 50 mit einem sterilisierenden Gas, wie Ethylenoxid, begast werden und so der Inhalt der Kartusche 50 sterilisiert werden. In der Wandung am hinteren Ende der Kartusche 50 direkt neben der Position des Förderkolbens 5 sind vier Begasungsöffnungen 56 vorgesehen, die das Innere der Kartusche 50 mit der Umgebung verbinden und durch die der Innenraum der Kartusche 50 von außen mit dem sterilisierenden Gas begast werden kann. Bei einer Bewegung des Förderkolbens 5 in Richtung des Kartuschenkopfs 10 werden diese rückseitigen Begasungsöffnungen 56 durch den Förderkolben 5 verschlossen, so dass keine aus der Ampulle 9 austretende Monomerflüssigkeit 2 aus dem hinteren Teil des Innenraums nach außen dringen kann. Dadurch kann das sterilisierende Gas durch die vordere Begasungsöffnung im Kartuschenkopf 10 und durch die hinteren Begasungsöffnungen 56 durch die Kartusche 50 geleitet werden.

Die zwischen den Streben der Wandung 7 des Verschlusssystems offenen Zwischenräume sind durch einen gasdurchlässigen aber für das Pulver 1 und den Knochenzementteig 44 undurchlässigen Filter 16 in Form einer Lochscheibe abgedeckt. Dadurch ist die Wandung 7 für Gase wie Ethylenoxid durchlässig aber für das Pulver 1 und den Knochenzementteig 44 undurchlässig. Die Wandung 7 ist durch ein Gerüst in Form des äußeren Rings mit den sich nach Innen zur Austragsöffnung hin radial erstreckenden Speichen geformt und somit durchlässig, wenn deren Zwischenräume nicht durch den Filter 16 abgedeckt wären. Der Aufbau der Wandung 7 ist am besten in Figur 11 zu erahnen entspricht aber dem der Wandungen nach den ersten beiden Ausführungsbeispielen.

Einteilig mit der Wandung 7 ausgeführt, ist an der Austragsöffnung, beziehungsweise an dem Inneren Ring der Wandung 7, der die Austragsöffnung begrenzt, ein Austragsrohr 18 angeordnet, durch das bei der Anwendung der Vorrichtung eine Applikation des Knochenzementteigs 44 erfolgt (siehe Figur 9 F). Das Austragsrohr 18 mündet also in die Austragsöffnung im Inneren der Kartusche 50. Mit der Wandung 7 ist das Austragsrohr 18 beweglich in einer Durchführung im Kartuschenkopf 10 angeordnet. Dadurch kann das Pulver 1 im vorderen Teil des Innenraums der Kartusche 50 mit Hilfe der Wandung 7 komprimiert werden, in dem die Wandung 7 mit Hilfe das Austragsrohrs 18 ins Innere der Kartusche 50 gedrückt wird. Das Austragsrohr 18 wird gegen eine Rückstellung über eine Presspassung mit der Durchführung in dem Kartuschenkopf 10 gehalten. Das Austragsrohr 18 wird gegen eine Rückstellung über Nocken 19 in der Durchführung und eine Vielzahl von übereinander außen in dem Austragsrohr 18 angeordneten Rillen in dem Kartuschenkopf 10 gehalten. Das Austragsrohr 18 ist durch eine Durchführung in dem Kartuschenkopf 10 in Längsrichtung (also in axialer Richtung des zylindrischen Innenraums der Kartusche 50) beweglich gelagert. Durch die Nocken 19 und die Rillen kann das Austragsrohr und damit die Wandung 7 in verschiedenen Stellungen unterschiedlich tief in die Kartusche 50 eingedrückt werden und damit ein Druck auf das Pulver 1 ausgeübt werden. Das Pulver 1 ist unter Druck in den vorderen Teil des Innenraums der Kartusche 50 zwischen der Wandung 7 mit dem Filter 16 und dem Austragskolben 6 eingepresst und steht unter einem elastischen mechanischen Druck. Durch die Arretierung der Rillen mit den Nocken 19 in der Durchführung soll vermieden werden, dass der von dem zusammengepressten Pulver 1 auf die Wandung 7 mit dem Filter 16 ausgeübte elastische Druck ausreicht, um die Wandung 7 mit dem Filter 16 in Richtung der Vorderseite der Kartusche 50 beziehungsweise an die Frontseite des durch den Kartuschenkopf 10 begrenzten Innenraums zu drücken und dadurch das Pulver 1 wieder zu entspannen. Die Wandung 7 ist mit einer umlaufenden Dichtung 32 gegen die Innenwand der Kartusche 50 abgedichtet. Zusätzlich ist in der Verbindung zwischen dem aufgeschraubten Kartuschenkopf 10 und der Kartusche 50 mit einem umlaufenden Dichtungsring 60 abgedichtet. Die Dichtung 60 verhindert, dass Knochenzementteig 44 zwischen dem Kartuschenkopf 10 und der Kartusche 50 nach außen gepresst wird.

Der Stopfen 54, mit dem die Austragsöffnung verschlossen ist, steckt in dem Austragsrohr 18 und verschließt damit die Kartusche 50 nach außen. Der Stopfen 54 ist beweglich in dem Austragsrohr 18 angeordnet und kann von Innen aus dem Austragsrohr 18 herausgedrückt werden.

Die Wandung 7 mit dem Filter 16 sowie dem dagegen beweglichen gehaltenen Stopfen 54 bilden ein Verschlusssystem für die Vorrichtung nach dem dritten Ausführungsbeispiel.

An der zum Pulver 1 weisenden Seite des Austragskolbens 6 ist ein für das Pulver 1 undurchlässiger aber für die Monomerflüssigkeit 2 durchlässiger Porenfilter 20 angeordnet. Damit soll verhindert werden, dass das Pulver 1 durch Durchgänge 22, die in dem Austragskolben 6 vorgesehen sind, in den hinteren Teil des Innenraums der Kartusche 50 vordringen kann. Der Porenfilter 20 deckt dabei die Durchgänge 22 ab, so dass das Pulver 1 auch nicht in die Durchgänge 22 vordringen kann. Hierdurch wird vermieden, dass die Monomerflüssigkeit 2 nach dem Öffnen der Ampulle 9 bereits vorzeitig mit Zementpulverpartikeln des Pulvers 1 reagiert, also bevor die Monomerflüssigkeit 2 in den vorderen Teil des Innenraums eingepresst wurde. Dadurch kann verhindert werden, dass die Durchgänge 22 durch anquellenden Knochenzement blockiert werden und dadurch keine weitere Einleitung von Monomerflüssigkeit 2 in das Pulver 1 mehr möglich ist.

Auf der dem Porenfilter 20 gegenüberliegenden Seite des Austragskolbens 6 ist ein Gitter 24 oder Sieb 24 angeordnet, mit dem das Vordringen von Splittern 62 der aufgebrochenen Ampulle 9 in die Durchgänge 22 verhindert wird. Auch hiermit soll sichergestellt werden, dass die Monomerflüssigkeit 2 aus dem hinteren Teil des Innenraums der Kartusche 50 problemlos in das Pulver 1 gedrückt werden kann. Beim Aufbrechen der Ampulle 9 wird zunächst ein Ampullenkopf 26 abgebrochen und dadurch die Ampulle 9 geöffnet (siehe Figur 9 B). Die Monomerflüssigkeit 2 aus der Ampulle 9 kann dann in den vorderen Teil des Innenraums der Kartusche 50 ausfließen und anschließend durch die Durchgänge 22 in das Pulver 1 gepresst werden (siehe Figur 9 C). Dabei wird die Ampulle 9 in derart kleine Splitter 62 zersplittert, dass diese in einen Hohlraum passen, der auf der dem Kartuschenboden zugewandten Seite (in Figur 9 nach rechts und in den Figuren 10 und 11 nach oben rechts hinten) des Austragskolbens 6 gebildet ist.

Am Kartuschenboden ist außen an der Kartusche 50 eine Halterung 36 zur Befestigung einer Auspresseinrichtung 40 (siehe Figur 9) vorgesehen.

An dem Austragskolben 6 sind seitlich mehrere Haken 38 als Rastmittel vorgesehen, die in eine dazu passende umlaufende Nut in der Wand des Innenraums der Kartusche 50 greifen. Die dadurch hervorgerufene Rastung des Austragskolbens 6 mit der Kartusche 50 ist stark genug, um dem Druck der beim Zersplittern der Ampulle 9 auftretenden Kräfte, um dem Druck der Monomerflüssigkeit 2 während des Vortriebs des Förderkolbens 5 zu wiederstehen und um dem Druck, der von dem Pulver 1 auf den Austragskolben 6 mittels der eingepressten Wandung 7 ausgeübt wird, standzuhalten. Erst wenn der Förderkolben 5 unmittelbar an dem Austragskolben 6 anliegt (siehe Figur 9 C), wird die Rastung gelöst, beziehungsweise werden die Haken 38 verformt und gleiten aus der Nut in der Wand des Innenraums der Kartusche 50, und anschließend wird der Austragskolben 6 von dem Förderkolben 5 in Richtung des Kartuschenkopfs 10 an der Vorderseite der Vorrichtung gedrückt.

In dem in der Rückseite des Austragskolbens 6 gebildeten Hohlraum im Austragskolben 6 kann vorzugsweise ein Füllmaterial (nicht gezeigt) vorgesehen sein, wie beispielsweise ein Schaumstoffeinsatz und/oder Kunststoffkugeln oder -körner. Damit soll das Volumen der Monomerflüssigkeit 2, die in diesem Hohlraum zurückbleibt und nicht von dem Förderkolben 5 in das Pulver 1 gepresst werden kann, möglichst klein gehalten werden. Des Weiteren kann dieses Füllmaterial als Transportschutz und Stoßschutz für die Ampulle 9 verwendet werden, damit die Ampulle 9 beim Transport der Vorrichtung im Ausgangszustand (siehe Figuren 9 A und 11) nicht aus Versehen aufbricht. Hierzu kann ein komprimierbarer Schaumstoff zusätzlich um die Ampulle 9 herum im Innenraum der Kartusche 50 angeordnet sein.

Der Ablauf eines beispielhaften Verfahrens ist in Figur 9 durch sechs übereinander gezeichnete Querschnittansichten Figur 9 A bis Figur 9 F dargestellt. Zunächst wird die Vorrichtung in eine Auspresseinrichtung 40 eingesetzt, dazu wird die Kartusche 50 mit der Halterung 36 an einem passenden Gegenstück 41 der Auspresseinrichtung 40 befestigt (siehe Figur 9 A).

Anschließend wird ein Stößel 42 der Auspresseinrichtung 40 gegen das Gegenstück 41 vorgetrieben. Der Stößel 42 liegt an dem Förderkolben 5 an. Dadurch wird der Förderkolben 5 von dem Stößel 42 in Richtung des Austragskolbens 6 gedrückt. Durch die Bewegung des Förderkolbens 5 wird die Ampulle 9 gegen den mit dem Rastmittel 38 arretierten Austragskolben 6 gedrückt. Der Ampullenkopf 26 bricht ab und die Ampulle 9 ist geöffnet (siehe Figur 9 B).

Die Vorrichtung in der Auspresseinrichtung 40 wird dabei bevorzugt mit dem Kartuschenkopf 10 nach oben gehalten, so dass bei einem nachfolgenden weiteren Vortreiben des Förderkolbens 5 zunächst die überstehende Luft aus dem hinteren Teil des Innenraums nach oben durch das Pulver 1 und durch den gasdurchlässigen Filter 16 nach außen gedrückt wird. Irgendwann wird die Monomerflüssigkeit 2 aus der Ampulle 9 von dem Förderkolben 5 durch das Gitter 24 und/oder das Sieb 24, durch die Durchgänge 22 und durch den Porenfilter 20 in den vorderen Teil des Innenraums in das Pulver 1 gedrückt. Dabei wird die Ampulle 9 weiter zusammengedrückt und zersplittert dabei in kleinere Splitter 62, die sich schließlich in dem rückseitigen Hohlraum des Austragskolbens 6 sammeln. Das Pulver 1 enthält ein hydrophiles Additiv, das eine große Oberflächenenergie bezüglich der wässrigen Monomerflüssigkeit 2 aufweist, die größer ist als die des Knochenzementpulvers. Gleichzeitig sind die Kapillarkräfte aufgrund des komprimierten Pulvers 1 groß, da die Zwischenräume zwischen den Pulverpartikeln klein sind. Zudem wird die Monomerflüssigkeit 2 mit Druck in das Pulver 1 gepresst. Durch all diese Maßnahmen wird die Monomerflüssigkeit 2 schnell in und durch das Pulver 1 geleitet und kann sich vollständig in dem Pulver 1 ausbreiten und verteilen, bevor die anquellenden Zementpulverpartikel eine weitere Ausbreitung der Monomerflüssigkeit 2 im Pulver 1 unterbinden. Schließlich trifft der Förderkolben 5 auf den Austragskolben 6 (siehe Figur 9 C).

Das Zementpulver in dem Pulver 1 reagiert mit der Monomerflüssigkeit 2 und bildet dort den Knochenzementteig 44. Um dabei das gewünschte Mischungsverhältnis zwischen Pulver 1 und Monomerflüssigkeit 2 im Knochenzementteig 44 zu erhalten, kann überschüssige Monomerflüssigkeit 2 an der Vorderseite der Kartusche 50 zwischen dem porösen Filter 16 der Wandung 7 und dem Kartuschenkopf 10 aufgenommen werden. Die Monomerflüssigkeit 2 wird dazu durch den porösen Filter 16 gedrückt, der für das Pulver 1 und den Knochenzementteig 44 undurchlässig ist. Durch Aufnahme der überschüssigen Monomerflüssigkeit 2 nach dem Durchtritt der Monomerflüssigkeit 2 durch das Pulver 1 bis zur Wandung 7 wird verhindert, dass der Knochenzementteig 44 zu dünnflüssig wird und dadurch eine unerwünschte Konsistenz erhält. Zudem wird zur Vermeidung einer zu dickflüssigen Konsistenz des Knochenzementteigs 44 die Monomerflüssigkeit 2 im Überschuss verwendet, so dass die Verluste durch die zwischen dem Austragskolben 6 und dem Förderkolben 5 sowie in den Durchgängen 22 des Austragskolbens 6 verbleibende Reste der Monomerflüssigkeit 2 ausgeglichen werden.

Durch einen weiteren Vortrieb des Förderkolbens 5 wird der Austragskolben 6 in Richtung des Kartuschenkopfs 10 getrieben und die Rastung beziehungsweise das Rastmittel 38 gelöst. Durch die Bewegung des Austragskolbens 6 in Richtung des Kartuschenkopfs 10 wird vom Knochenzementteig 44 ein Druck auf die Wandung 7, den Filter 16 und den Stopfen 54 in der Austragsöffnung des Verschlusssystems ausgeübt. Der Knochenzementteig 44 kann nicht durch den Filter 16 fließen, so dass der Druck des Knochenzementteigs 44 auf den Filter 16 und die Wandung 7 wirkt.

Da die Wandung 7, der Filter 16 und das Austragsrohr 18 sind nach dem Lösen der Arretierung zwischen den Nocken 19 und den Rillen im Außenumfang des Austragsrohrs 18 zunächst beweglich gegen die Kartusche 50 beziehungsweise den Kartuschenkopf 10 gelagert. Dadurch werden diese Teile des Verschlusssystems zusammen mit dem Stopfen 54 nach vorne gedrückt, bis die Wandung 7 von Innen an dem Kartuschenkopf 10 anliegt und dadurch gegen den Kartuschenkopf 10 und damit der Kartusche 50 fixiert ist (siehe Figur 9 D). Wenn also die Wandung 7 innen an dem Kartuschenkopf 10 anliegt, so gilt diese im Sinne der vorliegenden Patentanmeldung als gegen die Kartusche 50 fixiert. Der Stopfen 54 steckt derart fest in dem Austragsrohr 18, dass der Druck des Knochenzementteigs 44 nicht ausreicht, den Stopfen 54 gegen das Austragsrohr 18 zu bewegen, wenn er bereits ausreicht, um das Verschlusssystem 7, 16, 54 als Ganzes in Richtung des Kartuschenkopfs 10 zu verschieben. In anderen Worten ist der Druck des Knochenzementteigs 44, der notwendig ist, um die Wandung 7 gegen die Kartusche 50 zu bewegen, beziehungsweise um die Haftreibung der Wandung 7 gegen die Kartusche 50 zu überwinden, geringer als der Druck des Knochenzementteigs 44, der notwendig ist, um den Stopfen 54 gegen die Wandung 7 und das Austragsrohr 18 zu bewegen, beziehungsweise um die Haftreibung des Stopfens 54 gegen das Austragsrohr 18 zu überwinden.

Sobald die Wandung 7 innen am Kartuschenkopf 10 anliegt und der Förderkolben 5 zusammen mit dem daran anliegenden Austragskolben 6 weiter in Richtung des Kartuschenkopfs 10 vorgetrieben wird, wird der Stopfen 54 nach vorne aus dem Austragsrohr 18 herausgedrückt (siehe Figur 9 E). Während also die Wandung 7, die über den Anpressdruck des Knochenzementteigs 44 fest an der Vorderseite der Kartusche 50 anliegt, nicht mit dem Knochenzementteig 44 bewegt wird, wird der Stopfen 54 gegen die Wandung 7 bewegt und damit aus der Austragsöffnung herausgetrieben und so die Austragsöffnung in der Wandung 7 geöffnet. Schließlich fällt der Stopfen 54 vorne aus dem Austragsrohr 18 hinaus und der Knochenzementteig 44 tritt aus dem Austragsrohr 18 aus. Die Kartusche 50 ist nun nach außen geöffnet. Durch weiteres Vortreiben des Förderkolbens 5 und damit des Austragskolbens 6 wird der fertige Knochenzementteig 44 durch die Austragsöffnung und das Austragsrohr 18 nach außen gepresst und kann appliziert werden (siehe Figur 9 F).

Aufgrund des in dem Pulver 1 vorhandenen Additivs gelingt es, die Monomerflüssigkeit 2 an einer Grundfläche des vorderen Teils des zylindrischen Innenraums der Kartusche 50 einzupressen und dennoch eine vollständige Verteilung der Monomerflüssigkeit 2 in dem Pulver 1 zu erreichen. Durch den Aufbau der Vorrichtung gelingt es, eine herkömmliche Auspresseinrichtung 40 verwenden zu können und durch eine unidirektionale lineare Bewegung des Stößels 42 sowohl den Behälter 9 für die Monomerflüssigkeit 2 zu öffnen, die Monomerflüssigkeit 2 in das Pulver 1 zu pressen und dadurch den Knochenzementteig 44 zu mischen, als auch das Verschlusssystem zu öffnen und den gemischten Knochenzementteig 44 auszutreiben und zu applizieren. Mit dem Aufbau des Verschlusssystems gelingt es, die Kraft, die durch den Stößel 42 auf den Förderkolben 5 ausgeübt wird, zum Öffnen der Austragsöffnung nutzen können.

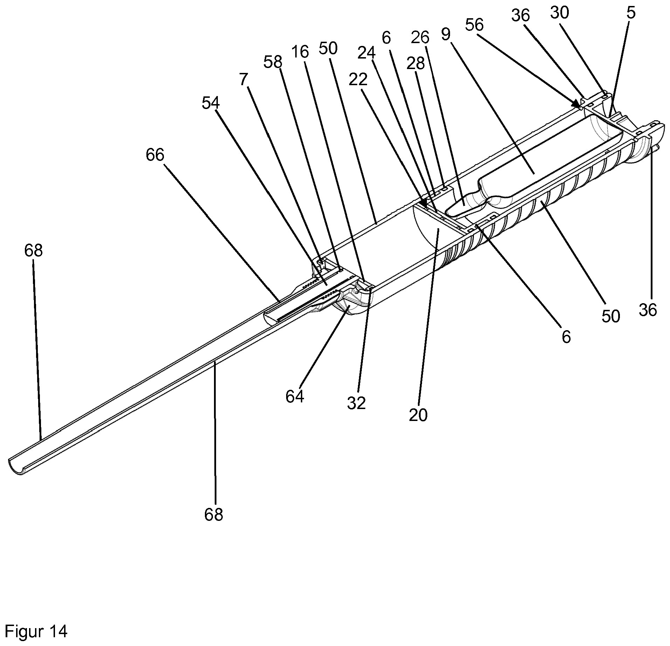

In den Figuren 14 bis 16 ist eine vierte beispielhafte Vorrichtung gezeigt, die mit einem erfindungsgemäßen Verfahren hergestellt wurde und die sich von der ersten beispielhaften Vorrichtung nach den Figuren 1 bis 5 dadurch unterscheidet, dass die vierte beispielhafte Vorrichtung analog der zweiten und der dritten beispielhaften Vorrichtung eine Kartusche 50 aufweist, die nicht aus zwei Kartuschenteilen besteht, wobei hier im Gegensatz zur zweiten und dritten Ausführung auch ein Kartuschenkopf 64 der Kartusche 50 einteilig mit der Kartusche 50 ausgeführt ist. Ferner weist die vierte beispielhafte Vorrichtung analog der zweiten beispielhaften Vorrichtung nach den Figuren 6 bis 8 einen Austragskolben 6 auf, der im Innenraum der Kartusche 50 nicht über ein Rastmittel mit der Kartusche 50 verbunden ist. Ein weiterer Unterschied zur ersten und zur zweiten beispielhaften Ausführung ist darin zu sehen, dass das Verschlusssystem analog der dritten Ausführung mit einem beweglichen Stopfen 54 ausgeführt ist, wobei im Unterschied zu dritten Ausführung eine Wandung 7, in der eine zentrale Austragsöffnung vorgesehen ist, bereits im Ausgangszustand an der Vorderseite der Kartusche 50 anliegend und dadurch fixiert angeordnet ist.

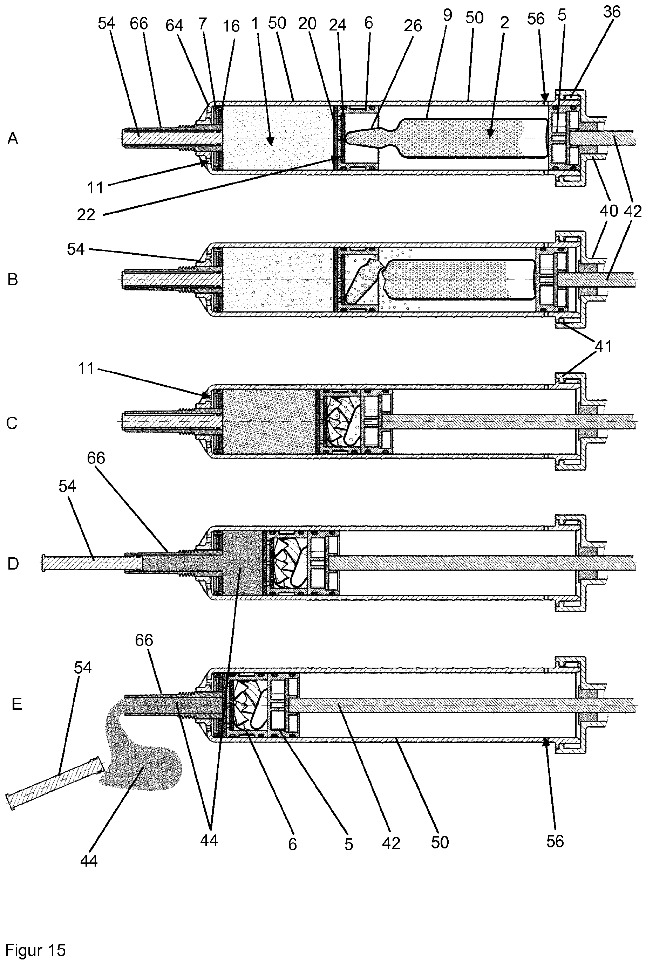

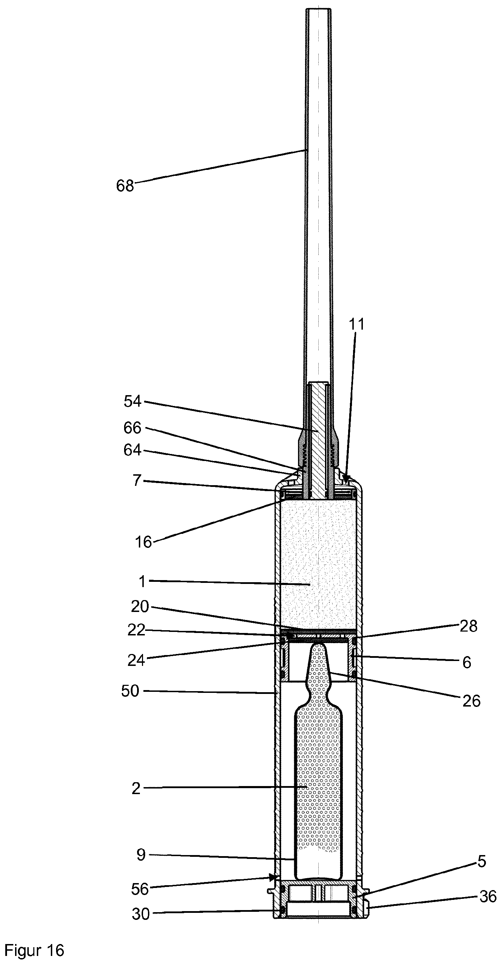

Dabei zeigt Figur 14 eine perspektivische Querschnittansicht der Vorrichtung im Ausgangszustand, Figur 15 fünf schematische Querschnittansichten A bis E der vierten beispielhaften Vorrichtung mit dem alternativen Verschlusssystem, die den Ablauf der Anwendung der Vorrichtung während der Herstellung und der Applikation des Knochenzementteigs 44 zeigen und Figur 16 eine schematische Querschnittansicht der Vorrichtung im Ausgangszustand.

Der Aufbau und die Funktionsweise der vierten beispielhaften Vorrichtung entsprechen weitgehend denen der zweiten beispielhaften Vorrichtung, so dass auch weitgehend auf die Figurenbeschreibung der anderen Ausführungsbeispiele verwiesen werden kann. Insbesondere sind das verwendete Pulver 1 und im Wesentlichen die Funktionsweise der beiden Kolben 5, 6 sind mit der zweiten Ausführung identisch.

Im Ausgangszustand der Vorrichtung sind das Pulver 1 als eine Ausgangskomponente eines PMMA-Knochenzements und eine Monomerflüssigkeit 2 als weitere Ausgangskomponente des PMMA-Knochenzements in der Vorrichtung enthalten. Das Pulver 1 enthält ein Knochenzementpulver als Hauptbestandteil sowie ein hydrophiles Additiv, mit dem die Monomerflüssigkeit 2 in dem Pulver 1 verteilbar ist. Das Pulver 1 und die Monomerflüssigkeit 2 sind in einer Kartusche 50 enthalten, wobei das Pulver 1 in einem vorderen Teil des Innenraums der Kartusche 50 und die Monomerflüssigkeit 2 in einem hinteren Teil des Innenraums der Kartusche 50 angeordnet ist. Gemeinsam begrenzt der hintere Innenraum und der vordere Innenraum einen zylindrischen Innenraum der Kartusche 50.

An der Rückseite der Vorrichtung (in Figur 14 nach rechts oben hinten, in Figur 15 rechts und in Figur 16 unten) ist ein Förderkolben 5 angeordnet, der in axialer Richtung linear in Richtung der Vorderseite der Vorrichtung (in Figur 14 nach links unten vorne, in Figur 15 links und in Figur 16 oben) im Innenraum der Kartusche 50 vortreibbar ist, beziehungsweise beweglich gelagert ist. Der Förderkolben 5 schließt die Rückseite des Innenraums der Kartusche 50 ab. Im hinteren Ende des vorderen Innenraums, beziehungsweise in der Verbindung vom vorderen Innenraum zum hinteren Innenraum der Kartusche 50 ist ein Austragskolben 6 angeordnet, der in axialer Richtung linear in Richtung der Vorderseite der Vorrichtung im Innenraum der Kartusche 50 vortreibbar ist, beziehungsweise beweglich gelagert ist. Der Austragskolben 6 ist also zwischen dem Pulver 1 und der Monomerflüssigkeit 2 im Innenraum der Kartusche 50 angeordnet.

An der Vorderseite der Vorrichtung ist ein Verschlusssystem angeordnet, mit dem der Innenraum der Kartusche 50 nach vorne abgeschlossen ist, das sich aber zum Austragen eines aus den Ausgangskomponenten 1, 2 gemischten Knochenzementteigs 44 (siehe Figuren 15 D und 15 E) öffnen lässt. Die Wandung 7 des Verschlusssystems weist eine zentrale kreisrunde Austragsöffnung auf und liegt in axialer Richtung des vorderen Innenraums der Kartusche 50 an der Innenseite des Kartuschenkopfs 64 an. Die Austragsöffnung ist durch einen inneren Ring begrenzt. Die Wandung 7 ist nach Art eines Rads mit einem äußeren Ring aufgebaut, der mit dem die Austragsöffnung begrenzenden inneren Ring über eine Mehrzahl von Streben verbunden ist, was andeutungsweise in Figur 14 zu erkennen ist. Der innere Ring, der äußere Ring und die Streben sind einteilig aus Kunststoff gefertigt. Ferner umfasst das Verschlusssystem einen Stopfen 54, mit dem die Austragsöffnung im Ausgangszustand, wie er in Figur 14, Figur 15 A und Figur 16 gezeigt ist, verschlossen ist.

Die Monomerflüssigkeit 2 ist in einer geschlossenen Ampulle 9 als Behälter 9 für die Monomerflüssigkeit 2 enthalten. Die Ampulle 9 besteht aus Glas oder aus einem Kunststoff, der chemisch gegen die Monomerflüssigkeit 2 beständig ist. Innerhalb der Ampulle 9 kann die Monomerflüssigkeit 2 über lange Zeit in der Vorrichtung gelagert werden.

Das Verschlusssystem der Vorrichtung ist in dem Kartuschenkopf 64 angeordnet, der den vorderen Bereich des vorderen Innenraums der Kartusche 50 begrenzt. Der Kartuschenkopf 64 ist ein Teil der Kartusche 50. In dem Kartuschenkopf 10 ist an der Fronseite eine Begasungsöffnung 11 vorgesehen. Die Begasungsöffnung 11 kann im Lagerungszustand beziehungsweise im Ausgangszustand der Vorrichtung mit einem Verschluss verschlossen sein. Durch die Begasungsöffnung 11 kann der Innenraum der Kartusche 50 mit einem sterilisierenden Gas, wie Ethylenoxid, begast werden und so der Inhalt der Kartusche 50 sterilisiert werden. In der Wandung am hinteren Ende der Kartusche 50 direkt neben der Position des Förderkolbens 5 sind mehrere Begasungsöffnungen 56 vorgesehen, die das Innere der Kartusche 50 mit der Umgebung verbinden und durch die der Innenraum der Kartusche 50 von außen mit dem sterilisierenden Gas begast werden kann. Bei einer Bewegung des Förderkolbens 5 in Richtung des Kartuschenkopfs 64 werden diese rückseitigen Begasungsöffnungen 56 durch den Förderkolben 5 verschlossen, so dass keine aus der Ampulle 9 austretende Monomerflüssigkeit 2 aus dem hinteren Teil des Innenraums nach außen dringen kann. Dadurch kann das sterilisierende Gas durch die vordere Begasungsöffnung 11 im Kartuschenkopf 64 und durch die hinteren Begasungsöffnungen 56 durch die Kartusche 50 geleitet werden.

Die zwischen den Streben der Wandung 7 des Verschlusssystems offenen Zwischenräume sind durch einen gasdurchlässigen aber für das Pulver 1 und den Knochenzementteig 44 undurchlässigen Filter 16 abgedeckt. Dadurch ist die Wandung 7 für Gase wie Ethylenoxid durchlässig aber für das Pulver 1 und den Knochenzementteig 44 undurchlässig. Die Wandung 7 ist durch ein Gerüst in Form des äußeren Rings mit den sich nach Innen zur Austragsöffnung hin radial erstreckenden Speichen geformt und somit durchlässig, wenn deren Zwischenräume nicht durch den Filter 16 abgedeckt wären. Der Aufbau der Wandung 7 ist am besten in Figur 14 zu erahnen entspricht aber dem der Wandungen nach den ersten beiden Ausführungsbeispielen.

Einteilig mit der Wandung 7 ausgeführt, ist an der Austragsöffnung, beziehungsweise an dem Inneren Ring der Wandung 7, der die Austragsöffnung begrenzt, ein Austragsrohrstutzen 66 mit einem Außengewinde angeordnet, durch das bei der Anwendung der Vorrichtung eine Applikation des Knochenzementteigs 44 erfolgt (siehe Figur 15 E). Der Austragsrohrstutzen 66 mündet also in die Austragsöffnung im Inneren der Kartusche 50. Auf den Austragsrohrstutzen 66 kann eine Austragsrohrverlängerung 68 aufgeschraubt werden, mit dem eine Applikation von Knochenzementteig 44 in schwerer zugänglichen Regionen durchgeführt werden kann. Mit der Wandung 7 ist der Austragsrohrstutzen 66 in einer Durchführung im Kartuschenkopf 10 angeordnet und kann theoretisch auch fest mit dem Kartuschenkopf 64 verbunden sein. Das Pulver 1 kann im vorderen Teil des Innenraums der Kartusche 50 mit Hilfe des Austragskolbens 6 komprimiert werden, in dem der Austragskolben 6 ins Innere der Kartusche 50 in Richtung des Kartuschenkopfs 64 gedrückt wird. Der Austragskolben 6 wird gegen eine Rückstellung über eine Passung mit der Wand des Innenraums der Kartusche 50 und/oder abgestützt durch die Ampulle 9 und den Förderkolben 5 in der Kartusche 50 gehalten. Das Pulver 1 ist unter Druck in den vorderen Teil des Innenraums der Kartusche 50 zwischen der Wandung 7 mit dem Filter 16 und dem Austragskolben 6 eingepresst und steht unter einem elastischen mechanischen Druck. Durch die Passung des Austragskolbens 6 mit der Innenwand der Kartusche 50 und/oder die Abstützung mit der Ampulle 9 und dem Förderkolben 5 soll vermieden werden, dass der von dem zusammengepressten Pulver 1 auf den Austragskolben 6 ausgeübte elastische Druck ausreicht, um den Austragskolben 6 zu bewegen und das Pulver 1 wieder zu entspannen. Die Wandung 7 ist mit einer umlaufenden Dichtung 32 gegen die Innenwand der Kartusche 50 abgedichtet.

Der Stopfen 54, mit dem die Austragsöffnung verschlossen ist, steckt in dem Austragsrohrstutzen 66 und verschließt damit die Kartusche 50 nach außen. Der Stopfen 54 ist beweglich in dem Austragsrohrstutzen 66 angeordnet und kann von Innen aus dem Austragsrohrstutzen 66 herausgedrückt werden.

Die innen am Kartuschenkopf 64 anliegende und dadurch mit der Kartusche 50 fixierte Wandung 7 mit dem Filter 16 sowie dem dagegen beweglichen gehaltenen Stopfen 54 bilden ein Verschlusssystem für die Vorrichtung nach dem vierten Ausführungsbeispiel.

An der zum Pulver 1 weisenden Seite des Austragskolbens 6 ist ein für das Pulver 1 undurchlässiger aber für die Monomerflüssigkeit 2 durchlässiger Porenfilter 20 angeordnet. Damit soll verhindert werden, dass das Pulver 1 durch Durchgänge 22, die in dem Austragskolben 6 vorgesehen sind, in den hinteren Teil des Innenraums der Kartusche 50 vordringen kann. Der Porenfilter 20 deckt dabei die Durchgänge 22 ab, so dass das Pulver 1 auch nicht in die Durchgänge 22 vordringen kann. Hierdurch wird vermieden, dass die Monomerflüssigkeit 2 nach dem Öffnen der Ampulle 9 bereits vorzeitig mit Zementpulverpartikeln des Pulvers 1 reagiert, also bevor die Monomerflüssigkeit 2 in den vorderen Teil des Innenraums eingepresst wurde. Dadurch kann verhindert werden, dass die Durchgänge 22 durch anquellenden Knochenzement blockiert werden und dadurch keine weitere Einleitung von Monomerflüssigkeit 2 in das Pulver 1 mehr möglich ist.

Auf der dem Porenfilter 20 gegenüberliegenden Seite des Austragskolbens 6 ist ein Gitter 24 oder Sieb 24 angeordnet, mit dem das Vordringen von Splittern der aufgebrochenen Ampulle 9 in die Durchgänge 22 verhindert wird. Auch hiermit soll sichergestellt werden, dass die Monomerflüssigkeit 2 aus dem hinteren Teil des Innenraums der Kartusche 50 problemlos in das Pulver 1 gedrückt werden kann. Beim Aufbrechen der Ampulle 9 wird zunächst ein Ampullenkopf 26 abgebrochen und dadurch die Ampulle 9 geöffnet (siehe Figur 15 B). Die Monomerflüssigkeit 2 aus der Ampulle 9 kann dann in den vorderen Teil des Innenraums der Kartusche 50 ausfließen und anschließend durch die Durchgänge 22 in das Pulver 1 gepresst werden (siehe Figur 15 C). Dabei wird die Ampulle 9 in derart kleine Splitter zersplittert, dass diese in einen Hohlraum passen, der auf der dem Kartuschenboden zugewandten Seite (in Figur 14 nach oben rechts hinten, in Figur 15 nach rechts und in Figur 16 nach unten) des Austragskolbens 6 gebildet ist.

Am Kartuschenboden ist außen an der Kartusche 50 eine Halterung 36 zur Befestigung einer Auspresseinrichtung 40 (siehe Figur 15) vorgesehen.

Der Stopfen 54 steckt derart fest in dem Austragsrohrstutzen 66, dass er durch die beim Zersplittern der Ampulle 9 auftretenden Kräften nicht bewegt wird, um dem Druck der Monomerflüssigkeit 2 während des Vortriebs des Förderkolbens 5 zu wiederstehen und um dem Druck, der von dem Pulver 1 auf den Austragskolben 6 mittels der eingepressten Wandung 7 ausgeübt wird, standzuhalten. Beim Eintragen der Monomerflüssigkeit 2 und beim Vortreiben des Förderkolbens 5 verdrängte Luft kann durch die Begasungsöffnungen 11 entweichen. Erst wenn der Förderkolben 5 unmittelbar an dem Austragskolben 6 anliegt (siehe Figur 15 C), wird die Haftreibung zwischen dem Stopfen 54 und dem Austragsrohrstutzen 64 überwunden und der Stopfen 54 wird aus dem Austragsrohrstutzen 66 gedrückt und dadurch die Kartusche 50 für den Knochenzementteig 44 nach außen geöffnet.

In dem in der Rückseite des Austragskolbens 6 gebildeten Hohlraum im Austragskolben 6 kann vorzugsweise ein Füllmaterial (nicht gezeigt) vorgesehen sein, wie beispielsweise ein Schaumstoffeinsatz und/oder Kunststoffkugeln oder -körner. Damit soll das Volumen der Monomerflüssigkeit 2, die in diesem Hohlraum zurückbleibt und nicht von dem Förderkolben 5 in das Pulver 1 gepresst werden kann, möglichst klein gehalten werden. Des Weiteren kann dieses Füllmaterial als Transportschutz und Stoßschutz für die Ampulle 9 verwendet werden, damit die Ampulle 9 beim Transport der Vorrichtung im Ausgangszustand (siehe Figuren 1 und 2) nicht aus Versehen aufbricht. Hierzu kann ein komprimierbarer Schaumstoff zusätzlich um die Ampulle 9 herum im Innenraum der Kartusche 50 angeordnet sein.

Der Ablauf eines beispielhaften Verfahrens ist in Figur 15 durch fünf übereinander gezeichnete Querschnittansichten Figur 15 A bis Figur 15 E dargestellt. Zunächst wird die Vorrichtung in eine Auspresseinrichtung 40 eingesetzt, dazu wird die Kartusche 50 mit der Halterung 36 an einem passenden Gegenstück 41 der Auspresseinrichtung 40 befestigt (siehe Figur 15 A).

Nach dem Einsetzen der Vorrichtung wird ein Stößel 42 der Auspresseinrichtung 40 gegen die Kartusche 50 vorgetrieben. Der Stößel 42 liegt an dem Förderkolben 5 an. Dadurch wird der Förderkolben 5 von dem Stößel 42 in Richtung des Austragskolbens 6 gedrückt. Durch die Bewegung des Förderkolbens 5 wird die Ampulle 9 gegen den von dem gepressten Pulver 1 und den Kartuschenkopf 64 gehaltenen Austragskolben 6 gedrückt. Der Ampullenkopf 26 bricht ab und die Ampulle 9 ist geöffnet (siehe Figur 15 B).

Die Vorrichtung in der Auspresseinrichtung 40 wird dabei bevorzugt mit dem Kartuschenkopf 64 nach oben gehalten, so dass bei einem nachfolgenden weiteren Vortreiben des Förderkolbens 5 zunächst die überstehende Luft aus dem hinteren Teil des Innenraums nach oben durch das Pulver 1, durch den gasdurchlässigen Filter 16 und die Begasungsöffnungen 11 nach außen gedrückt wird. Irgendwann wird die Monomerflüssigkeit 2 aus der Ampulle 9 von dem Förderkolben 5 durch das Gitter 24 und/oder das Sieb 24, durch die Durchgänge 22 und durch den Porenfilter 20 in den vorderen Teil des Innenraums in das Pulver 1 gedrückt. Dabei wird die Ampulle 9 weiter zusammengedrückt und zersplittert dabei in kleinere Splitter, die sich schließlich in dem rückseitigen Hohlraum des Austragskolbens 6 sammeln. Das Pulver 1 enthält ein hydrophiles Additiv, das eine große Oberflächenenergie bezüglich der wässrigen Monomerflüssigkeit 2 aufweist, die größer ist als die des Knochenzementpulvers. Gleichzeitig sind die Kapillarkräfte aufgrund des komprimierten Pulvers 1 groß, da die Zwischenräume zwischen den Pulverpartikeln klein sind. Zudem wird die Monomerflüssigkeit 2 mit Druck in das Pulver 1 gepresst. Durch all diese Maßnahmen wird die Monomerflüssigkeit 2 schnell in und durch das Pulver 1 geleitet und kann sich vollständig in dem Pulver 1 ausbreiten und verteilen, bevor die anquellenden Zementpulverpartikel eine weitere Ausbreitung der Monomerflüssigkeit 2 im Pulver 1 unterbinden. Schließlich trifft der Förderkolben 5 auf den Austragskolben 6 (siehe Figur 15 C).

Das Zementpulver in dem Pulver 1 reagiert mit der Monomerflüssigkeit 2 und bildet dort den Knochenzementteig 44. Um dabei das gewünschte Mischungsverhältnis zwischen Pulver 1 und Monomerflüssigkeit 2 im Knochenzementteig 44 zu erhalten, kann überschüssige Monomerflüssigkeit 2 an der Vorderseite der Kartusche 50 zwischen dem porösen Filter 16 der Wandung 7 und dem Kartuschenkopf 10 aufgenommen werden. Die Monomerflüssigkeit 2 wird dazu durch den porösen Filter 16 gedrückt, der für das Pulver 1 und den Knochenzementteig 44 undurchlässig ist. Durch Aufnahme der überschüssigen Monomerflüssigkeit 2 nach dem Durchtritt der Monomerflüssigkeit 2 durch das Pulver 1 bis zur Wandung 7 wird verhindert, dass der Knochenzementteig 44 zu dünnflüssig wird und dadurch eine unerwünschte Konsistenz erhält. Zudem wird zur Vermeidung einer zu dickflüssigen Konsistenz des Knochenzementteigs 44 die Monomerflüssigkeit 2 im Überschuss verwendet, so dass die Verluste durch die zwischen dem Austragskolben 6 und dem Förderkolben 5 sowie in den Durchgängen 22 des Austragskolbens 6 verbleibende Reste der Monomerflüssigkeit 2 ausgeglichen werden.

Durch einen weiteren Vortrieb des Förderkolbens 5 wird der Austragskolben 6 in Richtung des Kartuschenkopfs 64 getrieben. Durch die Bewegung des Austragskolbens 6 in Richtung des Kartuschenkopfs 64 wird vom Knochenzementteig 44 ein Druck auf den Stopfen 54 in der Austragsöffnung des Verschlusssystems ausgeübt.

Da die Wandung 7 innen am Kartuschenkopf 64 anliegt und sich daher nicht weiter in Richtung des Kartuschenkopfs 64 bewegen kann und der Förderkolben 5 zusammen mit dem daran anliegenden Austragskolben 6 in Richtung des Kartuschenkopfs 64 vorgetrieben wird, wird der Stopfen 54 nach vorne aus dem Austragsrohrstutzen 66 herausgedrückt (siehe Figuren 15 D und 15 E). Während also die Wandung 7, die über den Anpressdruck des Knochenzementteigs 44 fest an der Vorderseite der Kartusche 50 anliegt, nicht mit dem Knochenzementteig 44 bewegt wird, wird der Stopfen 54 gegen die Wandung 7 bewegt und damit aus der Austragsöffnung herausgetrieben und so die Austragsöffnung in der Wandung 7 geöffnet. Schließlich fällt der Stopfen 54 vorne aus dem Austragsrohrstutzen 66 hinaus und der Knochenzementteig 44 tritt aus dem Austragsrohrstutzen 66 aus. Die Kartusche 50 ist nun nach außen geöffnet. Durch weiteres Vortreiben des Förderkolbens 5 und damit des Austragskolbens 6 wird der fertige Knochenzementteig 44 durch die Austragsöffnung und den Austragsrohrstutzen 66 nach außen gepresst und kann appliziert werden (siehe Figur 15 E).

Aufgrund des in dem Pulver 1 vorhandenen Additivs gelingt es, die Monomerflüssigkeit 2 an einer Grundfläche des vorderen Teils des zylindrischen Innenraums der Kartusche 50 einzupressen und dennoch eine vollständige Verteilung der Monomerflüssigkeit 2 in dem Pulver 1 zu erreichen. Durch den Aufbau der Vorrichtung gelingt es, eine herkömmliche Auspresseinrichtung 40 verwenden zu können und durch eine unidirektionale lineare Bewegung des Stößels 42 sowohl den Behälter 9 für die Monomerflüssigkeit 2 zu öffnen, die Monomerflüssigkeit 2 in das Pulver 1 zu pressen und dadurch den Knochenzementteig 44 zu mischen, als auch das Verschlusssystem zu öffnen und den gemischten Knochenzementteig 44 auszutreiben und zu applizieren. Mit dem Aufbau des Verschlusssystems gelingt es, die Kraft, die durch den Stößel 42 auf den Förderkolben 5 ausgeübt wird, zum Öffnen der Austragsöffnung nutzen können.

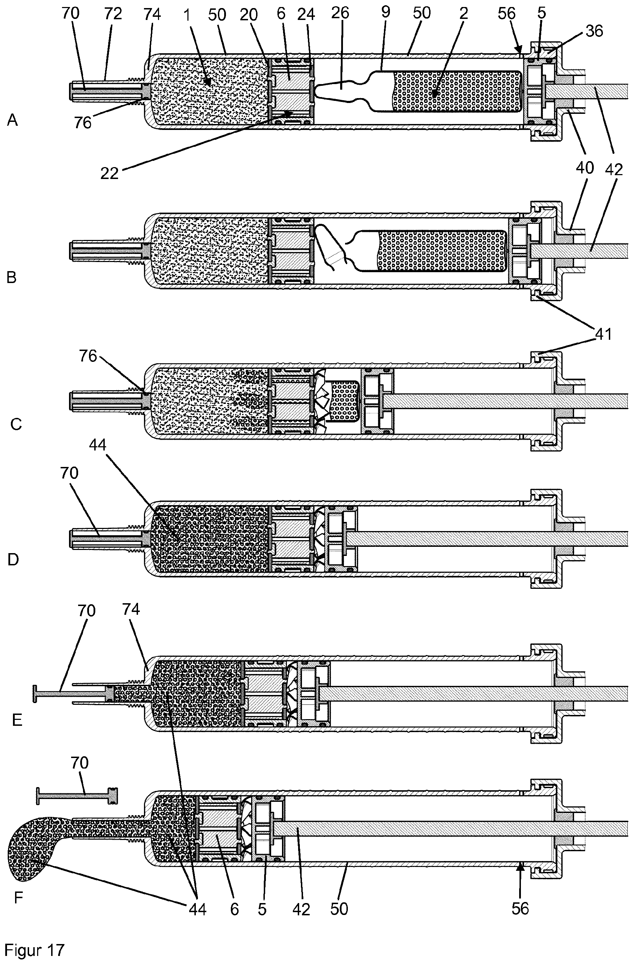

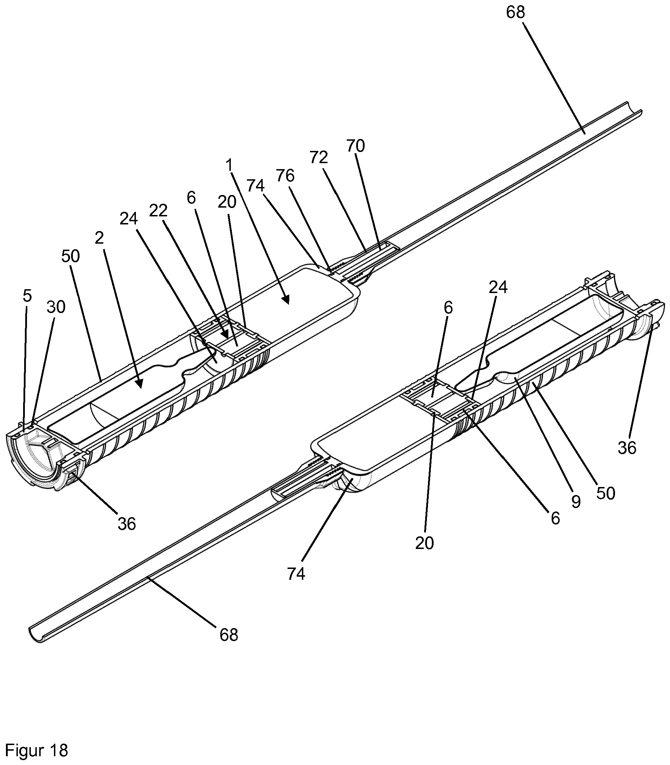

In den Figuren 17 und 18 ist eine fünfte beispielhafte Vorrichtung gezeigt, die mit einem erfindungsgemäßen Verfahren hergestellt wurde und die besonders kostengünstig im Aufbau ist und die sich von der ersten beispielhaften Vorrichtung nach den Figuren 1 bis 5 dadurch unterscheidet, dass keine separate Wandung 7 mehr vorhanden ist, sondern dass die Wandung 74 durch einen Kartuschenkopf 74 einer einteilig aus Kunststoff aufgebauten Kartusche 50 gebildet ist. Die fünfte beispielhafte Vorrichtung weist also analog der zweiten, dritten und vierten beispielhaften Vorrichtung eine Kartusche 50 auf, die nicht aus zwei Kartuschenteilen besteht, wobei hier im Gegensatz zur zweiten und dritten Ausführung und analog der vierten Ausführung auch der Kartuschenkopf 74 der Kartusche 50 einteilig mit der Kartusche 50 ausgeführt ist. Ferner weist die fünfte beispielhafte Vorrichtung analog der zweiten beispielhaften Vorrichtung nach den Figuren 6 bis 8 und dem vierten Ausführungsbeispiel nach den Figuren 14 bis 16 einen Austragskolben 6 auf, der im Innenraum der Kartusche 50 nicht über ein Rastmittel mit der Kartusche 50 verbunden ist. Ein weiterer Unterschied zur ersten und zur zweiten beispielhaften Ausführung ist darin zu sehen, dass das Verschlusssystem analog der dritten und vierten Ausführung mit einem beweglichen Stopfen 70 ausgeführt ist, wobei im Unterschied zu dritten und vierten Ausführung die Wandung 74, in der eine zentrale Austragsöffnung vorgesehen ist, einteilig mit der Kartusche 50 ausgeführt ist und dadurch stets mit der Kartusche 50 verbunden und dadurch fixiert ist.

Dabei zeigt Figur 17 sechs schematische Querschnittansichten A bis F der fünften beispielhaften Vorrichtung mit dem vereinfachten Aufbau, die den Ablauf der Anwendung der Vorrichtung während der Herstellung und der Applikation des Knochenzementteigs 44 zeigen und Figur 18 zwei perspektivische Querschnittansichten der Vorrichtung im Ausgangszustand.

Der Aufbau und die Funktionsweise der fünften beispielhaften Vorrichtung entsprechen weitgehend denen der ersten und vierten beispielhaften Vorrichtung, so dass auch weitgehend auf die Figurenbeschreibung der anderen Ausführungsbeispiele verwiesen werden kann. Insbesondere sind das verwendete Pulver 1 und im Wesentlichen die Funktionsweise der beiden Kolben 5, 6 bis auf die Rastung des Austragskolbens 6 mit der Innenseite der Kartusche 50 identisch.

Im Ausgangszustand der Vorrichtung sind das Pulver 1 als eine Ausgangskomponente eines PMMA-Knochenzements und eine Monomerflüssigkeit 2 als weitere Ausgangskomponente des PMMA-Knochenzements in der Vorrichtung enthalten. Das Pulver 1 enthält ein Knochenzementpulver als Hauptbestandteil sowie ein hydrophiles Additiv, mit dem die Monomerflüssigkeit 2 in dem Pulver 1 verteilbar ist. Das Pulver 1 und die Monomerflüssigkeit 2 sind in einer Kartusche 50 enthalten, wobei das Pulver 1 in einem vorderen Teil des Innenraums der Kartusche 50 und die Monomerflüssigkeit 2 in einem hinteren Teil des Innenraums der Kartusche 50 angeordnet ist. Gemeinsam begrenzt der hintere Innenraum und der vordere Innenraum einen zylindrischen Innenraum der Kartusche 50.

An der Rückseite der Vorrichtung (in Figur 17 rechts) ist ein Förderkolben 5 angeordnet, der in axialer Richtung linear in Richtung der Vorderseite der Vorrichtung (in Figur 17 links) im Innenraum der Kartusche 50 vortreibbar ist, beziehungsweise beweglich gelagert ist. Der Förderkolben 5 schließt die Rückseite des Innenraums der Kartusche 50 ab. Im hinteren Ende des vorderen Innenraums, beziehungsweise in der Verbindung vom vorderen Innenraum zum hinteren Innenraum der Kartusche 50 ist ein Austragskolben 6 angeordnet, der in axialer Richtung linear in Richtung der Vorderseite der Vorrichtung im Innenraum der Kartusche 50 vortreibbar ist, beziehungsweise beweglich gelagert ist. Der Austragskolben 6 ist also zwischen dem Pulver 1 und der Monomerflüssigkeit 2 im Innenraum der Kartusche 50 angeordnet.

An der Vorderseite der Vorrichtung ist ein Verschlusssystem angeordnet, mit dem der Innenraum der Kartusche 50 nach vorne abgeschlossen ist, das sich aber zum Austragen eines aus den Ausgangskomponenten 1, 2 gemischten Knochenzementteigs 44 (siehe Figuren 17 D, 17 E und 17 F) öffnen lässt. Die Wandung 74, die ein Teil des Verschlusssystems ist, weist eine zentrale kreisrunde Austragsöffnung auf und bildet den Kartuschenkopf 74 der Kartusche 50. Die Kartusche 50 ist zusammen mit der Wandung 74 einteilig aus Kunststoff gefertigt. Ferner umfasst das Verschlusssystem den Stopfen 70, mit dem die Austragsöffnung im Ausgangszustand, wie er in Figur 17 A und Figur 18 gezeigt ist, verschlossen ist.

Die Monomerflüssigkeit 2 ist in einer geschlossenen Ampulle 9 als Behälter 9 für die Monomerflüssigkeit 2 enthalten. Die Ampulle 9 besteht aus Glas oder aus einem Kunststoff, der chemisch gegen die Monomerflüssigkeit 2 beständig ist. Innerhalb der Ampulle 9 kann die Monomerflüssigkeit 2 über lange Zeit in der Vorrichtung gelagert werden.

Das Verschlusssystem der Vorrichtung ist durch die Wandung 74 und den Stopfen 70 gebildet. Damit ein Gas aus dem Inneren der Kartusche 50 beim Vortreiben der Kolben 5, 6 zur Wandung 74 entweichen kann und damit ein sterilisierendes Gas in den Innenraum der Kartusche 50 eingeleitet werden kann, ist der Stopfen mit einem umlaufenden porösen Kunststoffring 76 gegen die Austragsöffnung beziehungsweise gegen die Innenwand des Austragsrohrs 72 abgedichtet. Der poröse Kunststoffring 76 ist für Gase durchlässig und für das Pulver 1 undurchlässig und ist aus Polyethylen gefertigt. Die so gebildete Begasungsöffnung ist im Lagerungszustand beziehungsweise im Ausgangszustand der Vorrichtung mit einer vorderen Kreisscheibe des Stopfens 70 abgedeckt. Durch verschieben des Stopfens 70 aus dem Austragsrohr 72 hinaus, kann diese Öffnung geöffnet werden, um sterilisierendes Gas einzuspeisen. Durch den Kunststoffring 76 kann der Innenraum der Kartusche 50 mit einem sterilisierenden Gas, wie Ethylenoxid, begast werden und so der Inhalt der Kartusche 50 sterilisiert werden. In der Wandung am hinteren Ende der Kartusche 50 direkt neben der Position des Förderkolbens 5 sind mehrere Begasungsöffnungen 56 vorgesehen, die das Innere der Kartusche 50 mit der Umgebung verbinden und durch die der Innenraum der Kartusche 50 von außen mit dem sterilisierenden Gas begast werden kann. Bei einer Bewegung des Förderkolbens 5 in Richtung der Wandung 74 werden diese rückseitigen Begasungsöffnungen 56 durch den Förderkolben 5 verschlossen, so dass keine aus der Ampulle 9 austretende Monomerflüssigkeit 2 aus dem hinteren Teil des Innenraums nach außen dringen kann. Dadurch kann das sterilisierende Gas durch den porösen Kunststoffring 76 und durch die hinteren Begasungsöffnungen 56 durch die Kartusche 50 geleitet werden.

Einteilig mit der Kartusche 50 und der Wandung 74 ausgeführt, ist an der Austragsöffnung das Austragsrohr 72 mit einem Außengewinde angeordnet, durch das bei der Anwendung der Vorrichtung eine Applikation des Knochenzementteigs 44 erfolgt (siehe Figur 17 F). Auf das Außengewinde am Austragsrohr 72 kann eine Austragsrohrverlängerung 68 aufgeschraubt werden (siehe Figur 18), mit dem eine Applikation von Knochenzementteig 44 in schwerer zugänglichen Regionen durchgeführt werden kann.

Das Pulver 1 kann im vorderen Teil des Innenraums der Kartusche 50 mit Hilfe des Austragskolbens 6 komprimiert werden, in dem der Austragskolben 6 ins Innere der Kartusche 50 in Richtung der Wandung 74 gedrückt wird. Der Austragskolben 6 wird gegen eine Rückstellung über eine Passung mit der Wand des Innenraums der Kartusche 50 und/oder abgestützt durch die Ampulle 9 und den Förderkolben 5 in der Kartusche 50 gehalten. Das Pulver 1 ist unter Druck in den vorderen Teil des Innenraums der Kartusche 50 zwischen der Wandung 74 und dem Austragskolben 6 eingepresst und steht unter einem elastischen mechanischen Druck. Durch die Passung des Austragskolbens 6 mit der Innenwand der Kartusche 50 und/oder die Abstützung mit der Ampulle 9 und dem Förderkolben 5 soll vermieden werden, dass der von dem zusammengepressten Pulver 1 auf den Austragskolben 6 ausgeübte elastische Druck ausreicht, um den Austragskolben 6 zu bewegen und das Pulver 1 wieder zu entspannen.

Der Stopfen 70, mit dem die Austragsöffnung verschlossen ist, steckt in dem Austragsrohr 72 und verschließt damit die Kartusche 50 nach außen. Der Stopfen 70 ist beweglich in dem Austragsrohr 72 angeordnet und kann von Innen aus dem Austragsrohr 72 herausgedrückt werden.

In dem Austragskolben 6 sind Durchgänge 22 vorgesehen, durch die die Monomerflüssigkeit 2 mit Hilfe des Förderkolbens 5 in das Pulver 1 gepresst werden kann, so wie dies auch bei den anderen Ausführungsbeispielen vorgesehen ist. An der zum Pulver 1 weisenden Seite des Austragskolbens 6 sind über den Durchgängen 22 für das Pulver 1 undurchlässige aber für die Monomerflüssigkeit 2 durchlässige Porenfilter 20 angeordnet, die die Durchgänge 22 abdecken. Damit soll verhindert werden, dass das Pulver 1 durch die Durchgänge 22 in den hinteren Teil des Innenraums der Kartusche 50 oder in die Durchgänge 22 vordringen kann. Hierdurch wird vermieden, dass die Monomerflüssigkeit 2 nach dem Öffnen der Ampulle 9 bereits vorzeitig mit Zementpulverpartikeln des Pulvers 1 reagiert, also bevor die Monomerflüssigkeit 2 in den vorderen Teil des Innenraums eingepresst wurde. Dadurch kann verhindert werden, dass die Durchgänge 22 durch anquellenden Knochenzement blockiert werden und dadurch keine weitere Einleitung von Monomerflüssigkeit 2 in das Pulver 1 mehr möglich ist.

Auf der den Porenfiltern 20 gegenüberliegenden Seite des Austragskolbens 6 sind in Ausnehmungen über den Durchgängen 22 passende Gitter 24 angeordnet, mit denen das Vordringen von Splittern der aufgebrochenen Ampulle 9 in die Durchgänge 22 verhindert wird. Auch hiermit soll sichergestellt werden, dass die Monomerflüssigkeit 2 aus dem hinteren Teil des Innenraums der Kartusche 50 problemlos in das Pulver 1 gedrückt werden kann. Beim Aufbrechen der Ampulle 9 wird zunächst ein Ampullenkopf 26 abgebrochen und dadurch die Ampulle 9 geöffnet (siehe Figur 17 B). Die Monomerflüssigkeit 2 aus der Ampulle 9 kann dann in den vorderen Teil des Innenraums der Kartusche 50 ausfließen und anschließend durch die Durchgänge 22 in das Pulver 1 gepresst werden (siehe Figur 17 C). Dabei wird die Ampulle 9 in derart kleine Splitter zersplittert, dass diese in einen Hohlraum passen, der auf der dem Kartuschenboden zugewandten Seite (in Figur 17 rechts) des Austragskolbens 6 gebildet ist.

Am Kartuschenboden ist außen an der Kartusche 50 eine Halterung 36 zur Befestigung einer Auspresseinrichtung 40 (siehe Figur 17) vorgesehen.

Der Stopfen 54 steckt derart fest in dem Austragsrohr 72, dass er durch die beim Zersplittern der Ampulle 9 auftretenden Kräften nicht bewegt wird, um dem Druck der Monomerflüssigkeit 2 während des Vortriebs des Förderkolbens 5 zu wiederstehen und um dem Druck, der von dem Pulver 1 auf den Austragskolben 6 ausgeübt wird, standzuhalten. Beim Eintragen der Monomerflüssigkeit 2 und beim Vortreiben des Förderkolbens 5 verdrängte Luft kann durch den porösen Kunststoffring 76 entweichen. Erst wenn der Förderkolben 5 unmittelbar an dem Austragskolben 6 anliegt (siehe Figur 17 D), wird die Haftreibung zwischen dem Stopfen 54 und dem Austragsrohr 72 überwunden und der Stopfen 54 wird aus dem Austragsrohr 72 gedrückt und dadurch die Kartusche 50 für den Knochenzementteig 44 nach außen geöffnet.

In dem in der Rückseite des Austragskolbens 6 gebildeten Hohlraum im Austragskolben 6 kann vorzugsweise ein Füllmaterial (nicht gezeigt) vorgesehen sein, wie beispielsweise ein Schaumstoffeinsatz und/oder Kunststoffkugeln oder -körner. Damit soll das Volumen der Monomerflüssigkeit 2, die in diesem Hohlraum zurückbleibt und nicht von dem Förderkolben 5 in das Pulver 1 gepresst werden kann, möglichst klein gehalten werden. Des Weiteren kann dieses Füllmaterial als Transportschutz und Stoßschutz für die Ampulle 9 verwendet werden, damit die Ampulle 9 beim Transport der Vorrichtung im Ausgangszustand (siehe Figuren 17 A und 18) nicht aus Versehen aufbricht. Hierzu kann ein komprimierbarer Schaumstoff zusätzlich um die Ampulle 9 herum im Innenraum der Kartusche 50 angeordnet sein.

Der Ablauf eines beispielhaften Verfahrens ist in Figur 17 durch sechs übereinander gezeichnete Querschnittansichten Figur 17 A bis Figur 17 F dargestellt. Zunächst wird die Vorrichtung in eine Auspresseinrichtung 40 eingesetzt, dazu wird die Kartusche 50 mit der Halterung 36 an einem passenden Gegenstück 41 der Auspresseinrichtung 40 befestigt (siehe Figur 17 A).

Nach dem Einsetzen der Vorrichtung wird ein Stößel 42 der Auspresseinrichtung 40 gegen die Kartusche 50 vorgetrieben. Der Stößel 42 liegt an dem Förderkolben 5 an. Dadurch wird der Förderkolben 5 von dem Stößel 42 in Richtung des Austragskolbens 6 gedrückt. Durch die Bewegung des Förderkolbens 5 wird die Ampulle 9 gegen den von dem gepressten Pulver 1 und der Wandung 74 gehaltenen Austragskolben 6 gedrückt. Der Ampullenkopf 26 bricht ab und die Ampulle 9 ist geöffnet (siehe Figur 17 B).

Die Vorrichtung in der Auspresseinrichtung 40 wird dabei bevorzugt mit dem Austragsrohr 72 nach oben gehalten, so dass bei einem nachfolgenden weiteren Vortreiben des Förderkolbens 5 zunächst die überstehende Luft aus dem hinteren Teil des Innenraums nach oben durch das Pulver 1, durch den gasdurchlässigen Filter 16 und den porösen Kunststoffring 76 nach außen gedrückt wird. Irgendwann wird die Monomerflüssigkeit 2 aus der Ampulle 9 von dem Förderkolben 5 durch die Gitter 24, durch die Durchgänge 22 und durch die Porenfilter 20 in den vorderen Teil des Innenraums in das Pulver 1 gedrückt (siehe Figur 17 C). Dabei wird die Ampulle 9 weiter zusammengedrückt und zersplittert dabei in kleinere Splitter, die sich schließlich in dem rückseitigen Hohlraum des Austragskolbens 6 sammeln. Das Pulver 1 enthält ein hydrophiles Additiv, das eine große Oberflächenenergie bezüglich der wässrigen Monomerflüssigkeit 2 aufweist, die größer ist als die des Knochenzementpulvers. Gleichzeitig sind die Kapillarkräfte aufgrund des komprimierten Pulvers 1 groß, da die Zwischenräume zwischen den Pulverpartikeln klein sind. Zudem wird die Monomerflüssigkeit 2 mit Druck in das Pulver 1 gepresst. Durch all diese Maßnahmen wird die Monomerflüssigkeit 2 schnell in und durch das Pulver 1 geleitet und kann sich vollständig in dem Pulver 1 ausbreiten und verteilen, bevor die anquellenden Zementpulverpartikel eine weitere Ausbreitung der Monomerflüssigkeit 2 im Pulver 1 unterbinden. Schließlich trifft der Förderkolben 5 auf den Austragskolben 6 (siehe Figur 17 D).

Das Zementpulver in dem Pulver 1 reagiert mit der Monomerflüssigkeit 2 und bildet dort den Knochenzementteig 44. Durch einen weiteren Vortrieb des Förderkolbens 5 wird der Austragskolben 6 in Richtung der Wandung 74 getrieben. Durch die Bewegung des Austragskolbens 6 in Richtung der Wandung 74 wird vom Knochenzementteig 44 ein Druck auf den Stopfen 54 in der Austragsöffnung des Verschlusssystems ausgeübt.

Da die Wandung 74 fest mit der Kartusche 50 fixiert ist und der Förderkolben 5 zusammen mit dem daran anliegenden Austragskolben 6 in Richtung der Wandung 74 vorgetrieben wird, wird der Stopfen 54 nach vorne aus dem Austragsrohr 72 herausgedrückt (siehe Figuren 17 E und 17 F). Während also die Wandung 74 nicht mit dem Knochenzementteig 44 bewegt wird, wird der Stopfen 54 gegen die Wandung 74 bewegt und damit aus der Austragsöffnung herausgetrieben und so die Austragsöffnung in der Wandung 74 geöffnet. Schließlich fällt der Stopfen 54 vorne aus dem Austragsrohr 72 hinaus und der Knochenzementteig 44 tritt aus dem Austragsrohr 72 beziehungsweise aus der auf das Austragsrohr 72 aufgeschraubten Austragsrohrverlängerung 68 aus. Die Kartusche 50 ist nun nach außen geöffnet. Durch weiteres Vortreiben des Förderkolbens 5 und damit des Austragskolbens 6 wird der fertige Knochenzementteig 44 durch die Austragsöffnung und das Austragsrohr 72 nach außen gepresst und kann appliziert werden (siehe Figur 17 F).

Aufgrund des in dem Pulver 1 vorhandenen Additivs gelingt es, die Monomerflüssigkeit 2 an einer Grundfläche des vorderen Teils des zylindrischen Innenraums der Kartusche 50 einzupressen und dennoch eine vollständige Verteilung der Monomerflüssigkeit 2 in dem Pulver 1 zu erreichen. Durch den Aufbau der Vorrichtung gelingt es, eine herkömmliche Auspresseinrichtung 40 verwenden zu können und durch eine unidirektionale lineare Bewegung des Stößels 42 sowohl den Behälter 9 für die Monomerflüssigkeit 2 zu öffnen, die Monomerflüssigkeit 2 in das Pulver 1 zu pressen und dadurch den Knochenzementteig 44 zu mischen, als auch das Verschlusssystem zu öffnen und den gemischten Knochenzementteig 44 auszutreiben und zu applizieren. Mit dem Aufbau des Verschlusssystems gelingt es, die Kraft, die durch den Stößel 42 auf den Förderkolben 5 ausgeübt wird, zum Öffnen der Austragsöffnung nutzen können.

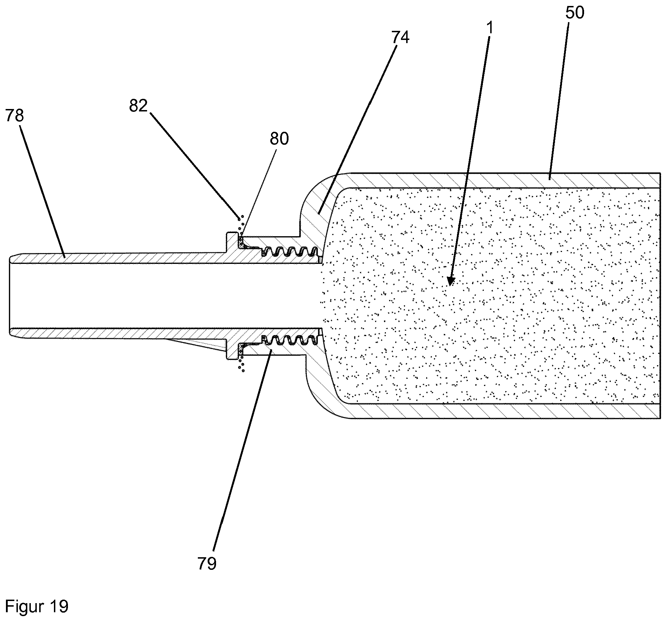

Eine weitere Variante einer Vorrichtung, die mit einem erfindungsgemäßen Verfahren hergestellt wurde, ist in den Figuren 19 bis 21 dargestellt. In diesen Figuren ist jeweils nur der vordere Teil der Vorrichtung gezeigt. Der restliche Aufbau (insbesondere der Austragskolben und der Förderkolben) ist dann beispielsweise zu einem der ersten fünf Ausführungsbeispiele identisch.

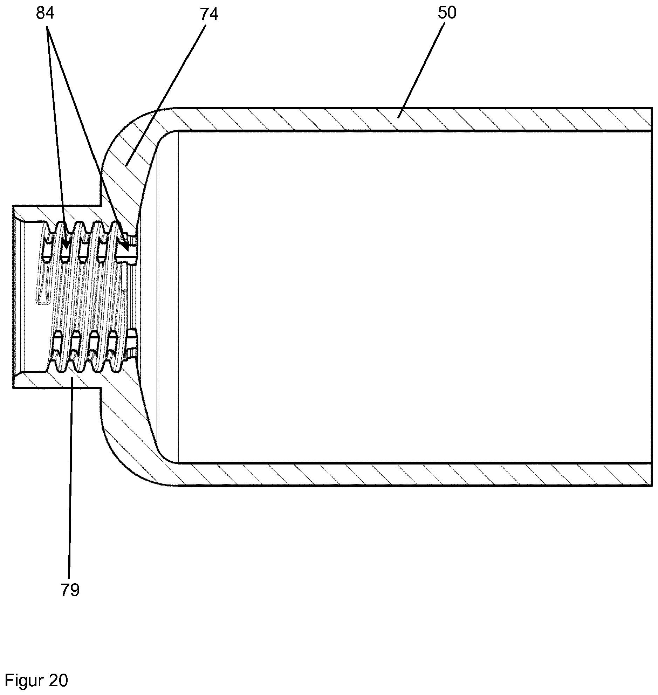

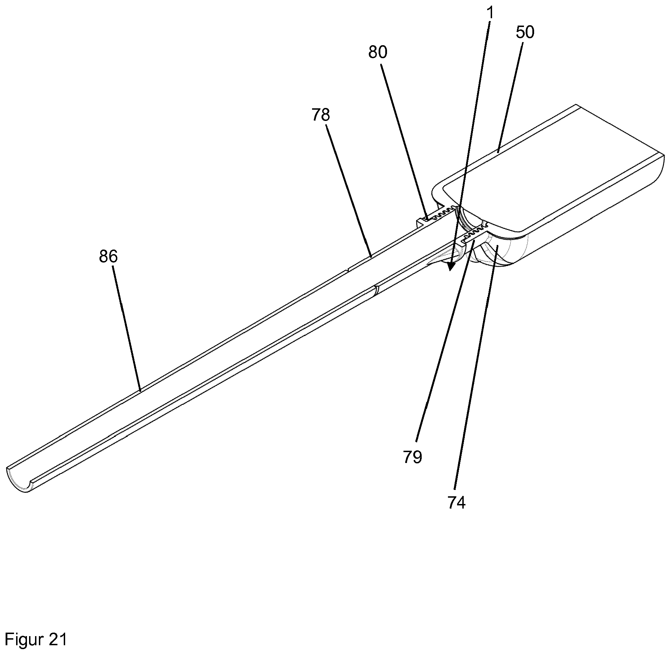

In den beiden Querschnittansichten nach den Figuren 19 und 21 ist zu erkennen, dass bei dieser Ausführung analog zu der fünften Ausführung der Vorrichtung eine Wandung 74 vorgesehen ist, die durch den Kartuschenkopf 74 gebildet ist. Ein Austragsrohr 78 ist in einen Stutzen 79 geschraubt. Dazu weist der Stutzen 79 ein Innengewinde auf und das Austragsrohr 78 ein Außengewinde. Der Stutzen 79 ist einteilig mit der Wandung 74 und der Kartusche 50 ausgeführt. Zwischen dem Austragsrohr 78 und dem Stutzen 79 ist ein poröser Kunststoffring 80 als Dichtung vorgesehen. Der poröse Kunststoffring 80 ist gegenüber dem Pulver 1 dicht aber gegenüber Gas durchlässig. Dadurch kann entweichendes Gas 82 aus dem Inneren der Kartusche 50 nach außen treten und sterilisierendes Gas wie Ethylenoxid eindringen. Damit der Gasdurchfluss durch das Gewinde nicht zu stark behindert wird, sind in dem Stutzen 79 Öffnungen 84 vorgesehen, die das Gewinde nicht vollständig durchbrechen, die aber einen Gasdurchfluss ermöglichen (siehe Figur 20). Das Austragsrohr 78 kann auch eine Austragsrohrverlängerung 86 aufweisen, die über eine Sollbruchstelle mit dem restlichen Austragsrohr 78 verbunden ist. Je nach Anforderung kann der Knochenzementteig dann durch die Austragsrohrverlängerung 86 (siehe Figur 21) oder durch das kürzere abgebrochene Austragsrohr 78 (siehe Figur 19) appliziert werden.

Die wesentlichen Bauteile mit dem erfindungsgemäßen Verfahren hergestellter Vorrichtungen können durch Spritzgießen aus Kunststoff kostengünstig hergestellt werden.

Bei allen verschiedenen Vorrichtungen nach den Figuren 1 bis 21 kann vorgesehen sein, dass am Austragsrohr 18, 72 oder am Austragsrohrstutzen 66 oder am Stutzen 79 ein elastischer Schlauch (nicht gezeigt) angeordnet ist, der in einem Trokar enden kann. Dadurch kann die Vorrichtung für die Vertebroplastie eingesetzt werden.

Um ein Nachfließen des Knochenzementteigs zu verhindern kann vorgesehen sein, dass am Austragsrohr 18, 72 oder am Austragsrohrstutzen 66 oder am Stutzen 79 ein Druckablassventil (nicht gezeigt) mit einer Kammer zur Aufnahme von Knochenzementteig 44 vorgesehen ist. Durch Öffnen des Druckablassventils kann ein in dem Innenraum auf dem Knochenzementteig 44 lastender Druck reduziert werden, ohne dass der Knochenzementteig 44 über längere Zeit an der Applikationsspitze nachläuft. Mit der Kammer wird verhindert, dass der Knochenzementteig in die Umgebung gelangt.

Das Pulver 1 enthält bei allen Ausführungsbeispielen entweder 0,0-15,0 Gew.-% Röntgenopaker, 0,4-3,0 Gew.-% Dibenzoylperoxid, 79,5-99,3 Gew.-% Polymethylmethacrylat und/oder Polymethylmethacrylat-Copolymer, und 0,1-2,5 Gew.-% Additiv oder das Pulver 1 enthält 1,0-10 Gew.-% Antiinfektivum oder Antiseptikum, 0,0-15,0 Gew.-% Röntgenopaker, 0,4-3,0 Gew.-% Dibenzoylperoxid, 69,5-98,3 Gew.-% Polymethylmethacrylat und/oder Polymethylmethacrylat-Copolymer, und 0,1-2,5 Gew.-% Additiv.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Pulver
- 2: Monomerflüssigkeit
- 3: Vorderer Kartuschenteil
- 4: Hinterer Kartuschenteil
- 5: Förderkolben
- 6: Austragskolben
- 7: Bewegliche Wandung des Verschlusssystems
- 8: Stopfen des Verschlusssystems
- 9: Behälter / Ampulle für die Monomerflüssigkeit
- 10: Kartuschenkopf
- 11: Begasungsöffnung
- 12: Kappe
- 14: Halterungsring des Verschlusssystems
- 16: Gasdurchlässiger Filter
- 18: Austragsrohr
- 19: Nocke
- 20: Flüssigkeitsdurchlässiger Porenfilter
- 21: Umlaufende Nut
- 22: Durchführung
- 24: Gitter / Sieb
- 26: Kartuschenkopf
- 28, 30: Umlaufender Dichtungsring
- 32, 34: Umlaufender Dichtungsring
- 36: Halterung zur Befestigung einer Auspresseinrichtung
- 38: Rastmittel / Haken
- 40: Auspresseinrichtung
- 41: Gegenstück zur Befestigung der Auspresseinrichtung
- 42: Stößel / Stange
- 44: Knochenzementteig
- 46: Kartusche
- 48: Austragsöffnung
- 50: Kartusche
- 52: Füllmaterial
- 54: Stopfen
- 56: Begasungsöffnung
- 58: Umlaufende Dichtung
- 60: Umlaufende Dichtung
- 62: Splitter des Behälters / der Ampulle
- 64: Kartuschenkopf
- 66: Austragsrohrstutzen
- 68: Austragsrohrverlängerung
- 70: Stopfen
- 72: Austragsrohr
- 74: Wandung / Kartuschenkopf
- 76: Poröser Kunststoffring
- 78: Austragsrohr
- 79: Stutzen
- 80: Poröser Kunststoffring
- 82: Entweichendes Gas
- 84: Öffnung
- 86: Austragsrohrverlängerung

## Patentansprüche

1. Verfahren zur Herstellung eines Prepacked-PMMA-Zementkartuschensystems, das zum Mischen und Applizieren eines PMMA-Knochenzementteigs (44) geeignet ist und in dem ein Pulver (1) als eine Ausgangskomponente des Knochenzements gelagert ist, mit den Schritten
Bereitstellen einer Kartusche (3, 4, 46, 50) mit einem zylindrischen Innenraum und eines Austragskolbens (6) mit einem zum zylindrischen Innenraum passenden Außenumfang,
Einfüllen des Pulvers (1) in den Innenraum der Kartusche (3, 4, 46, 50), wobei das Pulver (1) ein Zementpulver zur Herstellung des Knochenzementteigs (44) als Hauptkomponente aufweist und das Pulver (1) zusätzlich ein hydrophiles Additiv umfasst, das in dem Zementpulver verteilt ist, **gekennzeichnet durch** die Schritte
Komprimieren des Pulvers (1) in dem Innenraum der Kartusche (3, 4, 46, 50) zwischen dem Austragskolben (6) und einer Wandung (7, 74), die den zylindrischen Innenraum an einer Vorderseite begrenzt, durch Reduzieren des Abstands zwischen dem Austragskolben (6) und der Wandung (7, 74) im Innenraum,
Arretieren, Abstützen oder Befestigen der Wandung (7, 74) und/oder des Austragskolbens (6), so dass während der Lagerung des Pulvers (1) von der Wandung (7, 74) und dem Austragskolben (6) ein mechanischer Druck auf das Pulver (1) ausgeübt wird und das Pulver (1) zusammengepresst bleibt.

2. Verfahren nach Anspruch 1, **dadurch gekennzeichnet, dass**
der Austragskolben (6) durch Presspassung, ein Rastmittel (38) und/oder durch Abstützen gegen die Kartusche (3, 4, 46, 50) gehalten wird und die Wandung (74) einteilig mit der Kartusche (50) ausgeführt ist oder die Wandung (7) an der Innenseite der Kartusche (50) anliegt oder die Wandung (7) durch eine Arretierung oder durch eine Presspassung in der Kartusche (3, 4, 46, 50) gehalten wird.

3. Verfahren nach Anspruch 1 oder 2, **dadurch gekennzeichnet, dass**
in dem Innenraum der Kartusche (3, 4, 46, 50) zumindest ein Behälter (9) enthaltend eine Monomerflüssigkeit (2) als weitere Ausgangskomponente des Knochenzements und am Kartuschenboden der Kartusche (3, 4, 46, 50) ein Förderkolben (5) angeordnet wird, wobei der zumindest eine Behälter (9) zwischen dem Förderkolben (5) und dem Austragskolben (6) angeordnet wird.

4. Verfahren nach Anspruch 3, **dadurch gekennzeichnet, dass**
der Austragskolben (6) von dem zumindest einen Behälter (9) und dem am Kartuschenboden in dem Innenraum der Kartusche (3, 4, 46, 50) angeordneten Förderkolben (5) abgestützt wird.

5. Verfahren nach einem der Ansprüche 3 oder 4, **dadurch gekennzeichnet, dass**
der Behälter (9) für die Monomerflüssigkeit (2) eine Glasampulle (9) oder Kunststoffampulle (9) ist, die durch eine Bewegung des Förderkolbens (5) aufzubrechen ist, oder der Behälter (9) für die Monomerflüssigkeit (2) ein Folienbeutel ist, der durch die Bewegung des Förderkolbens (5) aufzuschlitzen, aufzustechen oder aufzureißen ist.

6. Verfahren nach einem Ansprüche 3 bis 5, **dadurch gekennzeichnet, dass**
der Förderkolben (5) den Innenraum der Kartusche (3, 4, 46, 50) am Kartuschenboden verschließt, insbesondere druck- und flüssigkeitsdicht verschließt.

7. Verfahren nach einem der Ansprüche 3 bis 6, **dadurch gekennzeichnet, dass**
in einem hinteren Teil des Innenraums der Kartusche (3, 4, 46, 50), in dem der zumindest eine Behälter (9) angeordnet ist, zusätzlich ein Füllmaterial (52) angeordnet wird, insbesondere zumindest ein Schaumstoffkörper und/oder Kunststoffkörner angeordnet wird.

8. Verfahren nach einem der vorangehenden Ansprüche, **dadurch gekennzeichnet, dass**
die Kartusche (3, 4, 46, 50) rohrförmig ist, das Pulver (1) in einen vorderen Teil des Innenraums der Kartusche (3, 4, 46, 50) eingefüllt wird.

9. Verfahren nach einem der vorangehenden Ansprüche, **dadurch gekennzeichnet, dass**
ein Leitungsmittel (22) vorgesehen wird, das den vorderen Teil und den hinteren Teil des Innenraums der Kartusche (3, 4) miteinander für die Monomerflüssigkeit (2) und für Gase durchlässig verbindet und das für das Pulver (1) undurchlässig ist, so dass durch Vortreiben des Förderkolbens (5) in Richtung des Austragskolbens (6) der Behälter (9) zu öffnen ist, die Monomerflüssigkeit (2) in das Pulver (1) zu pressen ist und anschließend der Austragskolben (6) mit dem Förderkolben (5) in Richtung der Vorderseite der Kartusche (3, 4, 46, 50) zu drücken ist.

10. Verfahren nach einem der vorangehenden Ansprüche, **dadurch gekennzeichnet, dass**
in dem Austragskolben (6) und/oder zwischen dem Austragskolben (6) und der Innenwand des Innenraums zumindest eine Durchführung (22) als Leitungsmittel (22) angeordnet wird, durch die der vordere Teil des Innenraums und der hintere Teil des Innenraums miteinander verbunden werden, wobei in oder an der zumindest einen Durchführung (22) ein für das Pulver (1) undurchlässiger und für die Monomerflüssigkeit (2) und Gase durchlässiger Filter (20) angeordnet wird.

11. Verfahren nach einem der vorangehenden Ansprüche, **dadurch gekennzeichnet, dass**
an dem Austragskolben (6) ein Rastmittel (38) angeordnet ist, so dass der Austragskolben (6) zwischen dem vorderen und dem hinteren Teil des Innenraums mit der Kartusche (3, 4, 46, 50) rasten kann, wobei vorzugsweise diese Rastung durch die beim Öffnen des Behälters (9) auftretenden Kräfte und einen auf die Monomerflüssigkeit (2) vom Förderkolben (5) ausgeübten Druck nicht zu lösen ist, aber durch einen direkt von dem Förderkolben (5) auf den Austragskolben (6) wirkenden Druck lösbar ist.

12. Verfahren nach einem der vorangehenden Ansprüche, **dadurch gekennzeichnet, dass**
an einem an der Vorderseite der Kartusche (3, 4, 46, 50) angeordnetem Kartuschenkopf (10, 64, 74) ein mehrteiliges Verschlusssystem umfassend eine Austragsöffnung (48) angeordnet wird, wobei zumindest zwei Teile (7, 8, 54, 70, 74) des Verschlusssystems angetrieben durch eine Bewegung des gemischten Knochenzementteigs (44) gegeneinander bewegbar sind und sich durch diese Bewegung der zumindest zwei Teile (7, 8, 54, 70, 74) des Verschlusssystems gegeneinander die Austragsöffnung (48) öffnet, und wobei die Bewegung des gemischten Knochenzementteigs (44) durch einen Druck des Austragskolbens (6) auf den Knochenzementteig (44) anzutreiben ist, wobei vorzugsweise ein Teil des Verschlusssystems, insbesondere ein Stopfen (8, 54, 70), eine für Gase durchlässige aber für das Pulver (1) und die Monomerflüssigkeit (2) undurchlässige Verbindung zwischen dem vorderen Teil des Innenraums der Kartusche (3, 4, 46, 50) und der Umgebung der Vorrichtung umfasst.

13. Verfahren nach einem der vorangehenden Ansprüche, **dadurch gekennzeichnet, dass**
das Pulver (1) mindestens ein partikuläres Polymethylmethacrylat oder Polymethylmethacrylat-Copolymer der Siebfraktion kleiner 100 µm, einen Initiator, und mindestens ein in Methylmethacrylat unlösliches, partikuläres oder faserförmiges Additiv aufweist, wobei das Additiv ein Aufsaugvermögen größer gleich 0,6 g Methylmethacrylat pro Gramm Additiv bei Raumtemperatur besitzt.

## Claims

1. A method for producing a prepacked PMMA cement cartridge system which is suitable for mixing and applying a PMMA bone cement dough (44) and in which a powder (1) is stored as a starting component of the bone cement, having the steps
providing a cartridge (3, 4, 46, 50) having a cylindrical interior and a discharge piston (6) having an external periphery matching the cylindrical interior,
filling the powder (1) into the interior of the cartridge (3, 4, 46, 50), wherein the powder (1) comprises a cement powder as main component for producing the bone cement dough (44), and the powder (1) additionally comprises a hydrophilic additive, which is distributed in the cement powder, **characterized by** the steps
compressing the powder (1) in the interior of the cartridge (3, 4, 46, 50) between the discharge piston (6) and a wall (7, 74), which delimits the cylindrical interior on a front side, by reducing the distance between the discharge piston (6) and the wall (7, 74) in the interior,
locking, supporting or fastening the wall (7, 74) and/or the discharge piston (6), so that, during the storage of the powder (1), a mechanical pressure is exerted onto the powder (1) by the wall (7, 74) and the discharge piston (6), and the powder (1) remains compacted.

2. The method according to claim 1, **characterised in that**
the discharge piston (6) is held against the cartridge (3, 4, 46, 50) by means of a press fit, a detent means (38) and/or by being supported, and the wall (74) is embodied in one part with the cartridge (50) or the wall (7) bears against the inner face of the cartridge (50), or the wall (7) is held in the cartridge (3, 4, 46, 50) by being locked or by a press fit.

3. The method according to claim 1 or 2, **characterised in that**
at least one container (9) containing a monomer liquid (2) as further starting component of the bone cement is arranged in the interior of the cartridge (3, 4, 46, 50), and a conveying piston (5) is arranged at the cartridge base of the cartridge (3, 4, 46, 50), wherein the at least one container (9) is arranged between the conveying piston (5) and the discharge piston (6).

4. The method according to claim 3, **characterised in that**
the discharge piston (6) is supported by the at least one container (9) and the conveying piston (5) arranged at the cartridge base in the interior of the cartridge (3, 4, 46, 50).

5. The method according to claim 3 or 4, **characterised in that**
the container (9) for the monomer liquid (2) is a glass ampoule (9) or plastic ampoule (9), which is breakable open by a movement of the conveying piston (5), or the container (9) for the monomer liquid (2) is a film bag, which is rippable, pierceable or slittable by the movement of the conveying piston (5).

6. The method according to any one claims 3 to 5, **characterised in that**
the conveying piston (5) closes the interior of the cartridge (3, 4, 46, 50) at the cartridge base, in particular closes it in a pressure-tight and liquid-tight manner.

7. The method according to any one of claims 3 to 6, **characterised in that**
at least one filling material (52) is arranged in the rear part of the interior of the cartridge (3, 4, 46, 50), in which the at least one container (9) is arranged, wherein the filling material (52) is preferably a foam material and/or is formed by plastic beads.

8. The method according to any one of the preceding claims, **characterised in that** the cartridge (3, 4, 46, 50) is tube shaped and the powder (1) is filled into a front part of the interior of the cartridge (3, 4, 46, 50).

9. The method according to any one of the preceding claims, **characterised in that** a conduit means (22) is provided, which connects the front part and the rear part of the interior of the cartridge (3, 4) to one another in a manner permeable for the monomer liquid (2) and for gases and which is impermeable for the powder (1), such that by advancing the conveying piston (5) in the direction of the discharge piston (6), the container (9) is openable, the monomer liquid (2) is pressable into the powder (1), and the discharge piston (6) is then pushable by the conveying piston (5) in the direction of the front side of the cartridge (3, 4, 46, 50).

10. The method according to any one of the preceding claims, **characterised in that** at least one feedthrough (22) is provided in the discharge piston (6) and/or between the discharge piston (6) and the inner wall of the interior as conduit means (22), by which the front part of the interior and the rear part of the interior are connected to one another, wherein a filter (20) impermeable for the powder (1) and permeable for the monomer liquid (2) and gases is arranged in or on the at least one feedthrough (22).

11. The method according to any one of the preceding claims, **characterised in that** a detent means (38) is arranged on the discharge piston (6), so that the discharge piston (6) can latch with the cartridge (3, 4, 46, 50) between the front and the rear part of the interior, wherein this latching cannot be released by the forces occurring as the container (9) is opened and a pressure exerted onto the monomer liquid (2) by the conveying piston (5), but is releasable by a pressure acting on the discharge piston (6) directly from the conveying piston (5).

12. The method according to any one of the preceding claims, **characterised in that** a multi-part closure system comprising a discharge opening (48) is arranged on a cartridge head (10, 64, 74) arranged at the front side of the cartridge (3, 4, 46, 50), wherein at least two parts (7, 8, 54, 70, 74) of the closure system are movable relative to one another, driven by a movement of the mixed bone cement dough (44), and the discharge opening (48) opens as a result of this movement of the at least two parts (7, 8, 54, 70, 74) of the closure system relative to one another, and wherein the movement of the mixed bone cement dough (44) is drivable by a pressure of the discharge piston (6) on the bone cement dough (44), wherein preferably part of the closure system, in particular a stopper (8, 54, 70), comprises a connection between the front part of the interior of the cartridge (3, 4, 46, 50) and the surrounding environment of the device, which connection is permeable for gases, but is impermeable for the powder (1) and the monomer liquid (2).

13. The method according to any one of the preceding claims, **characterised in that** the powder (1) comprises at least one particulate polymethyl methacrylate or polymethyl methacrylate copolymer of the sieve fraction smaller than 100 µm, an initiator, and at least one particulate or fibrous additive that is insoluble in methyl methacrylate, wherein the additive has an absorption capacity of greater than or equal to 0.6 g of methyl methacrylate per gram of additive at room temperature.

## Revendications

1. Procédé de fabrication d'un système de cartouches de ciment de PMMA préconditionné qui est approprié pour le mélange et l'application d'une pâte de ciment osseux de PMMA (44) et dans lequel une poudre (1) est conservée en tant que composant de départ du ciment osseux, avec les étapes :
de fourniture d'une cartouche (3, 4, 46, 50) avec un espace intérieur cylindrique et un piston d'évacuation (6) doté d'un pourtour extérieur correspondant à l'espace intérieur cylindrique,
de remplissage de la poudre (1) dans l'espace intérieur de la cartouche (3, 4, 46, 50), où la poudre (1) présente une poudre de ciment pour la fabrication de la pâte de ciment osseux (44) en tant que composant principal et la poudre (1) comprend en complément un additif hydrophile qui est dispersé dans la poudre de ciment, **caractérisé par** les étapes
de compression de la poudre (1) dans l'espace intérieur de la cartouche (3, 4, 46, 50) entre le piston d'évacuation (6) et une paroi (7, 74) qui délimite l'espace intérieur cylindrique au niveau d'un côté avant, par une réduction de la distance entre le piston d'évacuation (6) et la paroi (7, 74) dans l'espace intérieur,
d'immobilisation, de mise en appui ou de fixation de la paroi (7, 74), et/ou du piston d'évacuation (6), de sorte que, pendant le stockage de la poudre (1), une pression mécanique s'exerce par la paroi (7, 74) et le piston d'évacuation (6) sur la poudre (1), et la poudre (1) reste comprimée.

2. Procédé selon la revendication 1, **caractérisé en ce que**
le piston d'évacuation (6) est maintenu par un ajustement serré, un organe de butée (38), et/ou par un appui, contre la cartouche (3, 4, 46, 50), et la paroi (74) est conçue d'un seul tenant avec la cartouche (50), ou la paroi (7) s'applique contre le côté intérieur de la cartouche (50), ou la paroi (7) est maintenue par une immobilisation ou par un ajustement serré dans la cartouche (3, 4, 46, 50).

3. Procédé selon la revendication 1 ou la revendication 2, **caractérisé en ce qu'**au moins un récipient (9) contenant un liquide de monomère (2) servant d'autre composant de départ du ciment osseux est disposé dans l'espace intérieur de la cartouche (3, 4, 46, 50) et qu'un piston de transport (5) est disposé sur le fond de cartouche de la cartouche (3, 4, 46, 50), où l'au moins un récipient (9) est disposé entre le piston de transport (5) et le piston d'évacuation (6).

4. Procédé selon la revendication 3, **caractérisé en ce que** le piston d'évacuation (6) est soutenu par l'au moins un récipient (9) et le piston de transport (5) disposé dans le fond de cartouche dans l'espace intérieur de la cartouche (3, 4, 46, 50).

5. Procédé selon l'une des revendications 3 ou 4, **caractérisé en ce que** le récipient (9) pour le liquide de monomère (2) est une ampoule en verre (9) ou une ampoule en matière plastique (9), qui peut être cassée par un déplacement du piston de transport (5), ou le récipient (9) pour le liquide de monomère (2) est un pochon en film qui peut être ouvert, percé ou déchiré par le déplacement du piston de transport (5).

6. Procédé selon l'une des revendications 3 à 5, **caractérisé en ce que** le piston de transport (5) ferme l'espace intérieur de la cartouche (3, 4, 46, 50) au niveau du fond de cartouche, ferme notamment de manière étanche en pression et étanche pour les liquides.

7. Procédé selon l'une des revendications 3 à 6, **caractérisé en ce qu'**un matériau de remplissage (52), notamment, au moins une substance en mousse et/ou des granulés en matière plastique, est disposé de manière complémentaire dans une partie arrière de l'espace intérieur de la cartouche (3, 4, 46, 50) dans laquelle l'au moins un récipient (9) est disposé.

8. Procédé selon l'une des revendications précédentes, **caractérisé en ce que** la cartouche (3, 4, 46, 50) est de forme tubulaire, la poudre (1) est remplie dans une partie avant de l'espace intérieur de la cartouche (3, 4, 46, 50).

9. Procédé selon l'une des revendications précédentes, **caractérisé en ce qu'**un moyen de guidage (22) est prévu qui relie ensemble la partie avant et la partie arrière de l'espace intérieur de la cartouche (3, 4) et qui est perméable vis-à-vis du liquide de monomère (2) et aux gaz et ne laisse pas passer la poudre (1), de sorte que, par l'avancée du piston de transport (5) en direction du piston d'évacuation (6), le récipient (9) peut s'ouvrir, le liquide de monomère (2) peut se comprimer dans la poudre (1) et ensuite le piston d'évacuation (6) avec le piston de transport (5) peuvent être poussés en direction du côté avant de la cartouche (3, 4, 46, 50).

10. Procédé selon l'une des revendications précédentes, **caractérisé en ce qu'**au moins une conduite de passage (22) est disposée pour servir de moyen de guidage (22) dans le piston d'évacuation (6) et/ou entre le piston d'évacuation (6) et la paroi intérieure, par laquelle les partie avant de l'espace intérieur et partie arrière de l'espace intérieur sont reliées ensemble, où un filtre (20) perméable aux gaz et pour le liquide de monomère (2) et ne laissant pas passer la poudre (1) est disposé dans l'au moins une conduite de passage (22).

11. Procédé selon l'une des revendications précédentes, **caractérisé en ce qu'**une butée (38) est disposée sur le piston d'évacuation (6) de sorte que le piston d'évacuation (6) puisse venir en butée avec la cartouche (3, 4, 46, 50) entre la partie avant et arrière de l'espace intérieur, où, de préférence, cette butée ne peut être activée par les forces exercées lors de l'ouverture du récipient (9) et une pression exercée sur le liquide de monomère (2) par le piston de transport, mais peut être activée par une pression agissant directement sur le piston d'évacuation (6) par le piston de transport (5).

12. Procédé selon l'une des revendications précédentes, **caractérisé en ce qu'**un système de fermeture en plusieurs éléments comprenant un orifice d'évacuation (48) est disposé au niveau d'une tête de cartouche (10, 64, 74) disposée sur le côté avant de la cartouche (3, 4, 46, 50), où au moins deux éléments (7, 8, 54, 70, 74) du système de fermeture peuvent être déplacés l'un vers l'autre, entraînés par un déplacement de la pâte de ciment osseux (44) mélangée et que l'orifice d'évacuation (48) s'ouvre par ce déplacement de l'un vers l'autre des au moins deux éléments (7, 8, 54, 70, 74) du système de fermeture, et où le déplacement de la pâte de ciment osseux (44) mélangée peut être entraîné par une pression du piston d'évacuation (6) sur la pâte de ciment osseux (44), où, de préférence, un élément du système de fermeture, notamment, un bouchon (8, 54, 7) comprend une connexion perméable aux gaz, mais ne laissant pas passer la poudre (1) et le liquide de monomère (2) entre la partie avant de l'espace intérieur de la cartouche (3, 4, 46, 50) et l'environnement du dispositif.

13. Procédé selon l'une des revendications précédentes, **caractérisé en ce que** la poudre (1) présente au moins un poly méthyl méthacrylate particulaire ou un copolymère de poly méthyl méthacrylate avec une fraction granulométrique inférieure à 100 µm, un initiateur, et au moins un additif insoluble dans le méthacrylate de méthyle, particulaire ou en forme de fibre, où l'additif possède une capacité d'aspiration supérieure ou égale à 0,6 g de méthacrylate de méthyle par gramme d'additif à la température ambiante.
